# EUROPEAN PATENT APPLICATION

(11) **EP 1 441 013 A1**
(43) Date of publication of application: **28.07.2004**
(21) Application number: 02768109.7
(22) Date of filing: 27.09.2002
(51) Int. Cl.: C09D 11/00, C09B 29/42, C09B 33/12, C09B 33/24, C07D 213/72, C07D 213/85, C07D 401/14, C07D 487/04

(54) **WATERCOLOR INK**

(30) Priority: 28.09.2001 JP 2001301901; 28.09.2001 JP 2001301903; 28.03.2002 JP 2002090267
(71) Applicant: Mitsui Chemicals, Inc., Tokyo 105-7117 (JP)
(72) Inventor: KOHGO, Osamu c/o Mitsui Chemicals, Inc., Sodegaura-shi, Chiba 299-0265 (JP); NARUSE, Hiroshi c/o Mitsui Chemicals, Inc., Sodegaura-shi, Chiba 299-0265 (JP); FUJII, Kenichi c/o Mitsui Chemicals, Inc., Sodegaura-shi, Chiba 299-0265 (JP); MISAWA, Tsutami c/o Mitsui Chemicals, Inc., Sodegaura-shi, Chiba 299-0265 (JP); OGISO, Akira c/o Mitsui Chemicals, Inc., Sodegaura-shi, Chiba 299-0265 (JP); SAITO, Yasunori c/o Mitsui Chemicals, Inc., Sodegaura-shi, Chiba 299-0265 (JP); SUZUKI, Rihoko c/o Mitsui Chemicals, Inc., Sodegaura-shi, Chiba 299-0265 (JP)
(74) Representative: Paget, Hugh Charles Edward
(86) International application number: PCT/JP2002/010054
(87) International publication number: WO 2003/029363

(57) **Abstract**

Aqueous ink which contains compounds having one or more structures represented by general formula (1) in a molecule or salts thereof and which has a clear hue and is excellent in light fastness and moisture resistance is provided. 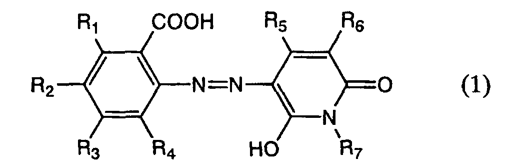 wherein each of R₁ to R₅ and R₇ independently, represents a hydrogen atom, a halogen atom, an unsubstituted or substituted alkyl group, an unsubstituted or substituted alkoxy group, an unsubstituted or substituted alkenyl group, an unsubstituted or substituted aryl group, a hydroxyl group, -COOH, -SO₃H, -CONR₈R₉ or -COXR₁₀, and R₆ represents a hydrogen atom, a cyano group, an aminocarbonyl group, a sulfomethyl group or -SO₃H

## Description

### Technical Field

The present invention relates to compounds and yellow aqueous ink having a clear hue, high light fastness and high moisture resistance.

### Background Art

Aqueous ink used in writing instruments or for recording of an ink-jet recording system or the like is basically made of a dye, water and an organic solvent. From a consideration of an odor and safety to the human body and the surrounding environment, aqueous ink containing water as a main solvent has been a mainstream. Further, as a dye, water-soluble dyes such as acid dyes, basic dyes, reactive dyes and direct dyes have been generally used.

Various characteristics have been required for these dyes and aqueous ink. Especially for aqueous ink used in a recording liquid of an ink-jet recording system, the following various characteristics are required. That is,
(1) physical properties of ink such as viscosity, surface tension, specific conductivity, density and pH are appropriate,
(2) long-term storage stability of ink is good,
(3) dissolution stability of a soluble component is high and clogging of a nozzle does not occur,
(4) quick-drying property of a recording medium is good, and
(5) a recorded image is sharp, and light fastness and moisture resistance are good.

Various studies have been made to satisfy these characteristics. For example, JP-A-2-153977 proposes ink containing pyridone azo compounds having a sulfonic group as a water-soluble yellow dye. However, these compounds are, though the hue is clear, low in light fastness, and thus do not satisfy all of the required characteristics. Further, azo dyes such as a pyrazolone azo dye have been studied as the yellow dye. However, those that satisfy all of the required characteristics have not been obtained at present.

For improving light fastness of ink, ink using pigments, ink using oil-soluble dyes and the like have been studied. However, the use of pigments has been problematic in that dispersion stability is poor and storage stability is insufficient to cause clogging of a nozzle. Further, the use of oil-soluble dyes has been problematic in environmental hygiene such as an odor or the like owing to the use of an organic solvent.

Especially, characteristics of ink used in an ink-jet recording system are greatly influenced by characteristics inherent in dyes. It is thus quite important to produce dyes that satisfy these conditions.

### Disclosure of the Invention

The invention aims to provide compounds and yellow aqueous ink having a clear hue, high light fastness and high moisture resistance.

The present inventors have assiduously conducted investigations to solve the foregoing problems, and have consequently found that aqueous ink containing compounds having a structure represented by general formula (1) in a molecule or salts thereof has a clear hue and high light fastness. This finding has led to the completion of the invention. That is, the invention is as follows.
(1) Aqueous ink which contains compounds having a structure represented by general formula (1) in a molecule or salts thereof 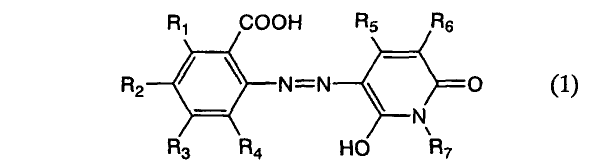 wherein
   each of R₁ to R₅ and R₇ independently, represents a hydrogen atom, a halogen atom, an unsubstituted or substituted alkyl group, an unsubstituted or substituted alkoxy group, an unsubstituted or substituted alkenyl group, an unsubstituted or substituted aryl group, a hydroxyl group, -COOH, -SO₃H, -CONR₈R₉ in which each of R₈ and R₉ independently, represents a hydrogen atom, an unsubstituted or substituted alkyl group, an unsubstituted or substituted aryl group or an unsubstituted or substituted heterocyclic group and R₈ and R₉ may form a ring by an unsubstituted or substituted alkylene group, -COXR₁₀ in which R₁₀ represents a hydrogen atom, an unsubstituted or substituted alkyl group, an unsubstituted or substituted aryl group or an unsubstituted or substituted heterocyclic group and X represents an oxygen atom or a sulfur atom, or a divalent to tetravalent bonding group through which compounds represented by formula (1) are bonded to form a polymeric structure, and R₆ represents a hydrogen atom, a cyano group, an aminocarbonyl group, a sulfomethyl group or -SO₃H.
(2) Aqueous ink which contains compounds having two or more groups represented by general formula (2) in a molecule or salts thereof 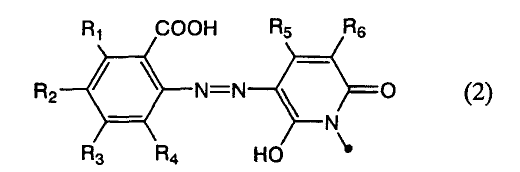 wherein
   each of R₁ to R₅ independently, represents a hydrogen atom, a halogen atom, an unsubstituted or substituted alkyl group, an unsubstituted or substituted alkoxy group, an unsubstituted or substituted alkenyl group, an unsubstituted or substituted aryl group, a hydroxyl group, -COOH, -SO₃H, -CONR₈R₉ in which each of R₈ and R₉ independently, represents a hydrogen atom, an unsubstituted or substituted alkyl group, an unsubstituted or substituted aryl group or an unsubstituted or substituted heterocyclic group and R₈ and R₉ may form a ring by an unsubstituted or substituted alkylene group, or -COXR₁₀ in which R₁₀ represents a hydrogen atom, an unsubstituted or substituted alkyl group, an unsubstituted or substituted aryl group or an unsubstituted or substituted heterocyclic group and X represents an oxygen atom or a sulfur atom, and R₆ represents a hydrogen atom, a cyano group, an aminocarbonyl group, a sulfomethyl group or -SO₃H.
(3) Ink for ink-jet recording which contains the aqueous ink according to (1) or (2).
(4) Compounds having a structure represented by general formula (1) in a molecule or salts thereof 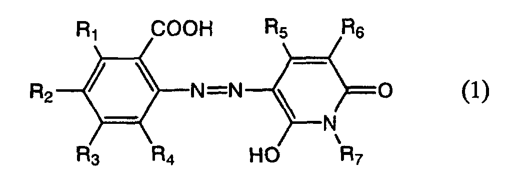 wherein
   each of R₁ to R₅ and R₇ independently, represents a hydrogen atom, a halogen atom, an unsubstituted or substituted alkyl group, an unsubstituted or substituted alkoxy group, an unsubstituted or substituted alkenyl group, an unsubstituted or substituted aryl group, a hydroxyl group, -COOH, -SO₃H, -CONR₈R₉ in which each of R₈ and R₉ independently, represents a hydrogen atom, an unsubstituted or substituted alkyl group, an unsubstituted or substituted aryl group or an unsubstituted or substituted heterocyclic group and R₈ and R₉ may form a ring by an unsubstituted or substituted alkylene group, -COXR₁₀ in which R₁₀ represents a hydrogen atom, an unsubstituted or substituted alkyl group, an unsubstituted or substituted aryl group or an unsubstituted or substituted heterocyclic group and X represents an oxygen atom or a sulfur atom, or a divalent to tetravalent bonding group through which compounds represented by formula (1) are bonded to form a polymeric structure, and R₆ represents a hydrogen atom, a cyano group, an aminocarbonyl group, a sulfomethyl group or -SO₃H, provided that at least one of R₁ to R₅ and R₇ represents a divalent to tetravalent bonding group through which compounds represented by formula (1) are bonded to form a polymeric structure.
(5) Compounds having a structure represented by general formula (1) in a molecule or salts thereof 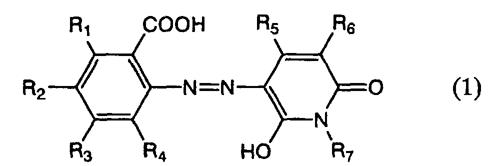 wherein
   each of R₁ to R₅ and R₇ independently, represents a hydrogen atom, a halogen atom, an unsubstituted or substituted alkyl group, an unsubstituted or substituted alkoxy group, an unsubstituted or substituted alkenyl group, an unsubstituted or substituted aryl group, a hydroxyl group, -COOH, -SO₃H, -CONR₈R₉ in which each of R₈ and R₉ independently, represents a hydrogen atom, an unsubstituted or substituted alkyl group, an unsubstituted or substituted aryl group or an unsubstituted or substituted heterocyclic group and R₈ and R₉ may form a ring by an unsubstituted or substituted alkylene group, -COXR₁₀ in which R₁₀ represents a hydrogen atom, an unsubstituted or substituted alkyl group, an unsubstituted or substituted aryl group or an unsubstituted or substituted heterocyclic group and X represents an oxygen atom or a sulfur atom, or a divalent to tetravalent bonding group through which compounds represented by formula (1) are bonded to form a polymeric structure, and R₆ represents a hydrogen atom, a cyano group, an aminocarbonyl group, a sulfomethyl group or -SO₃H, provided that in at least one of structures represented by general formula (1) in the molecule, at least one of R₁ to R₅ and R₇ is -COOH, a substituted alkyl group substituted with -COOH, a substituted alkoxy group substituted with -COOH, a substituted alkenyl group substitutedwith -COOH or a substituted aryl group substituted with -COOH.
(6) Compounds having two or more groups represented by general formula (2) in a molecule or salts thereof 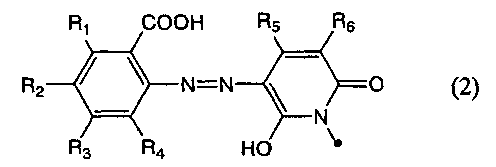 wherein
   each of R₁ to R₅ independently, represents a hydrogen atom, a halogen atom, an unsubstituted or substituted alkyl group, an unsubstituted or substituted alkoxy group, an unsubstituted or substituted alkenyl group, an unsubstituted or substituted aryl group, a hydroxyl group, -COOH, -SO₃H, -CONR₈R₉ in which each of R₈ and R₉ independently, represents a hydrogen atom, an unsubstituted or substituted alkyl group, an unsubstituted or substituted aryl group or an unsubstituted or substituted heterocyclic group and R₈ and R₉ may form a ring by an unsubstituted or substituted alkylene group, or -COXR₁₀ in which R₁₀ represents a hydrogen atom, an unsubstituted or substituted alkyl group, an unsubstituted or substituted aryl group or an unsubstituted or substituted heterocyclic group and X represents an oxygen atom or a sulfur atom, and R₆ represents a hydrogen atom, a cyano group, an aminocarbonyl group, a sulfomethyl group or -SO₃H.

### Best Mode for Carrying Out the Invention

The invention relates to compounds having a structure represented by general formula (1) in a molecule or salts thereof, compounds having two or more groups represented by general formula (2) or salts thereof, and aqueous ink containing these compounds or salts thereof.

Each of R₁ to R₅ in general formulas (1) and (2) and R₇ in general formula (1) independently, represents a hydrogen atom, a halogen atom, an unsubstituted or substituted alkyl group, an unsubstituted or substituted alkoxy group, an unsubstituted or substituted alkenyl group, an unsubstituted or substituted aryl group, a hydroxyl group, -COOH, -SO₃H, -CONR₈R₉ in which each of R₈ and R₉ independently, represents a hydrogen atom, an unsubstituted or substituted alkyl group, an unsubstituted or substituted aryl group or an unsubstituted or substituted heterocyclic group and R₈ and R₉ may form a ring by an unsubstituted or substituted alkylene group, -COXR₁₀ in which R₁₀ represents a hydrogen atom, an unsubstituted or substituted alkyl group, an unsubstituted or substituted aryl group or an unsubstituted or substituted heterocyclic group and X represents an oxygen atom or a sulfur atom, or a divalent to tetravalent bonding group through which compounds represented by formula (1) are bonded to form a polymeric structure. R₆ represents a hydrogen atom, a cyano group, an aminocarbonyl group, a sulfomethyl group or -SO₃H.

Preferably, in at least one of structures represented by general formula (1) in the molecule, at least one of R₁ to R₅ and R₇ is -COOH, a substituted alkyl group substituted with -COOH, a substituted alkoxy group substituted with -COOH, a substituted alkenyl group substituted with -COOH or a substituted aryl group substituted with -COOH.

The salts of the compounds having the structure represented by general formula (1) in the molecule include salts with an alkali metal, an alkaline earth metal and ammonium, and they independently form salts with a hydroxyl group, a thiol group, -COOH or -SO₃H in general formula (1).

Specific examples of the alkali metal include lithium, sodium, potassium, rubidium and cesium. Specific examples of the alkaline earth metal include magnesium, calcium and barium.

Specific examples of ammonium include NH₄, NH₃Et, HN₃Pr, NH₃Bu, NH₃C₈H₁₇, NH₂Et₂, NH₂Pr₂, NH₂Bu₂, NH₂(CH₂CH=CH₂)₂, NHEt₃, NHBu₃, NH(C₂H₄OH)₃, NH(CH₂CH=CH₂)₃, NMe₃CH₂C₆H₅, NMe₄, NBu₄ and the like.

Specific examples of the halogen atom include fluorine, chlorine, bromine and iodine.

Specific examples of the substituent in the substituted alkyl group, the substituted alkoxy group, the substituted alkenyl group, the substituted aryl group, the substituted heterocyclic group and the substituted alkylene group include a halogen atom, an alkyl group, an aryl group, a heterocyclic group, an alkenyl group, an alkoxy group, an alkylthio group, an aryloxy group, an arylthio group, an amino group, a monoalkylamino group, a dialkylamino group, a cyano group, a hydroxyl group, a thiol group, a carboxyl group, a sulfo group, a carbonyl group and the like. The alkyl group, the aryl group, the alkenyl group, the heterocyclic group and the alkylene group may be substituted with not only one substituent but also two or more substituents, and they may be substituted with substituents of one type or different types. The hydroxyl group, the thiol group, the carboxyl group and the sulfo group may form a salt with an alkali metal, an alkaline earth metal or ammonium

Specific examples of the unsubstituted or substituted alkyl group include linear alkyl groups having the total carbon number of from 1 to 20, such as a methyl group, an ethyl group, an n-propyl group, an n-butyl group, an n-pentyl group, an n-hexyl group, an n-heptyl group, an n-octyl group, an n-decyl group, an n-tetradecyl group, an n-octadecyl group and an n-icosyl group,
branched alkyl groups having the total carbon number of from 1 to 20, such as an isopropyl group, an isobutyl group, a sec-butyl group, an isopentyl group, a sec-pentyl group, a 1-methylpentyl group, a 2-methylpentyl group, a 3-methylpentyl group, a 4-methylpentyl group, a 1-ethylbutyl group, a 2-ethylbutyl group, a 1-methylhexyl group, a 2-methylhexyl group, a 3-methylhexyl group, a 4-methylhexyl group, a 5-methylhexyl group, a 1-ethylpentyl group, a 2-ethylpentyl group, a 3-ethylpentyl group, a 1-n-propylbutyl group, a 1-iso-propylbutyl group, a 1-isopropyl-2-methylpropyl group, a 1-methylheptyl group, a 2-methylheptyl group, a 3-methylheptyl group, a 4-methylheptyl group, a 5-methylheptyl group, a 6-methylheptyl group, a 1-ethylhexyl group, a 2-ethylhexyl group, a 3-ethylhexyl group, a 4-ethylhexyl group, a 1-n-propylpentyl group, a 2-n-propylpentyl group, a 1-isopropylpentyl group, a 2-isopropylpentyl group, a 1-n-butylbutyl group, a 1-isobutylbutyl group, a 1-sec-butylbutyl group, a 1-tert-butylbutyl group, a 2-tert-butylbutyl group, a 2-methyloctadecyl group, a tert-butyl group, a tert-pentyl group,
a 1,1-dimethylbutyl group, a 1,2-dimethylbutyl group, a 1,3-dimethylbutyl group, a 2,3-dimethylbutyl group, a 1-ethyl-2-methylpropyl group, a 1,1-dimethylpentyl group, a 1,2-dimethylpentyl group, a 1,3-dimethylpentyl group, a 1,4-dimethylpentyl group, a 2,2-dimethylpentyl group, a 2,3-dimethylpentyl group, a 2,4-dimethylpentyl group, a 3,3-dimethylpentyl group, a 3,4-dimethylpentyl group, a 1-ethyl-1-methylbutyl group, a 1-ethyl-2-methylbutyl group, a 1-ethyl-3-methylbutyl group, a 2-ethyl-1-methylbutyl group, a 2-ethyl-3-methylbutyl group, a 1,1-dimethylhexyl group, a 1,2-dimethylhexyl group, a 1,3-dimethylhexyl group, a 1,4-dimethylhexyl group, a 1,5-dimethylhexyl group, a 2,2-dimethylhexyl group, a 2,3-dimethylhexyl group, a 2,4-dimethylhexyl group, a 2,5-dimethylhexyl group, a 3,3-dimethylhexyl group, a 3,4-dimethylhexyl group, a 3,5-dimethylhexyl group, a 4,4-dimethylhexyl group, a 4,5-dimethylhexyl group, a 1-ethyl-2-methylpentyl group, a 1-ethyl-3-methylpentyl group, a 1-ethyl-4-methylpentyl group, a 2-ethyl-1-methylpentyl group, a 2-ethyl-2-methylpentyl group, a 2-ethyl-3-methylpentyl group, a 2-ethyl-4-methylpentyl group, a 3-ethyl-1-methylpentyl group, a 3-ethyl-2-methylpentyl group, a 3-ethyl-3-methylpentyl group, a 3-ethyl-4-methylpentyl group, a 1-n-propyl-1-methylbutyl group, a 1-n-propyl-2-methylbutyl group, a 1-n-propyl-3-methylbutyl group, a 1-isopropyl-1-methylbutyl group, a 1-isopropyl-2-methylbutyl group, a 1-isopropyl-3-methylbutyl group, a 1,1-diethylbutyl group, a 1,2-diethylbutykl group, a 2,3-dimethylhexadecyl group,
a 1,1,2-trimethylpropyl group, a 1,2,2-trimethylpropyl group, a 1,1,2-trimethylbutyl group, a 1,1,3-trimethylbutyl group, a 1,2,3-trimethylbutyl group, a 1,2,2-trimethylbutyl group, a 1,3,3-trimethylbutyl group, a 2,3,3-trimethylbutyl group, a 1,1,2-trimethylpentyl group, a 1,1,3-trimethylpentyl group, a 1,1,4-trimethylpentyl group, a 1,2,2-trimethylpentyl group, a 1,2,3-trimethylpentyl group, a 1,2,4-trimethylpentyl group, a 1,3,4-trimethylpentyl group, a 2,2,3-trimethylpentyl group, a 2,2,4-trimethylpentyl group, a 2,3,4-trimethylpentyl group, a 1,3,3-trimethylpentyl group, a 2,3,3-trimethylpentyl group, a 3,3,4-trimethylpentyl group, a 1,4,4-trimethylpentyl group, a 2,4,4-trimethylpentyl group, a 3,4,4-trimethylpentyl group, a 1-ethyl-1,2-dimethylbutyl group, a 1-ethyl-1,3-dimethylbutyl group, a 1-ethyl-2,3-dimethylbutyl group, a 2-ethyl-1,1-dimethylbutyl group, a 2-ethyl-1,2-dimethylbutyl group, a 2-ethyl-1,3-dimethylbutyl group, a 2-ethyl-2,3-dimethylbutyl group and a 2,2,3-trimethylheptadecyl group,
saturated cycloalkyl groups having the total carbon number of from 1 to 20, such as a cyclopentyl group, a cyclohexyl group, a methylcyclopentyl group, a methylcyclohexyl group, a 1,2-dimethylcyclohexyl group, a 1,3-dimethylcyclohexyl group, a 1,4-dimethylcyclohexyl group and an ethylcyclohexyl group,
aryl-substituted alkyl groups having the total carbon number of from 1 to 20, such as a benzyl group and a 4-methylbenzyl group,
halogenated alkyl groups having the total carbon number of from 1 to 20 and substituted with a halogen atom partially or completely, such as a fluoromethyl group, a trifluoromethyl group, a chloromethyl group, a dichloromethyl group, a trichloromethyl group, a bromomethyl group, a dibromomethyl group, a tribromomethyl group, a fluoroethyl group, a chloroethyl group, a bromoethyl group, a trifluoroethyl group, a pentafluoroethyl group, a tetrachloroethyl group and a hexafluoroisopropyl group,
hydroxyalkyl groups having the total carbon number of from 1 to 20 and substituted with a hydroxyl group partially or completely, such as a hydroxymethyl group, a 1-hydroxyethyl group, a 2-hydroxyethyl group, a 1-hydroxypropyl group, a 2-hydroxypropyl group, a 3-hydroxypropyl group, a 1-hydroxybutyl group, a 2-hydroxybutyl group, a 3-hydroxybutyl group, a 4-hydroxybutyl group, a 1-hydroxypentyl group, a 2-hydroxypentyl group, a 3-hydroxypentyl group, a 4-hydroxypentyl group, a 5-hydroxypentyl group, a 1-hydroxyhexyl group, a 2-hydroxyhexyl group, a 3-hydroxyhexyl group, a 4-hydroxyhexyl group, a 5-hydroxyhexyl group, a 6-hydroxyhexyl group,
a dihydroxypropyl group, a dihydroxybutyl group, a dihydroxypentyl group, a dihydroxyhexyl group, a trihydroxybutyl group, a trihydroxypentyl group, a trihydroxyhexyl group, a tetrahydroxypentyl group, a tetrahydroxyhexyl group and a pentahydroxyhexyl group,
carboxyalkyl groups having the total carbon number of from 1 to 20 and substituted with a carboxyl group partially or completely, such as a carboxymethyl group, a 1-carboxyethyl group, a 2-carboxyethyl group, a 1-carboxypropyl group, a 2-carboxypropyl group, a 3-carboxypropyl group, a 1-carboxybutyl group, a 2-carboxybutyl group, a 3-carboxybutyl group, a 4-carboxybutyl group, a 1-carboxypentyl group, a 2-carboxypentyl group, a 3-carboxypentyl group, a 4-carboxypentyl group, a 5-carboxypentyl group, a 1-carboxyhexyl group, a 2-carboxyhexyl group, a 3-carboxyhexyl group, a 4-carboxyhexyl group, a 5-carboxyhexyl group, a 6-carboxyhexyl group,
a dicarboxymethyl group, a 1,2-dicarboxyethyl group, a 2,2-dicarboxyethyl group, a 2,3-dicarboxypropyl group, a 1,2-dicarboxypropyl group, a 3,3-dicarboxypropyl group, a 1,2-dicarboxybutyl group, a 2,3-dicarboxybutyl group, a 3,4-dicarboxybutyl group, a 2,4-dicarboxybutyl group, a 1,2-dicarboxypentyl group, a 2,3-dicarboxypentyl group, a 2,4-dicarboxypentyl group, a 2,5-dicarboxypentyl group, a 4,5-dicarboxypentyl group, a 1,2-dicarboxyhexyl group, a 1,3-dicarboxyhexyl group, a 2,3-dicarboxyhexyl group, a 2,4-dicarboxyhexyl group, a 2,5-dicarboxyhexyl group, a 2,6-dicarboxyhexyl group, a 3,5-dicarboxyhexyl group, a 3,6-dicarboxyhexyl group,
a 1,2,2-tricarboxybutyl group, a 1,2,4-tricarboxybutyl group, a 2,3,4-tricarboxybutyl group, a 3,4,4-tricarboxybutyl group, a 1,2,2-tricarboxypentyl group, a 1,2,3-tricarboxypentyl group, a 2,3,4-tricarboxypentyl group, a 3,4,5-tricarboxypentyl group, a 3,5,5-tricarboxypentyl group, a 1,2,3-tricarboxyhexyl group, a 1,2,4-tricarboxyhexyl group, a 2,3,4-tricarboxyhexyl group, a 2,3,5-tricarboxyhexyl group, a 2,3,6-tricarboxyhexyl group, a 3,4,5-tricarboxyhexyl group, a 3,4,6-tricarboxyhexyl group, a 4,5,6-tricarboxyhexyl group,
a 1,2,3,3-tetracarboxypentyl group, a 2,3,4,5-tetracarboxypentyl group, a 2,4,5,5-tetracarboxypentyl group, a 3,4,5,5-tetracarboxypentyl group, a 1,2,3,3,5-pentacarboxyhexyl group, a 1,2,3,4,5-pentacarboxyhexyl group, a 2,3,4,5,5-pentacarboxyhexyl group, a 3,4,4,5,5-pentacarboxyhexyl group, and a 1,2,4,5,5-tetracarboxyhexyl group.
hydroxycarboxyalkyl groups having the total carbon number of from 1 to 20 and substituted with a hydroxyl group and a carboxyl group partially or completely, such as a hydroxycarboxymethyl group, a 1-hydroxy-2-carboxyethyl group, a 2-hydroxy-1-carboxyethyl group, a 1-hydroxy-2-carboxypropyl group, a 1,2-dihydroxy-2-carboxypropyl group, a 1,2-dihydroxy-3-carboxypropyl group, a 4-hydroxy-1-carboxybutyl group, a 1,2-dihydroxy-2-carboxybutyl group, a 1,2-dihydroxy-3-carboxybutyl group, a 1,2,3-trihydroxy-4-carboxybutyl group, a 4-hydroxy-1-carboxypentyl group, a 5-hydroxy-2-carboxypentyl group, a 1,2-dihydroxy-3-carboxypentyl group, a 1,2-dihydroxy-4-carboxypentyl group, a 1,2,2-trihydroxy-5-carboxypentyl group, a 5-hydroxy-1-carboxyhexyl group, a 1,2-dihydroxy-2-carboxyhexyl group, a 1,2-dihydroxy-3-carboxyhexyl group, a 1,2,2-trihydroxy-4-carboxyhexyl group, a 1,2,2-trihydroxy-5-carboxyhexyl group, a 2,3-dihydroxy-6-carboxyhexyl group,
hydroxycarboxyalkyl groups having the total carbon number of from 1 to 20 and substituted with a hydroxyl group and a carboxyl group partially or completely, such as a hydroxycarboxymethyl group, a 1-hydroxy-2-carboxyethyl group, a 2-hydroxy-1-carboxyethyl group, a 1-hydroxy-2-carboxypropyl group, a 1,2-dihydroxy-2-carboxypropyl group, a 1,2-dihydroxy-3-carboxypropyl group, a 4-hydroxy-1-carboxybutyl group, a 1,2-dihydroxy-2-carboxybutyl group, a 1,2-dihydroxy-3-carboxybutyl group, a 1,2,3-trihydroxy-4-carboxybutyl group, a 4-hydroxy-1-carboxypentyl group, a 5-hydroxy-2-carboxypentyl group, a 1,2-dihydroxy-3-carboxypentyl group, a 1,2-dihydroxy-4-carboxypentyl group, a 1,2,2-trihydroxy-5-carboxypentyl group, a 5-hydroxy-1-carboxyhexyl group, a 1,2-dihydroxy-2-carboxyhexyl group, a 1,2-dihydroxy-3-carboxyhexyl group, a 1,2,2-trihydroxy-4-carboxyhexyl group, a 1,2,2-trihydroxy-5-carboxyhexyl group, a 2,3-dihydroxy-6-carboxyhexyl group,
a 1-hydroxy-2,3-dicarboxypropyl group, a 2-hydroxy-3,4-dicarboxybutyl group, a 2,3-dihydroxy-3,5-dicarboxypentyl group, a 1,2,3-trihydroxy-5,6-dicarboxyhexyl group, a 2-hydroxy-3,4,4-tricarboxybutyl group, a 2,3-hydroxy-2,3,5-tricarboxypentyl group, a 5-hydroxy-2,3,6-tricarboxyhexyl group, a 5-hydroxy-2,3,4,4-tetracarboxypentyl group, a 2-hydroxy-2,4,6,6-tetracarboxyhexyl group and a 2,3-dihydroxy-2,3,4,5,6-pentacarboxyhexyl group,
sulfoalkyl groups having the total carbon number of from 1 to 20 and substituted with a sulfo group partially or completely, such as a sulfomethyl group, a 1-sulfoethyl group, a 2-sulfoethyl group, a 1-sulfopropyl group, a 2-sulfopropyl group, a 3-sulfopropyl group, a 1-sulfobutyl group, a 2-sulfobutyl group, a 3-sulfobutyl group, a 4-sulfobutyl group, a 1-sulfopentyl group, a 2-sulfopentyl group, a 3-sulfopentyl group, a 4-sulfopentyl group, a 5-sulfopentyl group, a 1-sulfohexyl group, a 2-sulfohexyl group, a 3-sulfohexyl group, a 4-sulfohexyl group, a 5-sulfohexyl group, a 6-sulfohexyl group,
a disulfopropyl group, a disulfobutyl group, a disulfopentyl group, a disulfohexyl group, a trisulfobutyl group, a trisulfopentyl group, a trisulfohexyl group, a tetrasulfopentyl group, a tetrasulfohexyl group and a pentasulfohexyl group,
hydroxysulfoalkyl groups having the total carbon number of from 1 to 20 and substituted with a hydroxyl group and a sulfo group, such as a hydroxysulfomethyl group, a 2-hydroxy-1-sulfoethyl group, a 1-hydroxy-2-sulfoethyl group, a 2,3-dihydroxy-1-sulfopropyl group, a 2,3-dihydroxy-2-sulfopropyl group, a 2-hydroxy-3-sulfopropyl group, a 4-hydroxy-1-sulfobutyl group, a 3,4-dihydroxy-2-sulfobutyl group, a 2,3,4-trihydroxy-3-sulfobutyl group, a 2,3,4-trihydroxy-4-sulfobutyl group, a 5-hydroxy-1-sulfopentyl group, a 2,5-dihydroxy-2-sulfopentyl group, a 2,5-dihydroxy-3-sulfopentyl group, a 1,2,5-trihydroxy-4-sulfopentyl group, a 1,2,3,4-tetrahydroxy-5-sulfopentyl group, a 6-hydroxy-1-sulfohexyl group, a 5-hydroxy-2-sulfohexyl group, a 6-hydroxy-3-sulfohexyl group, a 5,6-dihydroxy-4-sulfohexyl group, a 1,3,6-trihydroxy-5-sulfohexyl group, a 2,3,4,5-tetrahydroxy-6-sulfohexyl group,
a 1-hydroxy-2,3-disulfopropyl group, a 2-hydroxy-3, 4-disulfobutyl group, a 2,3,-dihydroxy-4,5-disulfopentyl group, a 2,3,3-trihydroxy-4,5-disulfohexyl group, a 2-hydroxy-2,3,4-trisulfobutyl group, a 2-hydroxy-3,4,5-trisulfopentyl group, a 2,4-dihydroxy-3,5,6-trisulfohexyl group, a 1,3-dihydroxy-2,4,5,5-tetrasulfopentyl group, a 1,3,6-trihydroxy-2,4,5,6-tetrasulfohexyl group and a 3,5-dihydroxy-1,2,3,4,6-pentasulfohexyl group,
carboxysulfoalkyl groups having the total carbon number of from 1 to 20 and substituted with a carboxyl group and a sulfo group, such as a carboxysulfomethyl group, a carboxysulfoethyl group, a carboxysulfopropyl group, a carboxysulfobutyl group, a carboxysulfopentyl group, a carboxysulfohexyl group, a dicarboxysulfoethyl group, a dicarboxysulfopropyl group, a dicarboxysulfobutyl group, a dicarboxysulfohexyl group, a carboxydisulfoethyl group,a carboxydisulfopropyl group, a carboxydisulfobutyl group, a carboxydisulfohexyl group and a dicarboxydisulfo group,
and hydroxycarboxysulfoalkyl groups having the total carbon number of from 1 to 20 and substituted with a carboxyl group and a sulfo group, such as a hydroxycarboxysulfoethyl group, a hydroxycarboxysulfopropyl group, a hydroxycarboxysulfobutyl group, a hydroxycarboxysulfopentyl group, a hydroxycarboxysulfohexyl group, a dihydroxycarboxysulfopropyl group, a dihydroxycarboxysulfobutyl group, a dihydroxycarboxysulfopentyl group, a dihydroxycarboxysulfohexyl group, a hydroxydicarboxysulfoethyl group, a hydroxydicarboxysulfopropyl group, a hydroxydicarboxysulfobutyl group, a hydroxydicarboxysulfohexyl group, a hydroxycarboxydisulfoethyl group, a hydroxycarboxydisulfopropyl group, a hydroxycarboxydisulfobutyl group, a hydroxycarboxydisulfohexyl group and a hydroxydicarboxydisulfo group.

Specific examples of the unsubstituted or substituted alkoxy group include linear or branched alkoxy groups such as amethoxy group, an ethoxy group, an n-propoxy group, an isopropoxy group, an n-butoxy group, an isobutoxy group, a tert-butoxy group, an n-pentyloxy group, an isopentyloxy group, an n-hexyloxy group, an isohexyloxy group, a 2-ethylhexyloxy group, a 3,5,5-trimethylhexyloxy group, an n-heptyloxy group, an n-octyloxy group and an n-nonyloxy group,
cycloalkoxy groups such as a cyclopentyloxy group and a cyclohexyloxy group,
alkoxyalkoxy groups such as a methoxymethoxy group, an ethoxymethoxy group, an ethoxyethoxy group, an n-propoxymethoxy group, an isopropoxymethoxy group, an n-propoxyethoxy group, an isopropoxyethoxy group, an n-butoxyethoxy group, an isobutoxyethoxy group, a tert-butoxyethoxy group, an n-pentyloxyethoxy group, an isopentyloxyethoxy group, an n-hexyloxyethoxy group, an isohexyloxyethoxy group, a 2-ethylhexyloxyethoxy group, a 3,5,5-trimethylhexyloxyethoxy group, an n-heptyloxyethoxy group, an n-octyloxyethoxy group and an n-nonyloxyethoxy group, and aralkyloxy groups such as a benzyloxy group.

Specific examples of the unsubstituted or substituted alkenyl group include monovalent unsaturated hydrocarbon groups having the total carbon number of 20 or less, such as a vinyl group, a 1-propenyl group, a 2-propenyl group, an isopropenyl group, a 2-butenyl group and a 2-pentenyl group, divalent unsaturated hydrocarbon groups having the total carbon number of 10 or less, such as a 1, 3-butadienyl group and a 2,4-pentadienyl group, and aryl-substituted unsaturated hydrocarbon groups having the total carbon number of 20 or less, such as a styryl group and a cinnamyl group. Specific examples of the unsubstituted or substituted alkyl group also include alkyl groups in which a part of carbon-carbon single bonds are replaced with carbon-carbon double bonds.

Specific examples of the unsubstituted or substituted aryl group include aromatic hydrocarbons having the total carbon number of 20 or less, such as a phenyl group, a naphthyl group and an anthranyl group,
alkyl-substituted aryl groups having the total carbon number of 20 or less, such as a 2-methylphenyl group, a 3-methylphenyl group, a 4-methylphenyl group, a 2-ethylphenyl group, a propylphenyl group, a butylphenyl group, a hexylphenyl group, a cyclohexylphenyl group, an octylphenyl group, a 2-methyl-1-naphthyl group, a 3-methyl-1-naphthyl group, a 4-methyl-1-naphthyl group, a 5-methyl-1-naphthyl group, a 6-methyl-1-naphthyl group, a 7-methyl-1-naphthyl group, a 8-methyl-1-naphthyl group, a 1-methyl-2-naphthyl group, a 3-methyl-2-naphthyl group, a 4-methyl-2-naphthyl group, a 5-methyl-2-naphthyl group, a 6-methyl-2-naphthyl group, a 7-methyl-2-naphthyl group, a 8-methyl-2-naphthyl group, a 2-ethyl-1-naphthyl group,
a 2,3-dimethylphenyl group, a 2,4-dimethylphenyl group, a 2,5-dimethylphenyl group, a 2,6-dimethylphenyl group, a 3,4-dimethylphenyl group, a 3,5-dimethylphenyl group, a 3,6-dimethylphenyl group,
a 2,3,4-trimethylphenyl group, a 2,3,5-trimethylphenyl group, a 2,3,6-trimethylphenyl group, a 2,4,5-trimethylphenyl group, a 2,4,6-trimethylphenyl group and a 3,4,5-trimethylphenyl group,
aryl groups having the total carbon number of 20 or less and substituted with an unsubstituted or substituted aryl group such as a biphenyl group, a styrylphenyl group and a stilbenyl group,
hydroxyaryl groups having the total carbon number of 20 or less, such as a 2-hydroxyphenyl group, a 3-hydroxyphenyl group, a 4-hydroxyphenyl group, a 2-hydroxy-1-naphthyl group, a 3-hydroxy-1-naphthyl group, a 4-hydroxy-1-naphthyl group, a 5-hydroxy-1-naphthyl group, a 6-hydroxy-1-naphthyl group, a 7-hydroxy-1-naphthyl group, a 8-hydroxy-1-naphthyl group, a 1-hydroxy-2-naphthyl group, a 3-hydroxy-2-naphthyl group, a 4-hydroxy-2-naphthyl group, a 5-hydroxy-2-naphthyl group, a 6-hydroxy-2-naphthyl group, a 7-hydroxy-2-naphthyl group, a 8-hydroxy-2-naphthyl group,
a 2,3-dihydroxyphenyl group, a 2,4-dihydroxyphenyl group, a 2,5-dihydroxyphenyl group, a 2,6-dihydroxyphenyl group, a 3,4-dihydroxyphenyl group, a 3,5-dihydroxyphenyl group, a 3,6-dihydroxyphenyl group, a 2,3-dihydroxy-1-naphthyl group, a 2,4-dihydroxy-1-naphthyl group, a 2,6-dihydroxy-1-naphthyl group, a 2,7-dihydroxy-1-naphthyl group, a 2,8-dihydroxy-1-naphthyl group, a 3,4-dihydroxy-1-naphthyl group, a 3,5-dihydroxy-1-naphthyl group, a 3,6-dihydroxy-1-naphthyl group, a 3,7-dihydroxy-1-naphthyl group, a 3,8-dihydroxy-1-naphthyl group, a 4,5-dihydroxy-1-naphthyl group, a 4,6-dihydroxy-1-naphthyl group, a 4,7-dihydroxy-1-naphthyl group, a 4,8-dihydroxy-1-naphthyl group, a 5,6-dihydroxy-1-naphthyl group, a 5,7-dihydroxy-1-naphthyl group, a 5,8-dihydroxy-1-naphthyl group, a 6,7-dihydroxy-1-naphthyl group, a 6,8-dihydroxy-1-naphthyl group, a 7,8-dihydroxy-1-naphthyl group, a 1,3-dihydroxy-2-naphthyl group, a 1,4-dihydroxy-2-naphthyl group, a 1,6-dihydroxy-2-naphthyl group, a 1,7-dihydroxy-2-naphthyl group, a 1,8-dihydroxy-2-naphthyl group, a 3,4-dihydroxy-2-naphthyl group, a 3,5-dihydroxy-2-naphthyl group, a 3,6-dihydroxy-2-naphthyl group, a 3,7-dihydroxy-2-naphthyl group, a 3,8-dihydroxy-2-naphthyl group, a 4,5-dihydroxy-2-naphthyl group, a 4,6-dihydroxy-2-naphthyl group, a 4,7-dihydroxy-2-naphthyl group, a 4,8-dihydroxy-2-naphthyl group, a 5,6-dihydroxy-2-naphthyl group, a 5,7-dihydroxy-2-naphthyl group, a 5,8-dihydroxy-2-naphthyl group, a 6,7-dihydroxy-2-naphthyl group, a 6,8-dihydroxy-2-naphthyl group and a 7,8-dihydroxy-2-naphthyl group,
carboxyaryl groups having the total carbon number of 20 or less, such as a 2-carboxyophenyl group, a 3-carboxyphenyl group, a 4-carboxyphenyl group, a 2,3-dicarboxyphenyl group, a 2,4-dicarboxyphenyl group, a 2,5-dicarboxyphenyl group, a 2,6-dicarboxyphenyl group, a 3,4-dicarboxyphenyl group, a 3,5-dicarboxyphenyl group, a 3,6-dicarboxyphenyl group,
a 2,3,4-tricarboxyphenyl group, a 2,3,5-tricarboxyphenyl group, a 2,3,6-tricarboxyphenyl group, a 2,4,5-tricarboxyphenyl group, a 2,4,6-tricarboxyphenyl group, a 3,4,5-tricarboxyphenyl group, a 4-carboxy-1-naphthyl group, a 5-carboxy-1-naphthyl group, a 6-carboxy-1-naphthyl group, a 7-carboxy-1-naphthyl group, a 8-carboxy-1-naphthyl group, a 1-carboxy-2-naphthyl group, a 3-carboxy-2-naphthyl group, a 4-carboxy-2-naphthyl group, a 5-carboxy-2-naphthyl group, a 6-carboxy-2-naphthyl group, a 7-carboxy-2-naphthyl group and a 8-carboxy-2-naphthyl group,
monohydroxymonocarboxyaryl groups having the carbon number of 10 or less, such as a 2-hydroxy-3-carboxyphenyl group, a 2-hydroxy-4-carboxyphenyl group, a 2-hydroxy-5-carboxyphenyl group, a 2-hydroxy-6-carboxyphenyl group, a 3-hydroxy-2-carboxyphenyl group, a 3-hydroxy-4-carboxyphenyl group, a 3-hydroxy-5-carboxyphenyl group, a 3-hydroxy-6-carboxyphenyl group, a 4-hydroxy-2-carboxyphenyl group and a 4-hydroxy-3-carboxyphenyl group,
dihydroxymonocarboxyaryl groups having the total carbon number of 20 or less, such as a 2,3-dihydroxy-4-carboxyphenyl group, a 2,3-dihydroxy-5-carboxyphenyl group, a 2,3-dihydroxy-6-carboxyphenyl group, a 2,4-dihydroxy-3-carboxyphenyl group, a 2,4-dihydroxy-5-carboxyphenyl group, a 2,4-dihydroxy-6-carboxyphenyl group, a 2,5-dihydroxy-3-carboxyphenyl group, a 2,5-dihydroxy-4-carboxyphenyl group, a 2,5-dihydroxy-6-carboxyphenyl group, a 2,6-dihydroxy-3-carboxyphenyl group, a 2,6-dihydroxy-4-carboxyphenyl group, a 3,4-dihydroxy-2-carboxyphenyl group, a 3,4-dihydroxy-5-carboxyphenyl group, a 3,4-dihydroxy-6-carboxyphenyl group, a 3,5-dihydroxy-2-carboxyphenyl group, a 3,5-dihydroxy-4-carboxyphenyl group, a 3,6-dihydroxy-2-carboxyphenyl group, a 3,6-dihydroxy-4-carboxyphenyl group and a 3,6-dihydroxy-5-carboxyphenyl group,
monohydroxydicarboxyaryl groups having the total carbon number of 20 or less, such as a 2,3-dicarboxy-4-hydroxyphenyl group, a 2,3-dicarboxy-5-hydroxyphenyl group, a 2,3-dicarboxy-6-hydroxyphenyl group, a 2,4-dicarboxy-3-hydroxyphenyl group, a 2,4-dicarboxy-5-hydroxyphenyl group, a 2,4-dicarboxy-6-hydroxyphenyl group, a 2,5-dicarboxy-3-hydroxyphenyl group, a 2,5-dicarboxy-4-hydroxyphenyl group, a 2,5-dicarboxy-6-hydroxyphenyl group, a 2,6-dicarboxy-3-hydroxyphenyl group, a 2,6-dicarboxy-4-hydroxyphenyl group, a 3,4-dicarboxy-2-hydroxyphenyl group, a 3,4-dicarboxy-5-hydroxyphenyl group, a 3,4-dicarboxy-6-hydroxyphenyl group, a 3,5-dicarboxy-2-hydroxyphenyl group, a 3,5-dicarboxy-4-hydroxyphenyl group, a 3,6-dicarboxy-2-hydroxyphenyl group, a 3,6-dicarboxy-4-hydroxyphenyl group and a 3,6-dicarboxy-5-hydroxyphenyl group,
sulfoaryl groups having the total carbon number of 20 or less, such as a 2-sulfophenyl group, a 3-sulfophenyl group, a 4-sulfophenyl group, a 2-sulfo-1-naphthyl group, a 3-sulfo-1-naphthyl group, a 4-sulfo-1-naphthyl group, a 5-sulfo-1-naphthyl group, a 6-sulfo-1-naphthyl group, a 7-sulfo-1-naphthyl group, a 8-sulfo-1-naphthyl group, a 1-sulfo-2-naphthyl group, a 3-sulfo-2-naphthyl group, a 4-sulfo-2-naphthyl group, a 5-sulfo-2-naphthyl group, a 6-sulfo-2-naphthyl group, a 7-sulfo-2-naphthyl group, a 8-sulfo-2-naphthyl group,
a 2,3-disulfophenyl group, a 2,4-disulfophenyl group, a 2,5-disulfophenyl group, a 2,6-disulfophenyl group, a 3,4-disulfophenyl group, a 3,5-disulfophenyl group, a 3,6-disulfophenyl group, a 2,3-disulfo-1-naphthyl group, a 2,4-disulfo-1-naphthyl group, a 2,6-disulfo-1-naphthyl group, a 2,7-disulfo-1-naphthyl group, a 2,8-disulfo-1-naphthyl group, a 3,4-disulfo-1-naphthyl group, a 3,5-disulfo-1-naphthyl group, a 3,6-disulfo-1-naphthyl group, a 3,7-disulfo-1-naphthyl group, a 3,8-disulfo-1-naphthyl group, a 4,5-disulfo-1-naphthyl group, a 4,6-disulfo-1-naphthyl group, a 4,7-disulfo-1-naphthyl group, a 4,8-disulfo-1-naphthyl group, a 5,6-disulfo-1-naphthyl group, a 5,7-disulfo-1-naphthyl group, a 5,8-disulfo-1-naphthyl group, a 6,7-disulfo-1-naphthyl group, a 6,8-disulfo-1-naphthyl group, a 7,8-disulfo-1-naphthyl group, a 1,3-disulfo-2-naphthyl group, a 1,4-disulfo-2-naphthyl group, a 1,6-disulfo-2-naphthyl group, a 1,7-disulfo-2-naphthyl group, a 1,8-disulfo-2-naphthyl group, a 3,4-disulfo-2-naphthyl group, a 3,5-disulfo-2-naphthyl group, a 3,6-disulfo-2-naphthyl group, a 3,7-disulfo-2-naphthyl group, a 3,8-disulfo-2-naphthyl group, a 4,5-disulfo-2-naphthyl group, a 4,6-disulfo-2-naphthyl group, a 4,7-disulfo-2-naphthyl group, a 4,8-disulfo-2-naphthyl group, a 5,6-disulfo-2-naphthyl group, a 5,7-disulfo-2-naphthyl group, a 5,8-disulfo-2-naphthyl group, a 6,7-disulfo-2-naphthyl group, a 6, 8-disulfo-2-naphthyl group, a 7,8-disulfo-2-naphthyl group,
a 2,3,4-trisulfophenyl group, a 2,3,5-trisulfophenyl group, a 2,3,6-trisulfophenyl group, a 2,4,5-trisulfophenyl group, a 2,4,6-trisulfophenyl group, a 3,4,5-trisulfophenyl group, a trisulfo-1-naphthyl group and a trisulfo-2-naphthyl group,
monohydroxymonosulfoaryl groups having the total carbon number of 20 or less, such as a 2-hydroxy-3-sulfophenyl group, a 2-hydroxy-4-sulfophenyl group, a 2-hydroxy-5-sulfophenyl group, a 2-hydroxy-6-sulfophenyl group, a 3-hydroxy-2-sulfophenyl group, a 3-hydroxy-4-sulfophenyl group, a 3-hydroxy-5-sulfophenyl group, a 3-hydroxy-6-sulfophenyl group, a 4-hydroxy-2-sulfophenyl group, a 4-hydroxy-3-sulfophenyl group, a hydroxy-2-sulfo-1-naphthyl group, a hydroxy-3-sulfo-1-naphthyl group, a hydroxy-4-sulfo-1-naphthyl group, a hydroxy-5-sulfo-1-naphthyl group, a hydroxy-6-sulfo-1-naphthyl group, a hydroxy-7-sulfo-1-naphthyl group, a hydroxy-8-sulfo-1-naphthyl group, a hydroxy-1-sulfo-2-naphthyl group, a hydroxy-3-sulfo-2-naphthyl group, a hydroxy-4-sulfo-2-naphthyl group, a hydroxy-5-sulfo-2-naphthyl group, a hydroxy-6-sulfo-2-naphthyl group, a hydroxy-7-sulfo-2-naphthyl group and a hydroxy-8-sulfo-2-naphthyl group,
dihydroxymonosulfoaryl groups having the total carbon number of 20 or less, such as a 2,3-dihydroxy-4-sulfophenyl group, a 2,3-dihydroxy-5-sulfophenyl group, a 2,3-dihydroxy-6-sulfophenyl group, a 2,4-dihydroxy-3-sulfophenyl group, a 2,4-dihydroxy-5-sulfophenyl group, a 2,4-dihydroxy-6-sulfophenyl group, a 2,5-dihydroxy-3-sulfophenyl group, a 2,5-dihydroxy-4-sulfophenyl group, a 2,5-dihydroxy-6-sulfophenyl group, a 2,6-dihydroxy-3-sulfophenyl group, a 2,6-dihydroxy-4-sulfophenyl group, a 3,4-dihydroxy-2-sulfophenyl group, a 3,4-dihydroxy-5-sulfophenyl group, a 3,4-dihydroxy-6-sulfophenyl group, a 3,5-dihydroxy-2-sulfophenyl group, a 3,5-dihydroxy-4-sulfophenyl group, a 3,6-dihydroxy-2-sulfophenyl group, a 3,6-dihydroxy-4-sulfophenyl group, a 3,6-dihydroxy-5-sulfophenyl group, a dihydroxy-2-sulfo-1-naphthyl group, a dihydroxy-3-sulfo-1-naphthyl group, a dihydroxy-4-sulfo-1-naphthyl group, a dihydroxy-5-sulfo-1-naphthyl group, a dihydroxy-6-sulfo-1-naphthyl group, a dihydroxy-7-sulfo-1-naphthyl group, a dihydroxy-8-sulfo-1-naphthyl group, a dihydroxy-1-sulfo-2-naphthyl group, a dihydroxy-3-sulfo-2-naphthyl group, a dihydroxy-4-sulfo-2-naphthyl group, a dihydroxy-5-sulfo-2-naphthyl group, a dihydroxy-6-sulfo-2-naphthyl group, a dihydroxy-7-sulfo-2-naphthyl group and a dihydroxy-8-sulfo-2-naphthyl group,
monohydroxydisulfoaryl groups having the total carbon number of 20 or less, such as a hydroxy-3,6-disulfo-1-naphthyl group, a hydroxy-3,7-disulfo-1-naphthyl group, a hydroxy-4,5-disulfo-1-naphthyl group, a hydroxy-4,7-disulfo-1-naphthyl group, a hydroxy-4,8-disulfo-1-naphthyl group, a hydroxy-5,8-disulfo-1-naphthyl group, a hydroxy-6,7-disulfo-1-naphthyl group, a hydroxy-3,6-disulfo-2-naphthyl group, a hydroxy-3,7-disulfo-2-naphthyl group, a hydroxy-4,5-disulfo-2-naphthyl group, a hydroxy-4,7-disulfo-2-naphthyl group, a hydroxy-4,8-disulfo-2-naphthyl group, a hydroxy-5,8-disulfo-2-naphthyl group and a hydroxy-6,7-disulfo-2-naphthyl group,
carboxysulfoaryl groups having the total carbon number of 20 or less, such as a carboxysulfophenyl group, a carboxydisulfophenyl group, a dicarboxysulfophenyl group, a dicarboxydisulfophenyl group, a carboxysulfonaphthyl group, a carboxydisulfonaphthyl group, a dicarboxysulfonaphthyl group and a dicarboxydisulfonaphthyl group,
carboxysulfoaryl groups having the total carbon number of 20 or less, such as a hydroxycarboxysulfophenyl group, a hydroxycarboxydisulfophenyl group, a hydroxydicarboxysulfophenyl group, a hydroxydicarboxydisulfophenyl group, a dihydroxycarboxysulfophenyl group, a hydroxycarboxysulfonaphthyl group, a hydroxycarboxydisulfonaphthyl group, a hydroxydicarboxysulfonaphthyl group, a hydroxydicarboxydisulfonaphthyl group and a dihydroxycarboxysulfonaphthyl group,
monoalkoxyaryl groups having the total carbon number of 20 or less and substituted with unsubstituted or substituted alkyloxy having 10 or less carbon atoms, such as a 2-methoxyphenyl group, a 3-methoxyphenyl group, a 4-methoxyphenyl group, a 2-ethoxyphenyl group, a propoxyphenyl group, a butoxyphenyl group, a hexyloxyphenyl group, a cyclohexyloxyphenyl group, an octyloxyphenyl group, a 2-methoxy-1-naphthyl group, a 3-methoxy-1-naphthyl group, a 4-methoxy-1-naphthyl group,a 5-methoxy-1-naphthyl group, a 6-methoxy-1-naphthyl group, a 7-methoxy-1-naphthyl group, a 8-methoxy-1-naphthyl group, a 1-methoxy-2-naphthyl group, a 3-methoxy-2-naphthyl group, a 4-methoxy-2-naphthyl group, a 5-methoxy-2-naphthyl group, a 6-methoxy-2-naphthyl group, a 7-methoxy-2-naphthyl group, a 8-methoxy-2-naphthyl group and a 2-ethoxy-1-naphthyl group,
dialkoxyaryl groups having the total carbon number of 20 or less and substituted with unsubstituted or substituted alkyloxy having 10 or less carbon atoms, such as a 2,3-dimethoxyphenyl group, a 2,4-dimethoxyphenyl group, a 2,5-dimethoxyphenyl group, a 2,6-dimethoxyphenyl group, a 3,4-dimethoxyphenyl group, a 3,5-dimethoxyphenyl group, a 3,6-dimethoxyphenyl group, a 4,5-dimethoxy-1-naphthyl group, a 4,7-dimethoxy-1-naphthyl group, a 4,8-dimethoxy-1-naphthyl group, a 5,8-dimethoxy-1-naphthyl group and a 5,8-dimethoxy-2-naphthyl group,
trialkoxyaryl groups having the total carbon number of 20 or less and substituted with unsubstituted or substituted alkyloxy having 10 or less carbon atoms, such as a 2,3,4-trimethoxyphenyl group, a 2,3,5-trimethoxyphenyl group, a 2,3,6-trimethoxyphenyl group, a 2,4,5-trimethoxyphenyl group, a 2,4,6-trimethoxyphenyl group and a 3,4,5-trimethoxyphenyl group,

Aryl groups having the total carbon number of 20 or less and substituted with a halogen atom, such as a chlorophenyl group, a dichlorophenyl group, a trichlorophenyl group, a bromophenyl group, a dibromophenyl group, an iodophenyl group, a fluorophenyl group, a chloronaphthyl group, a bromonaphthyl group, a difluorophenyl group, a trifluorophenyl group, a tetrafluorophenyl group and a pentafluorophenyl group,
halogenated alkylaryl groups having the total carbon number of 20 or less and substituted with a halogen alkyl group having the total carbon number of 10 or less and substituted partially or completely with a halogen atom, such as a trif luoromethylphenyl group and a trichloromethylphenyl group,
monoaminoaryl groups having the total carbon number of 20 or less, such as a 2-aminophenyl group, a 3-aminophenyl group, a 4-aminophenyl group, a 2-amino-1-naphthyl group, a 3-amino-1-naphthyl group, a 4-amino-1-naphthyl group, a 5-amino-1-naphthyl group, a 6-amino-1-naphthyl group, a 7-amino-1-naphthyl group, a 8-amino-1-naphthyl group, a 1-amino-2-naphthyl group, a 3-amino-2-naphthyl group, a 4-amino-2-naphthyl group, a 5-amino-2-naphthyl group, a 6-amino-2-naphthyl group, a 7-amino-2-naphthyl group and a 8-amino-2-naphthyl group,
diaminoaryl groups having the total carbon number of 20 or less, such as a 2,3-diaminophenyl group, a 2,4-diaminophenyl group, a 2,5-diaminophenyl group, a 2,6-diaminophenyl group, a 3,4-diaminophenyl group, a 3,5-diaminophenyl group, a 3,6-diaminophenyl group, a diamino-1-naphthyl group and a diamino-2-naphthyl group,
N-monosubstituted amino-substituted aryl groups having the total carbon number of 20 or less, such as an N-methylaminophenyl group, an N-ethylaminophenyl group, an N-phenylaminophenyl group, an N-tolylaminophenyl group and an N-cyclohexylaminophenyl group,
N,N-disubstituted-amino-substituted aryl groups having the total carbon number of 20 or less, such as an N,N-dimethylaminophenyl group, an N,N-diethylaminophenyl group, an N-phenyl-N-methylaminophenyl group, an N-tolyl-N-ethylaminophenyl group, an N-chlorophenyl-N-cyclohexylaminophenyl group and an N,N-ditolylaminophenyl group,
aminosulfoaryl groups having the total carbon number of 20 or less, such as a 3-amino-2-sulfophenyl group, a 4-amino-2-sulfophenyl group, a 5-amino-2-sulfophenyl group, a 6-amino-2-sulfophenyl group, a 2-amino-3-sulfophenyl group, a 4-amino-3-sulfophenyl group, a 5-amino-3-sulfophenyl group, a 6-amino-3-sulfophenyl group, a 2-amino-4-sulfophenyl group and a 3-amino-4-sulfophenyl group, and
aminocarboxyaryl groups having the total carbon number of 20 or less, such as a 3-amino-2-carboxyphenyl group, a 4-amino-2-carboxyphenyl group, a 5-amino-2-carboxyphenyl group, a 6-amino-2-carboxyphenyl group, a 2-amino-3-carboxyphenyl group, a 4-amino-3-carboxyphenyl group, a 5-amino-3-carboxyphenyl group, a 6-amino-3-carboxyphenyl group, a 2-amino-4-carboxyphenyl group and a 3-amino-4-carboxyphenyl group. Further, alkylthioaryl groups and arylthioaryl groups such as a methylthiophenyl group, an ethylthiophenyl group, a methylthionaphthyl group and a phenylthiophenyl group are listed.

Specific examples of the unsubstituted or substituted alkylene group having the total carbon number of 20 or less, which include linear or branched alkylene groups having the total carbon number of 20 or less,
linear alkylene groups having the total carbon number of 20 or less, such as an ethylene group, a trimethylene group, a tetramethylene group, a pentamethylene group, a hexamethylene group, a heptamethylene group and an octamethylene group, and
branched alkylene groups having the total carbon number of 20 or less, such as a propylene group and an ethylethylene group.

Specific examples of the unsubstituted or substituted heterocyclic group include heterocyclic aliphatic hydrocarbons having the total carbon number of 20 or less, such as a tetrahydrofuryl group, a tetrahydropiranyl group, a pyrrolidinyl group, a pyrrolinyl group, an imidazolidinyl group, an imidazolinyl group, a pyrazolidinyl group, a pyrazolinyl group, a piperidyl group, a piperazinyl group and a morpholinyl group,
heterocyclic aromatic hydrocarbons having the total carbon number of 20 or less, such as a thienyl group, a furyl group, a pyranyl group, an isobenzofuranyl group, a chromenyl group, a pyrrolyl group, an imidazolyl group, a pyrazolyl group, an isothiazolyl group, an isoxazolyl group, a pyridyl group, a pyrazinyl group, a pyrimidyl group, a pyridazinyl group, a triazinyl group, an indolizinyl group, an isoindolyl group, an indolyl group, an indazolyl group, a quinolyl group and an isoquinolyl group,
heterocyclic aliphatic or aromatic hydrocarbons having the total carbon number of 20 or less and substituted with an alkyl group or an aryl group having 10 or less carbon atoms, such as a methylthienyl group,
a methylfuryl group, a methylpiranyl group, a methylimidazolyl group and a phenylpyrazolyl group,
heterocyclic aliphatic or aromatic hydrocarbons having the total carbon number of 20 or less and substituted with a hydroxy group, such as a hydroxypyridyl group, a hydroxypyrazolyl group, a hydroxyimidazolyl group and a dihydroxytriazyl group,
heterocyclic aliphatic or aromatic hydrocarbons having the total carbon number of 20 or less and substituted with a cyano group, such as a cyanopyridyl group and a dicyanoimidazolyl,
heterocyclic aliphatic or aromatic hydrocarbons having the total carbon number of 20 or less and substituted with a halogen atom, such as a dichlorotriazyl group and a chloropyridyl group, and
heterocyclic aliphatic or aromatic hydrocarbons having the total carbon number of 20 or less and substituted with a carboxy group, such as a carboxypyridyl group and a carboxypyrazolyl group.

The compounds or the salts thereof contained in the aqueous ink of the invention are compounds having the structure represented by general formula (1) in the molecule or salts thereof, and further compounds having two or more structures represented by general formula (1) in the form of the structure represented by general formula (2) or salts thereof. The compounds having the structure represented by general formula (1) in the molecule as here referred to also include compounds having plural structures represented by general formula (1), namely, compounds in which structures represented by general formula (1) are bonded through a divalent or higher bonding group. As the compounds, the compounds having two or more groups represented by general formula (2) in the molecule are specifically shown. More specifically, compounds having two to four groups represented by general formula (2) in a molecule, as represented by general formulas (3) to (5), can be listed.

The compounds in which the structures are bonded to each other in the positions of R₇ in general formula (1) have been here shown. However, the compounds in which the structures are bonded to each other in the positions of R₁ to R₅, for example, the compounds in which the structures are bonded to each other in the different positions of R₁ and R₇, are also included in the invention. Further, the compounds having five or more structures represented by general formula (1) in the molecule are naturally included in the invention. 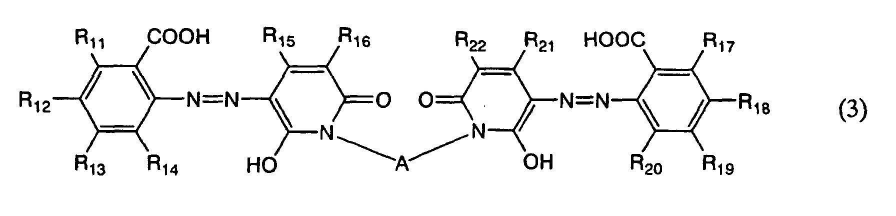 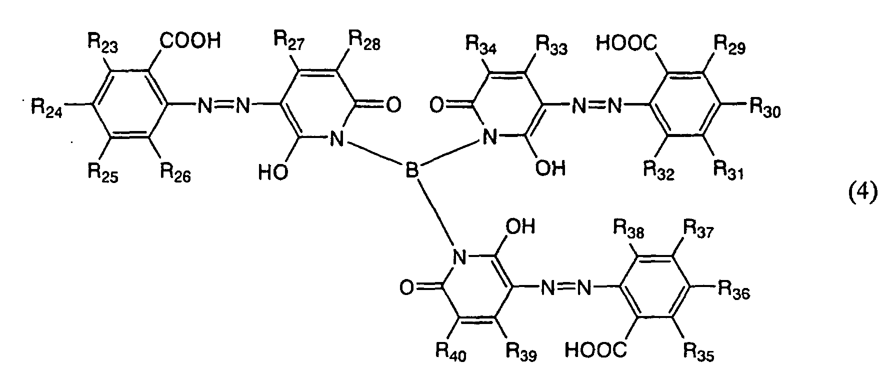 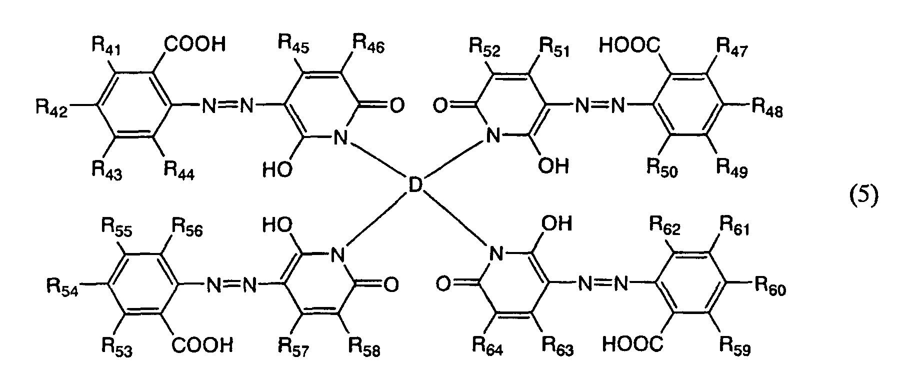 wherein
each of R₁₁ to R₁₅, R₁₇ to R₂₁, R₂₃ to R₂₇, R₂₉ to R₃₃, R₃₅ to R₃₉, R₄₁ to R₄₅, R₄₇ to R₅₁, R₅₃ to R₅₇ and R₅₉ to R₆₃ independently, represents the same as each of R₁ to R₅ in general formula (1), each of R₁₆, R₂₂, R₂₈, R₃₄, R₄₀, R₄₆, R₅₂, R₅₈ and R₆₄ independently, represent the same as R₆ in general formula (1), A represents a divalent group, B represents a trivalent group, and D represents a tetravalent group.

Specific examples of the bonding group in the invention are listed below.

Specific examples of the divalent bonding group include divalent groups of formulas (6) to (36). Preferable examples, in view of the light fastness, include divalent groups of formulas (10) to (21), (24) to (31), (34) and (35). More preferable examples include divalent groups of formulas (10), (12), (34) and (35). However, the divalent bonding group in the invention is not limited to these specific examples.

In formulas (6) to (36), each of m and n independently, represents an integer of 20 or less, preferably 15 or less, more preferably 10 or less.

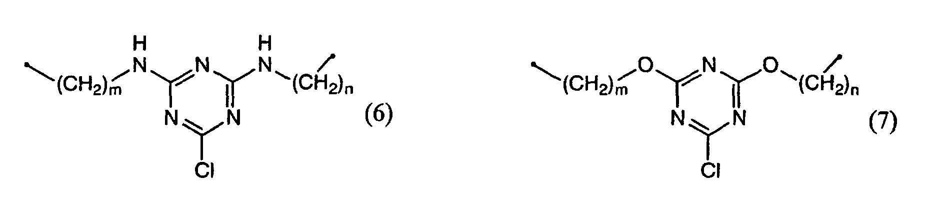

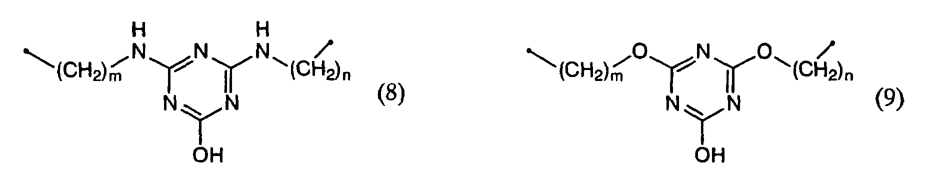

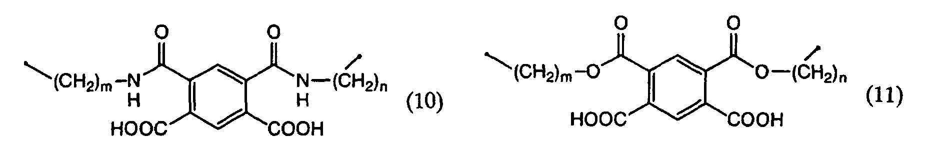

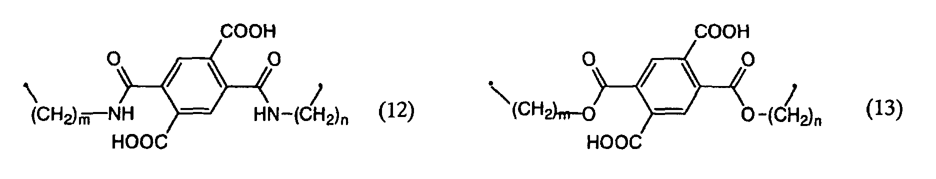

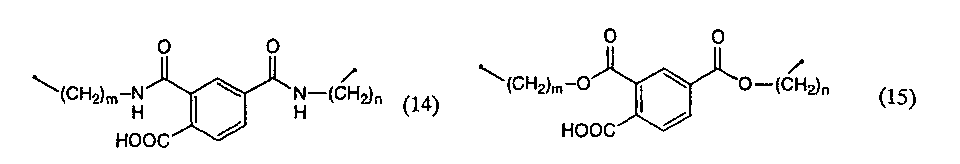

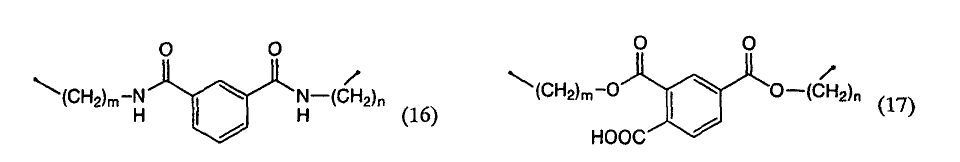

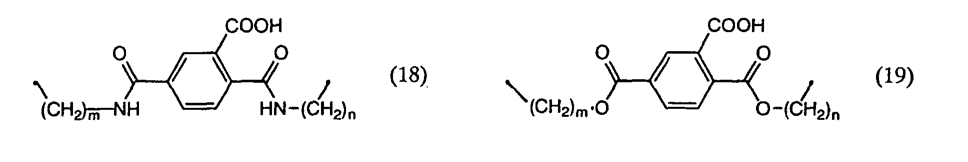

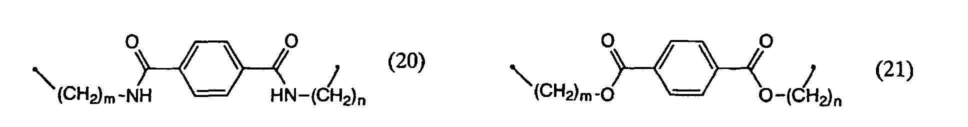

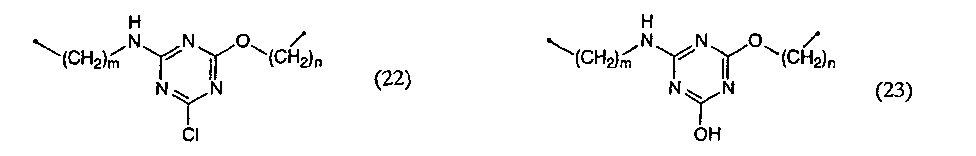

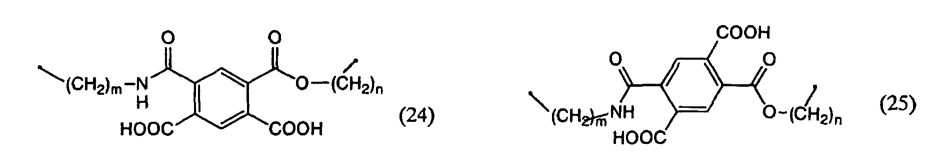

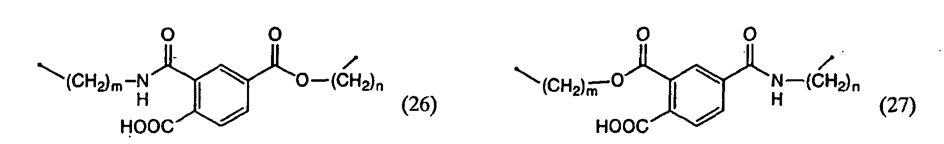

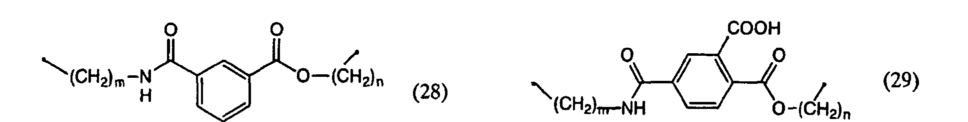

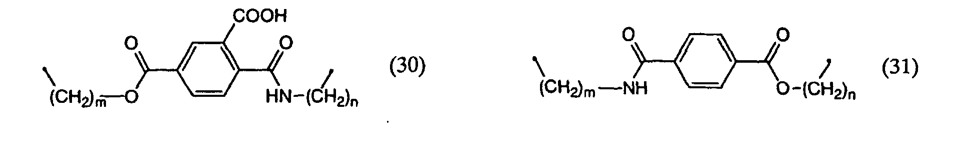

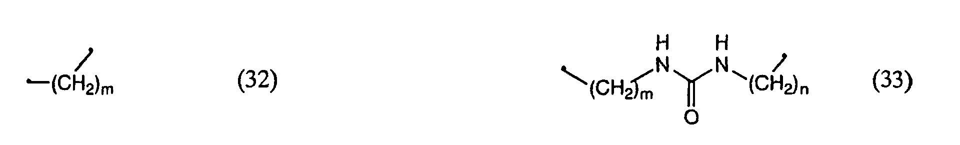

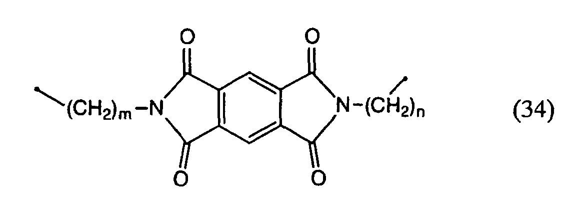

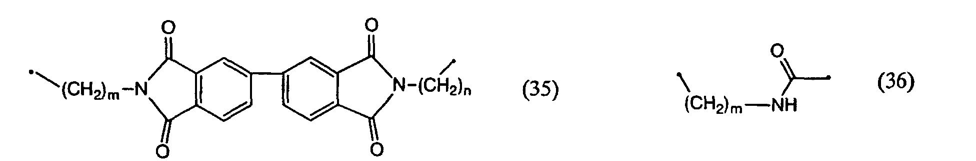

Specific examples of the trivalent bonding group include trivalent groups of formulas (37) to (47). However, the trivalent bonding group is not limited to these groups.

In formulas (37) to (47), each of m, n and k independently, represents an integer of 20 or less, preferably 15 or less, more preferably 10 or less.

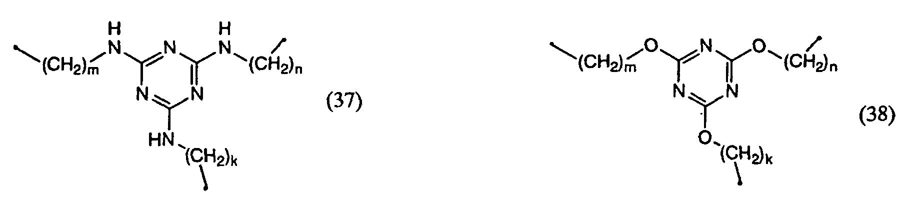

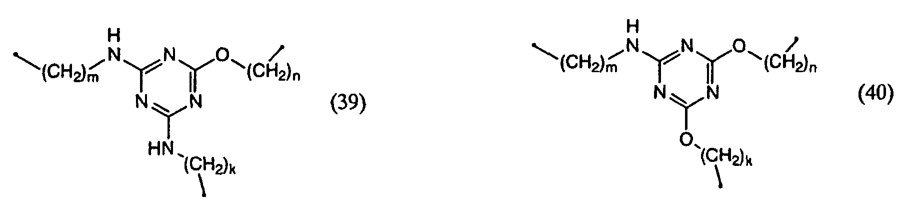

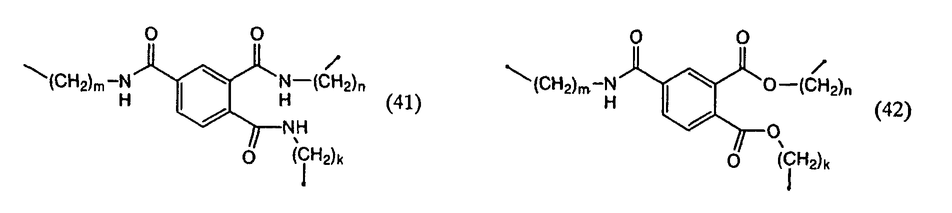

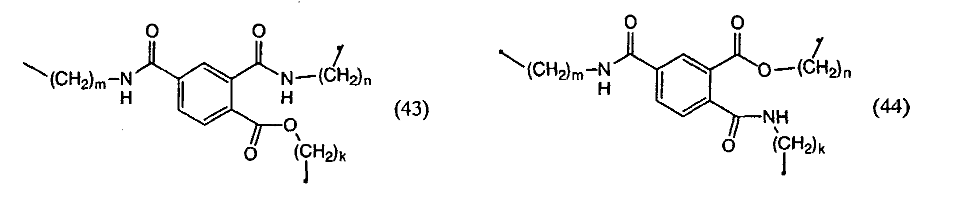

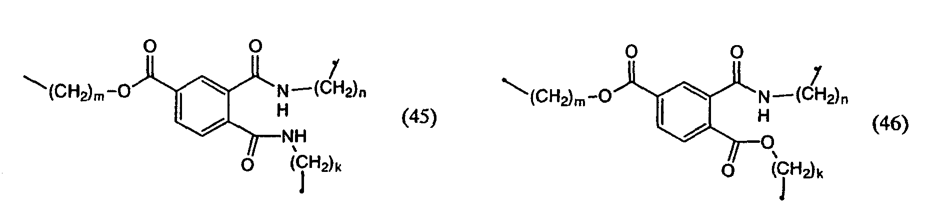

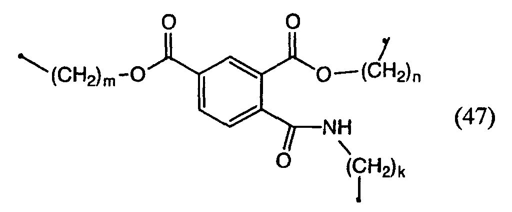

Specific examples of the tetravalent bonding group include trivalent groups of formulas (48) to (54). However, the trivalent bonding group is not limited to these groups. In formulas (48) to (54), each of j, k, m and n independently, represents an integer of 20 or less, preferably 15 or less, more preferably 10 or less.

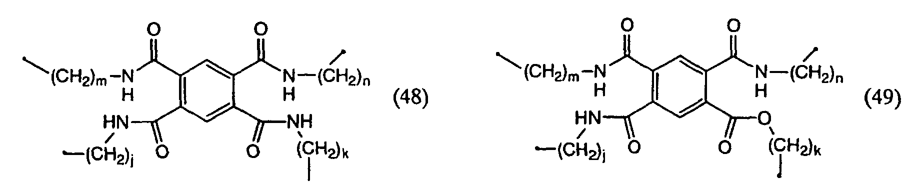

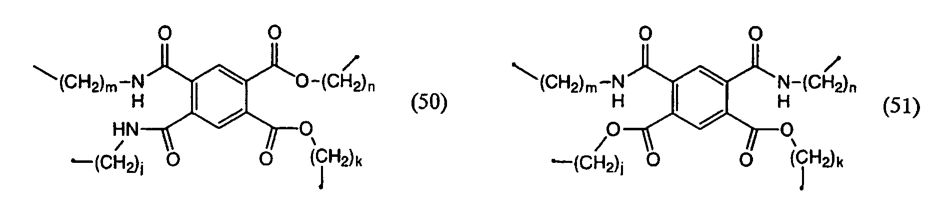

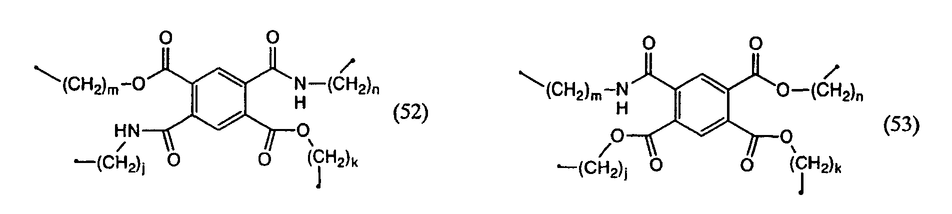

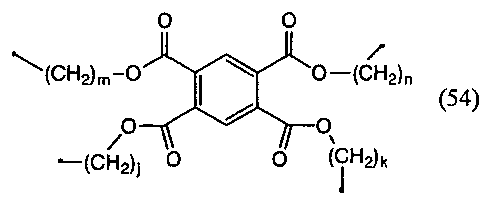

Of the foregoing examples of the divalent to tetravalent bonding groups, the bonding groups having the phenyl structure are preferable in view of the light fastness.

Specific examples of the compounds having the structure represented by general formula (1) in the molecule or the salts thereof in the invention are shown in Tables 1 and 2. In Table 1, M₁ to M₄ represent a hydrogen atom, an alkali metal, an alkaline earth metal or ammonium. The compounds represented by general formula (1) are not limited to the compounds and the salts thereof in Tables 1 and 2.

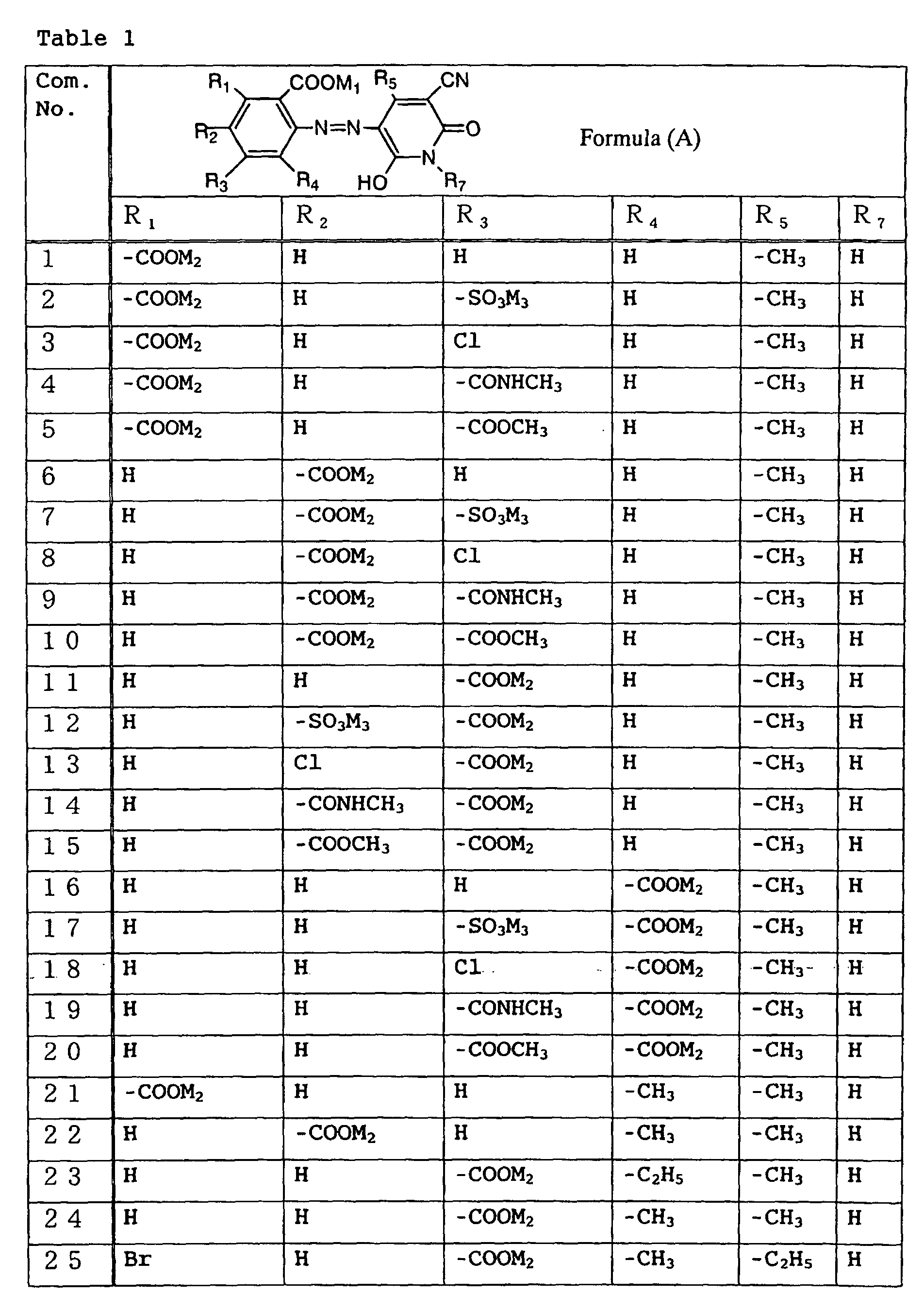

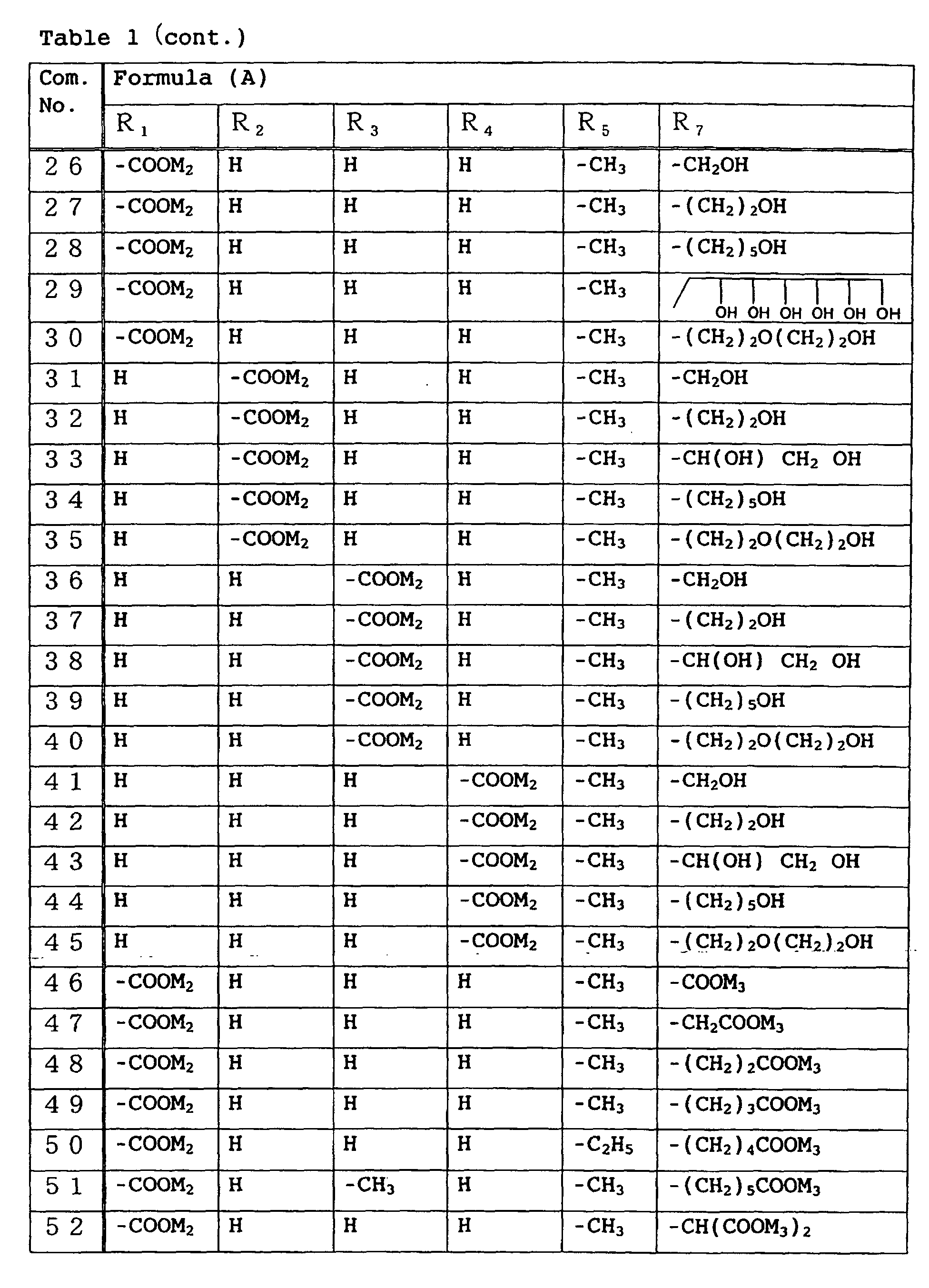

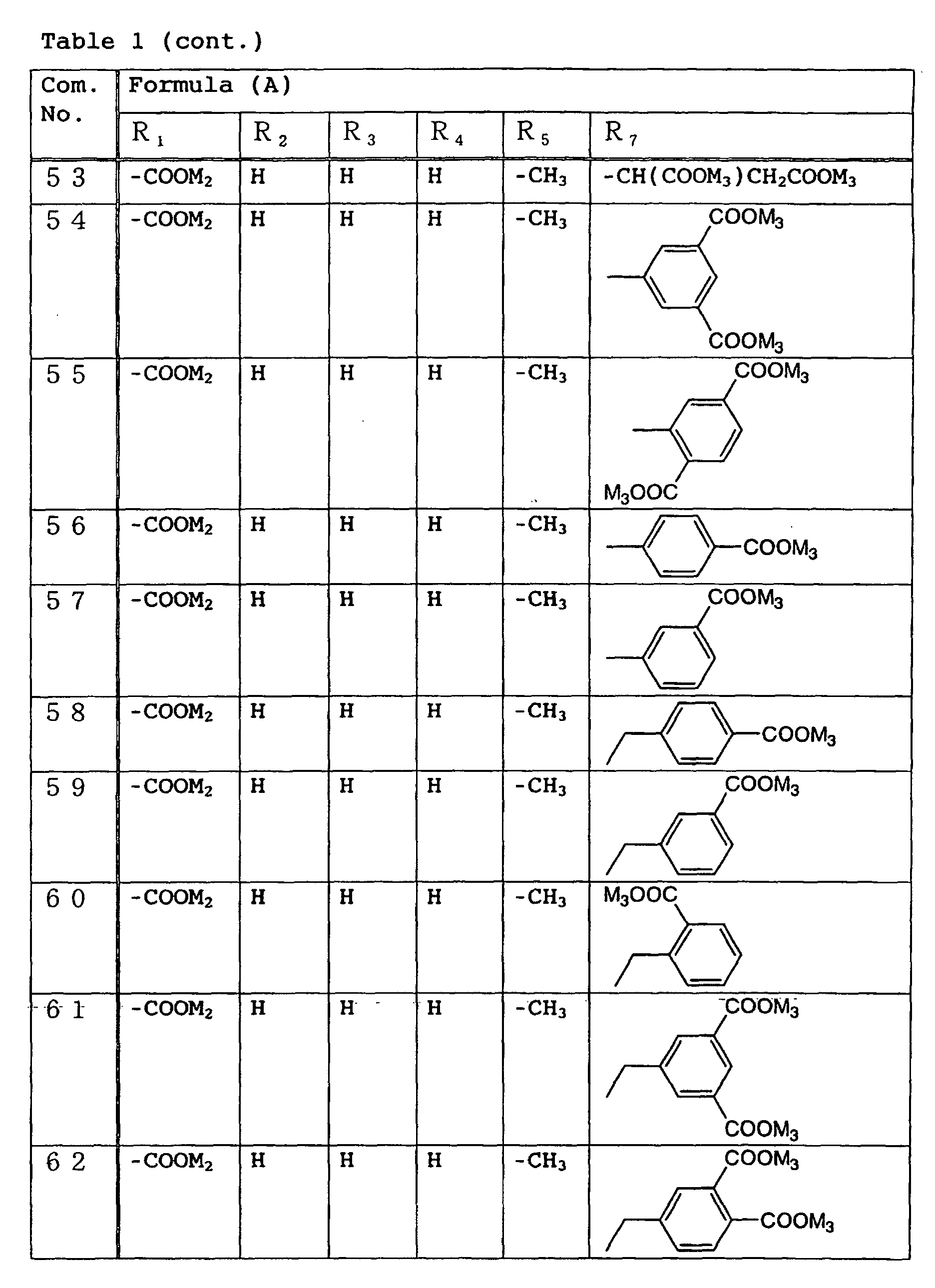

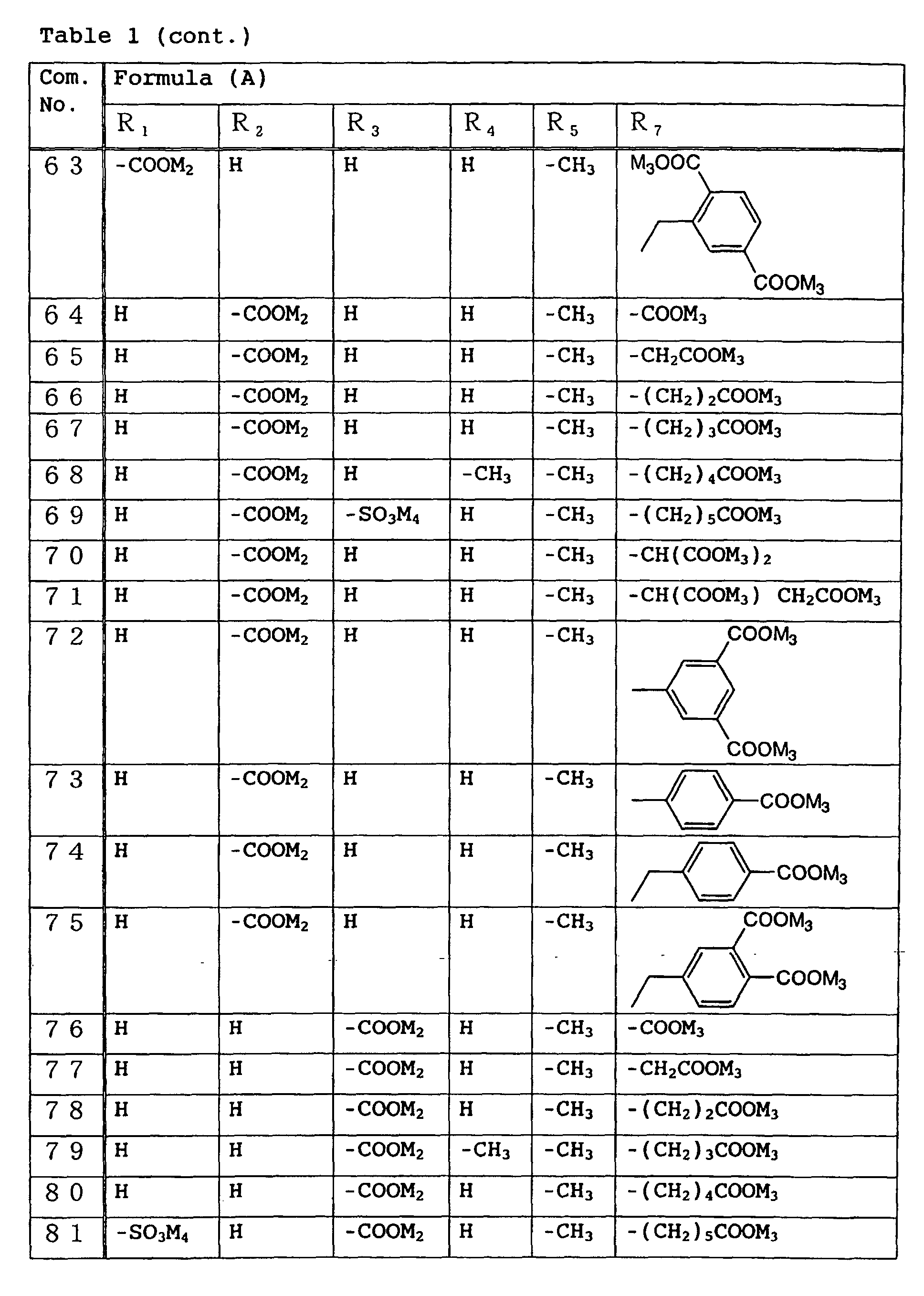

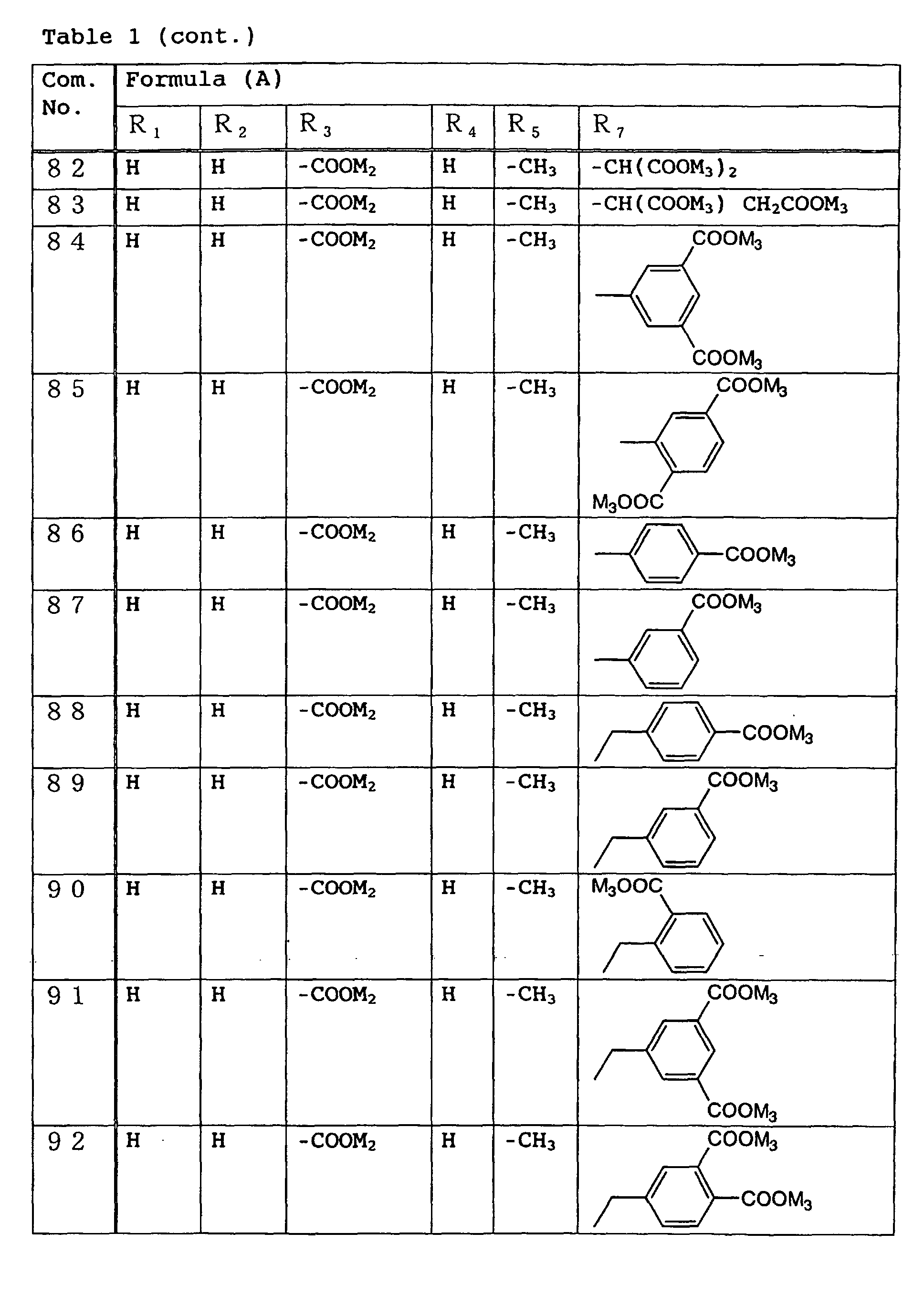

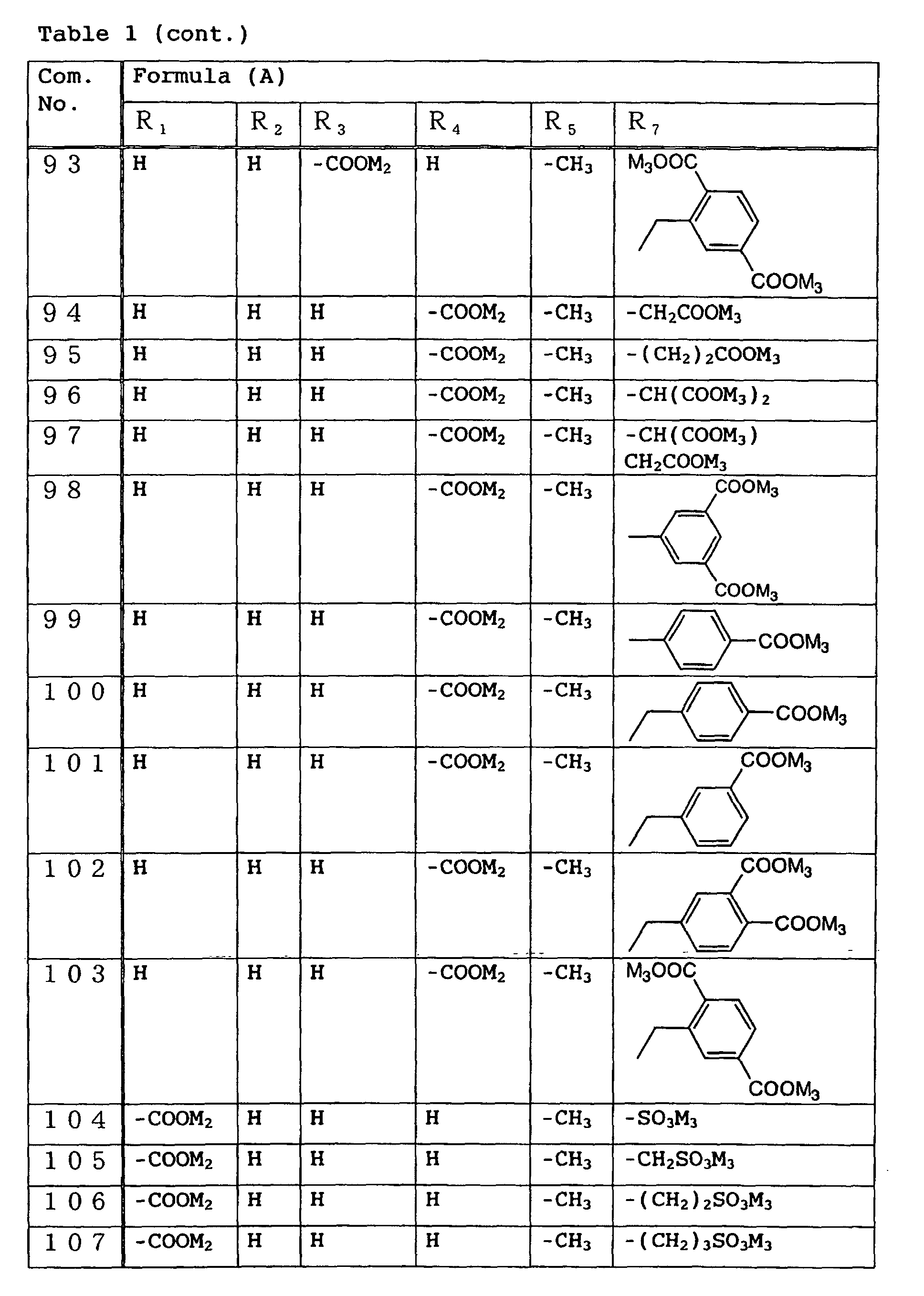

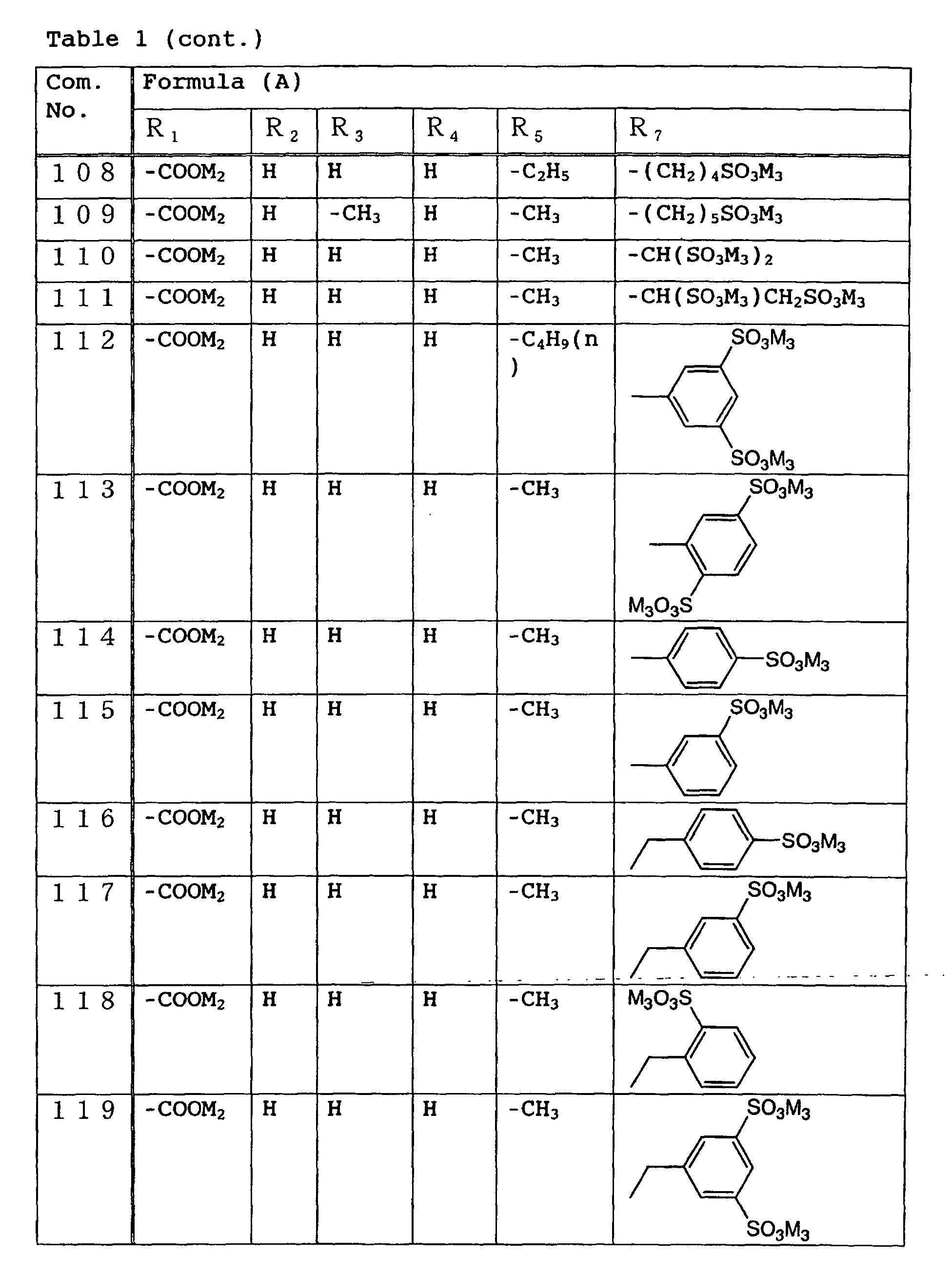

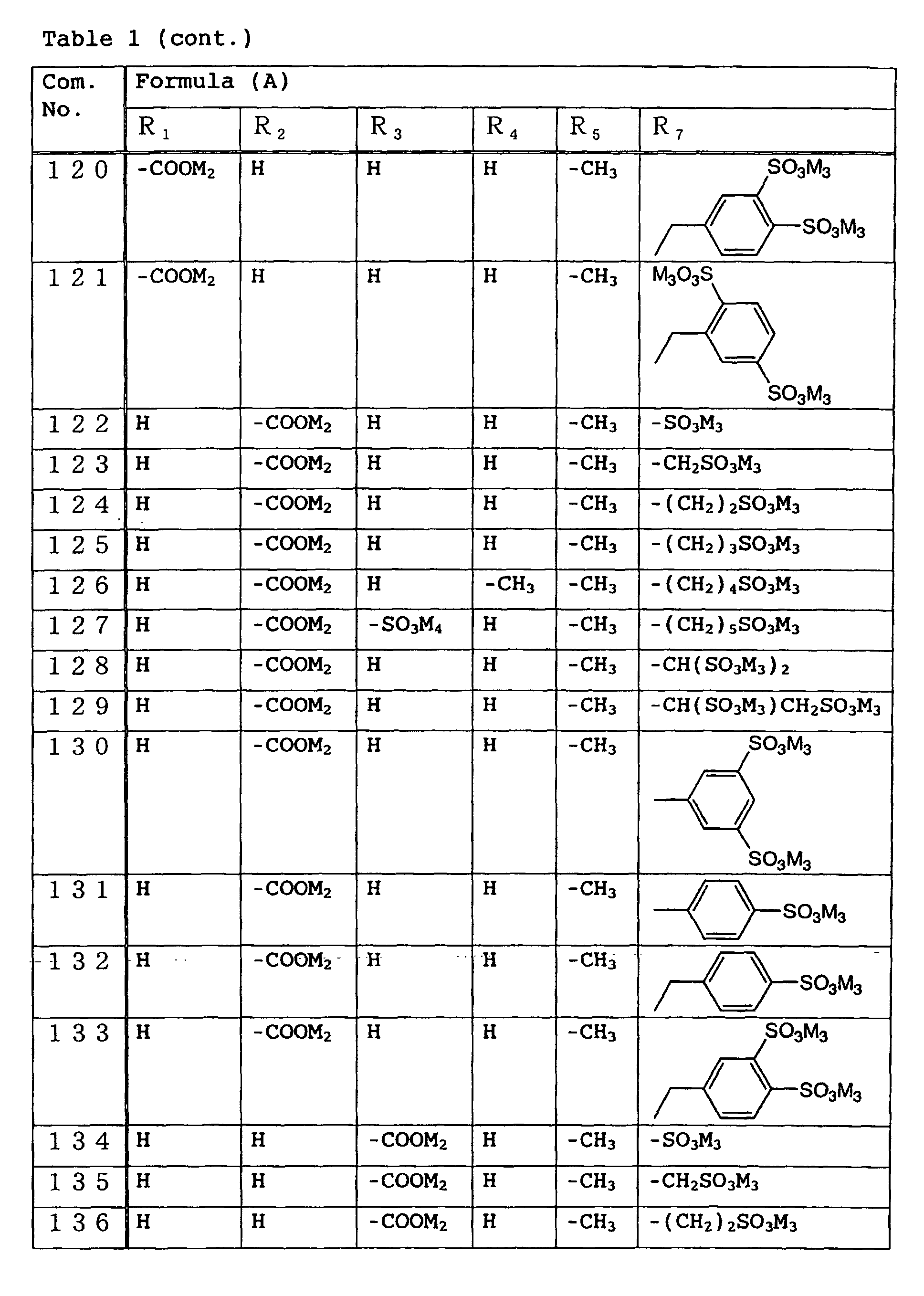

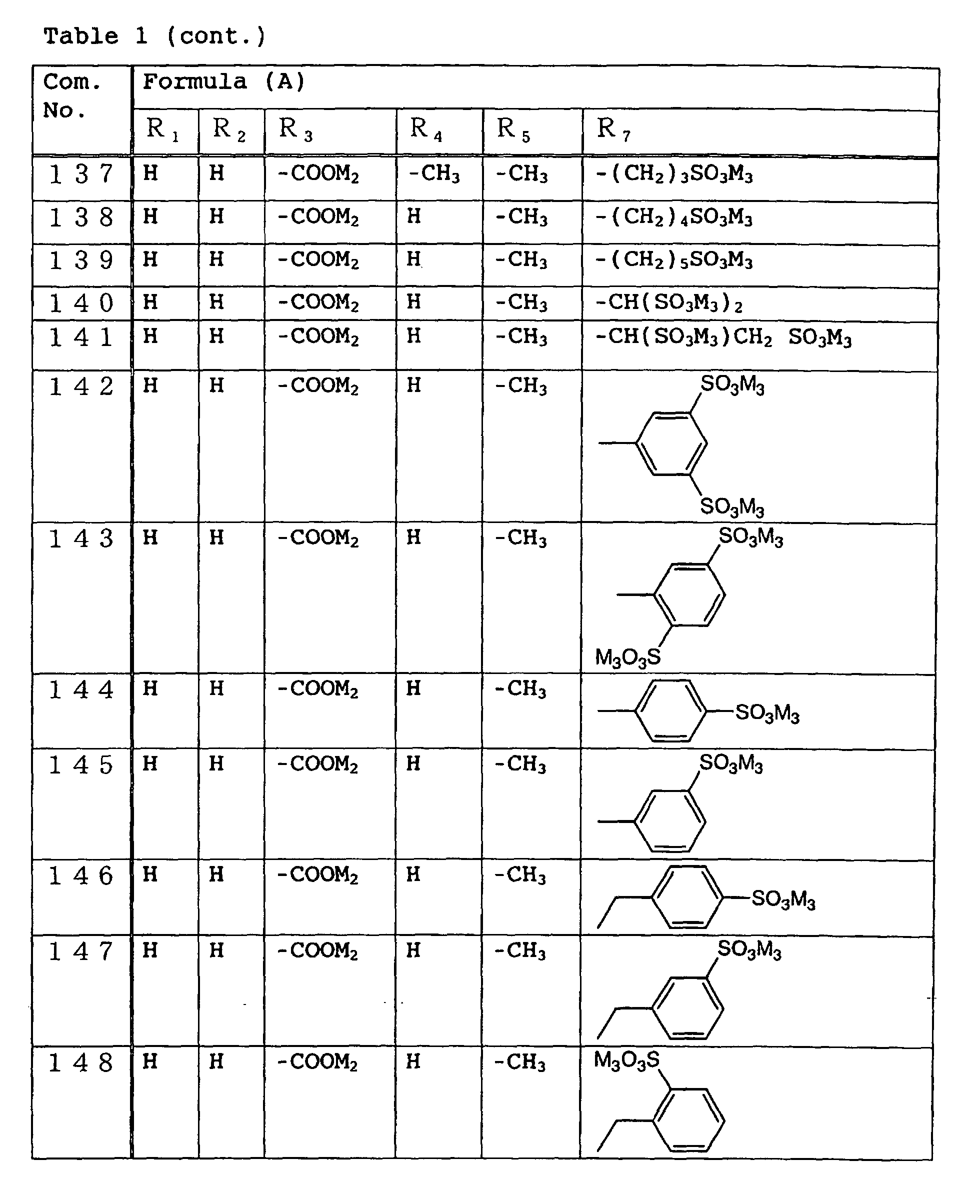

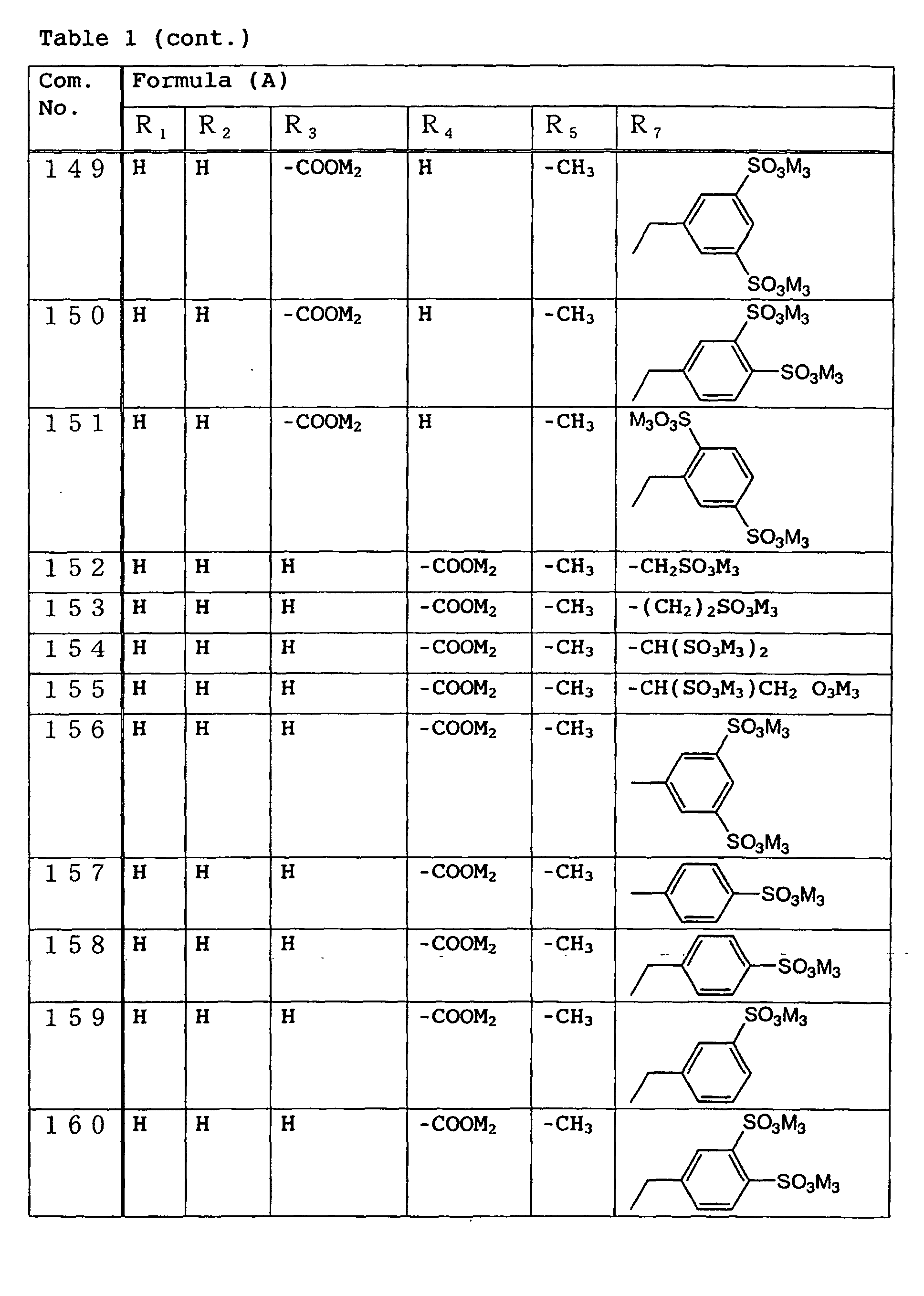

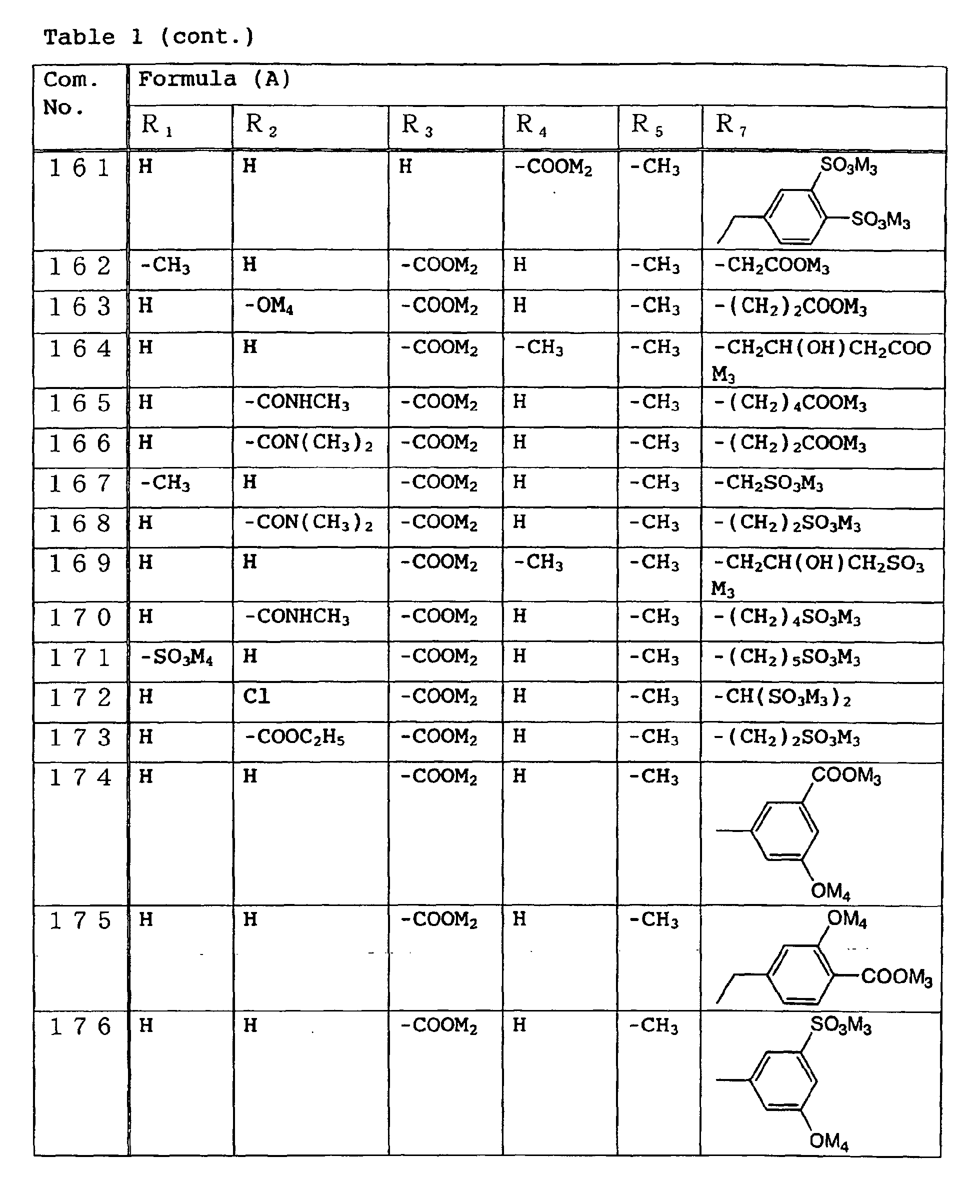

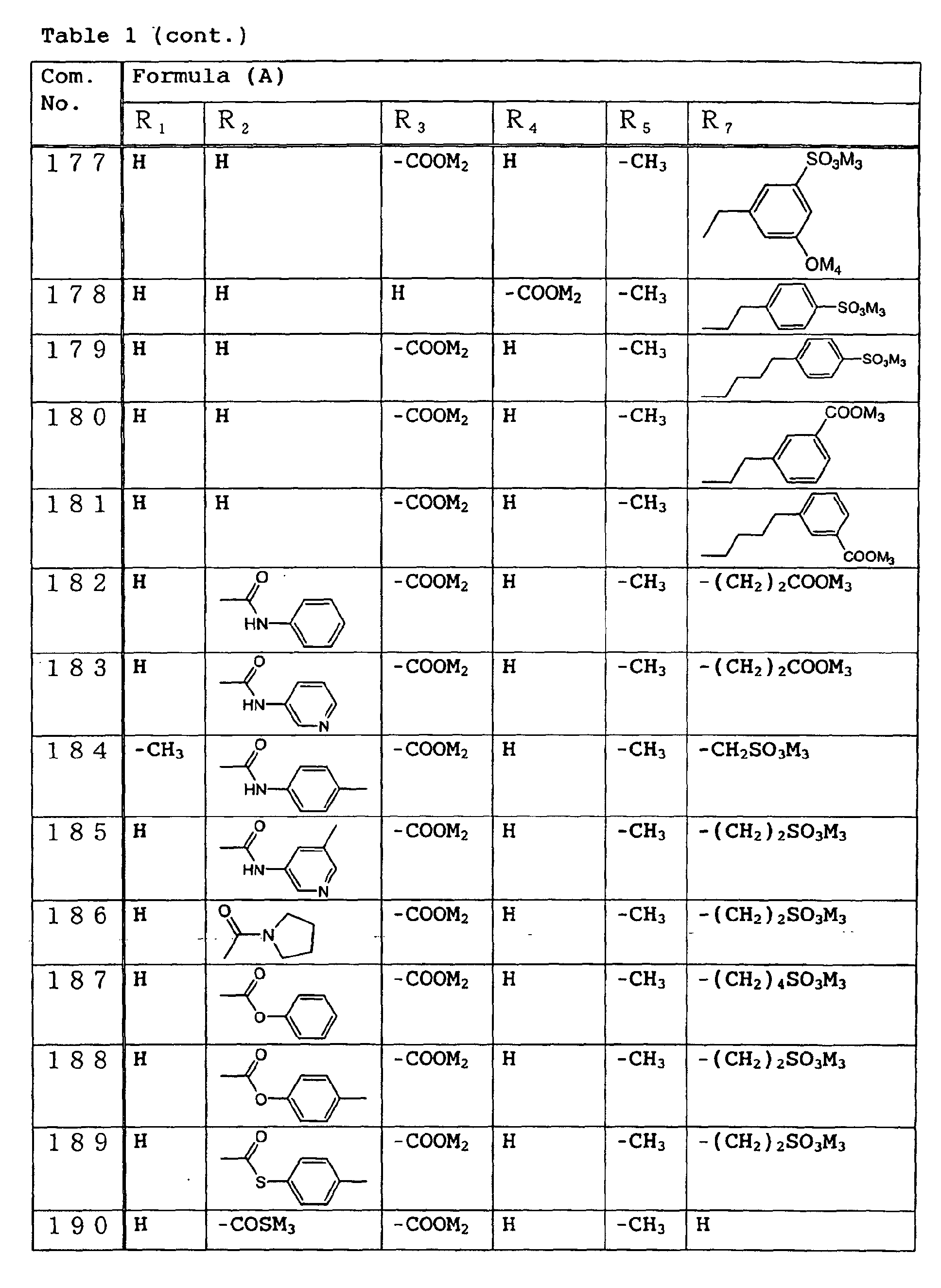

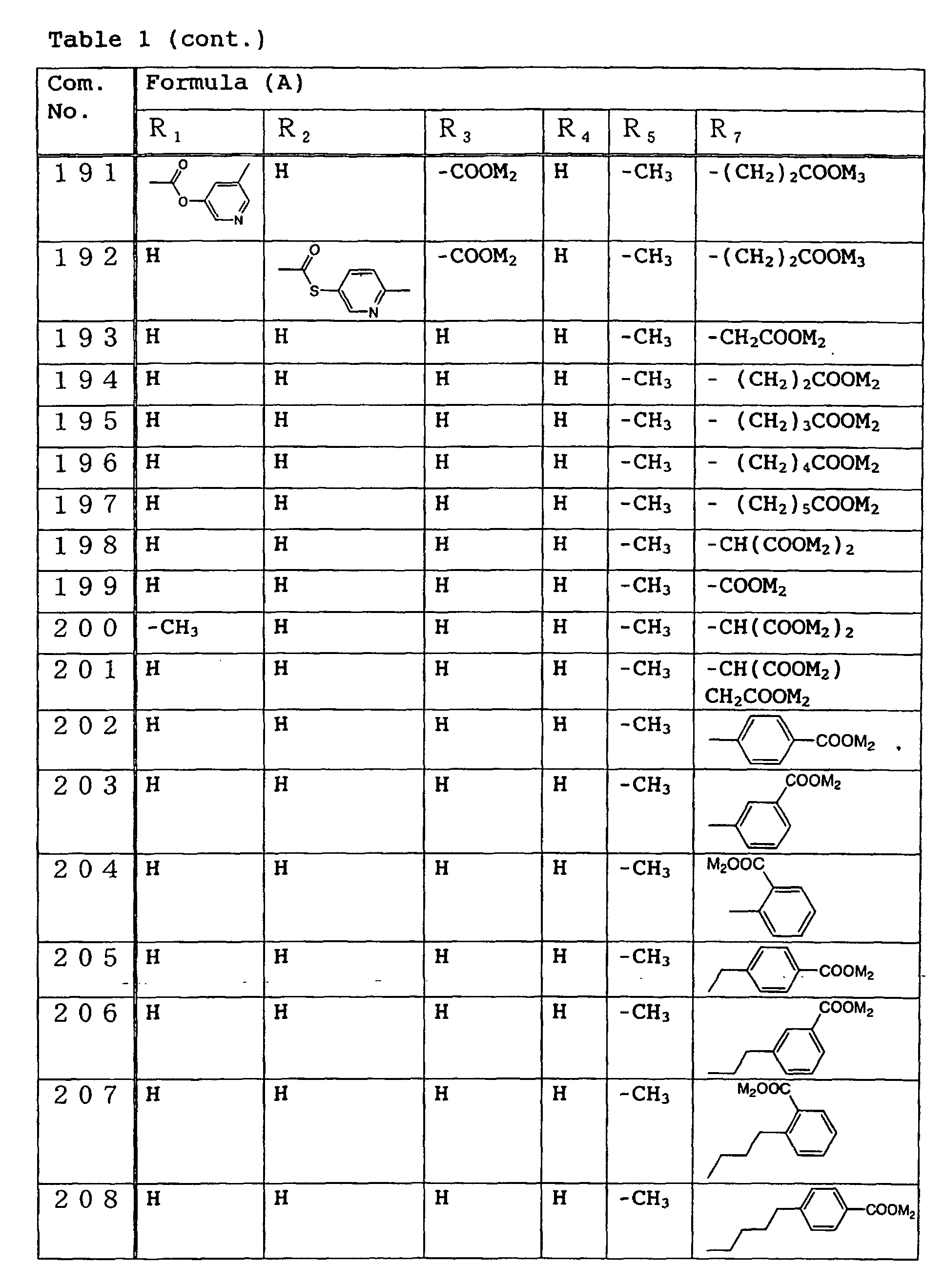

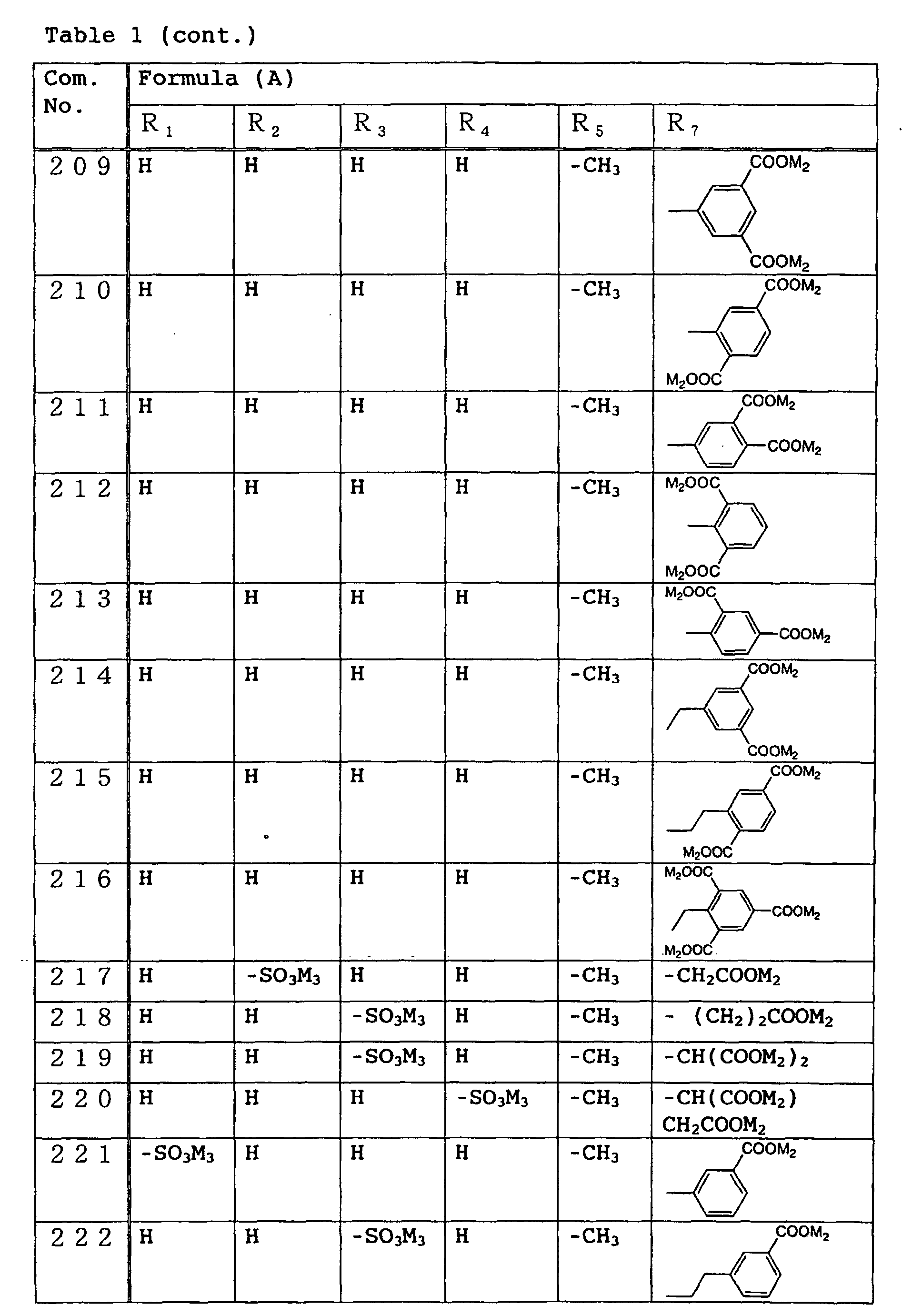

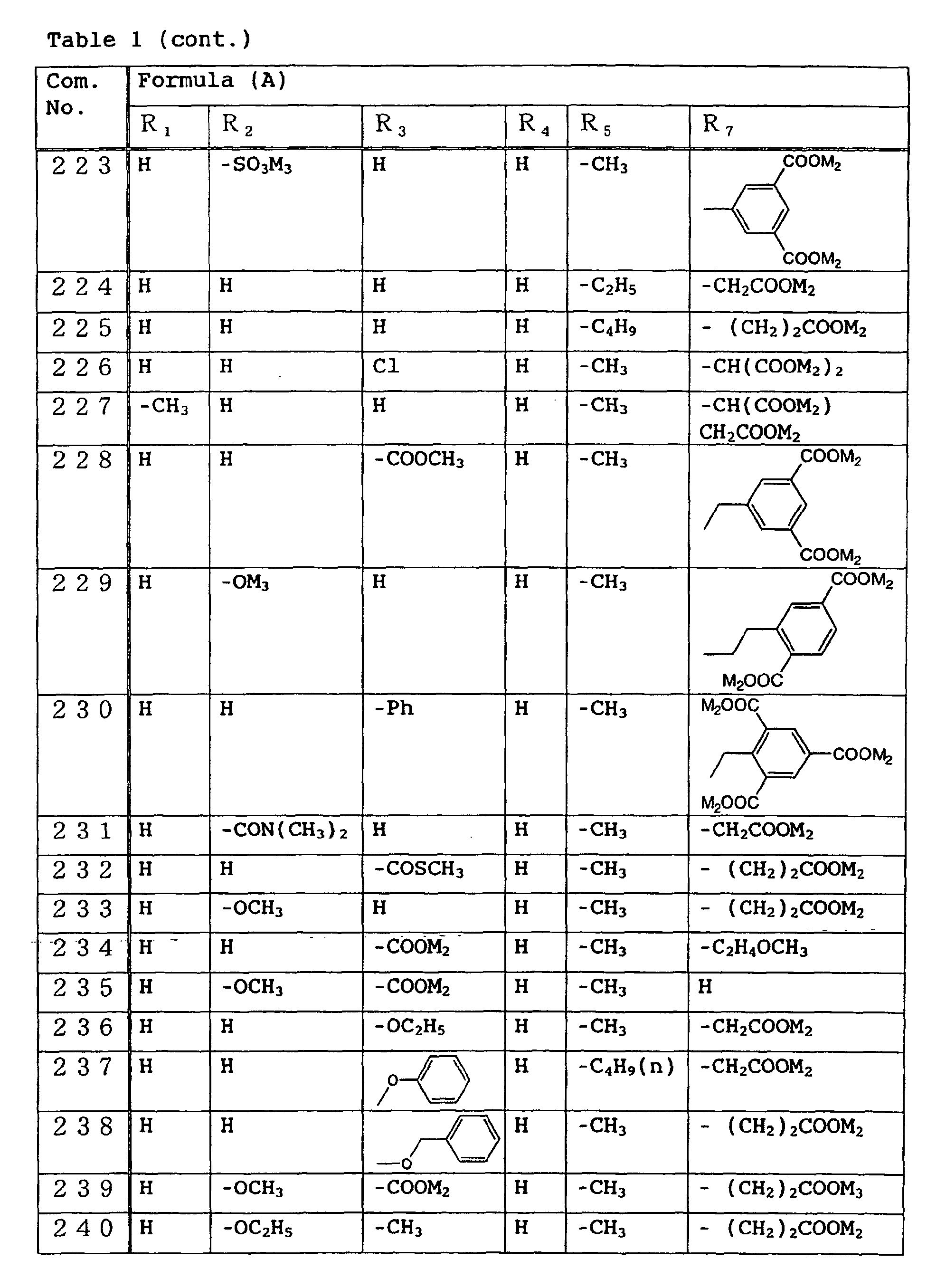

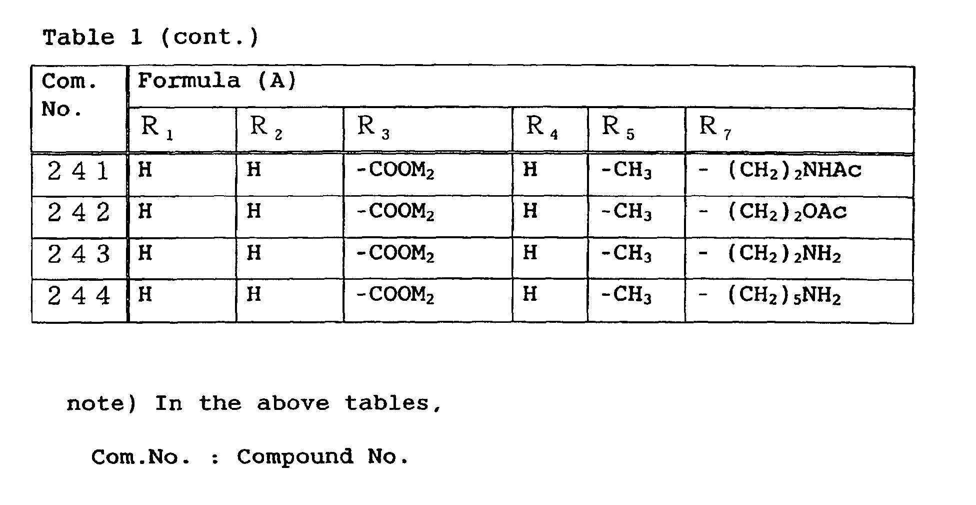

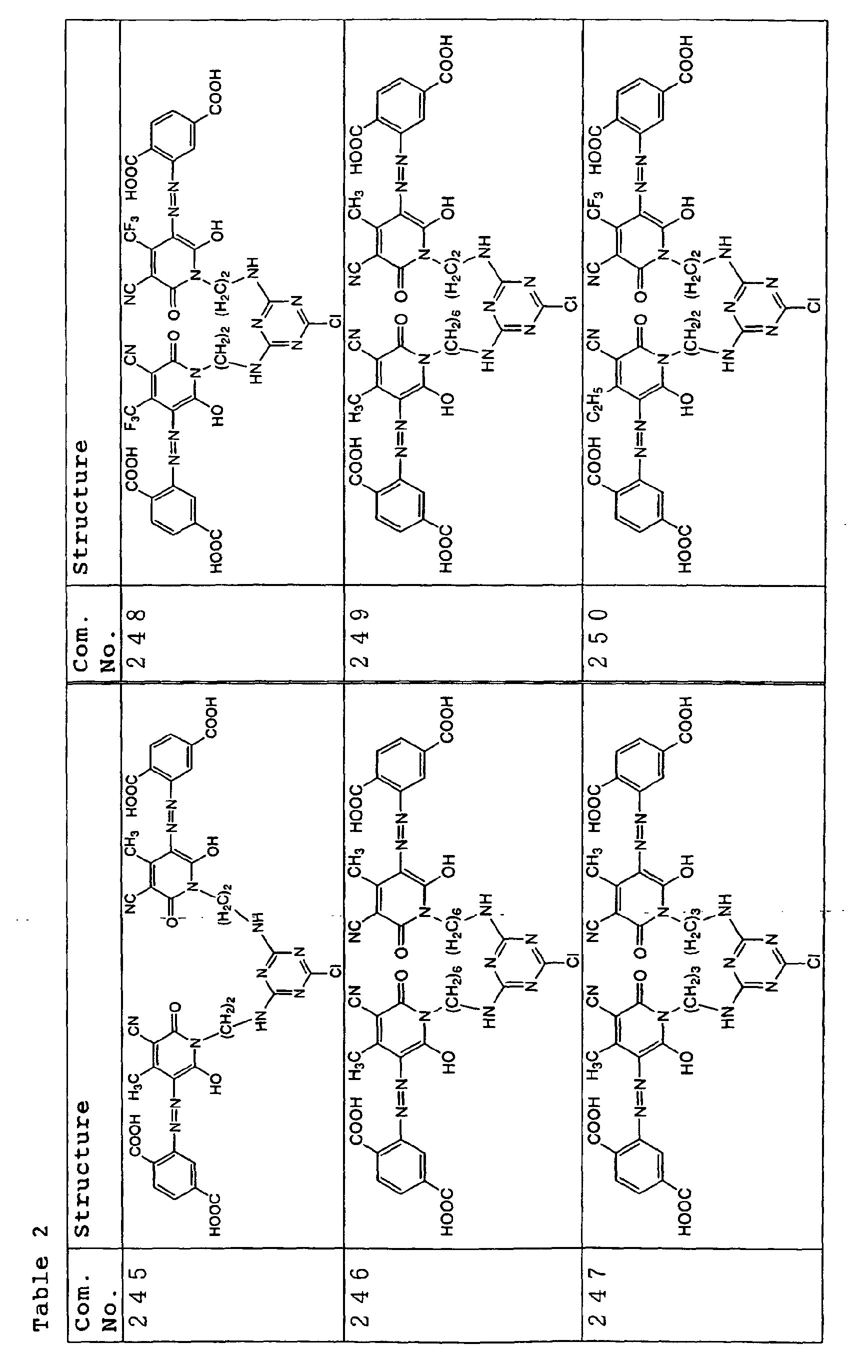

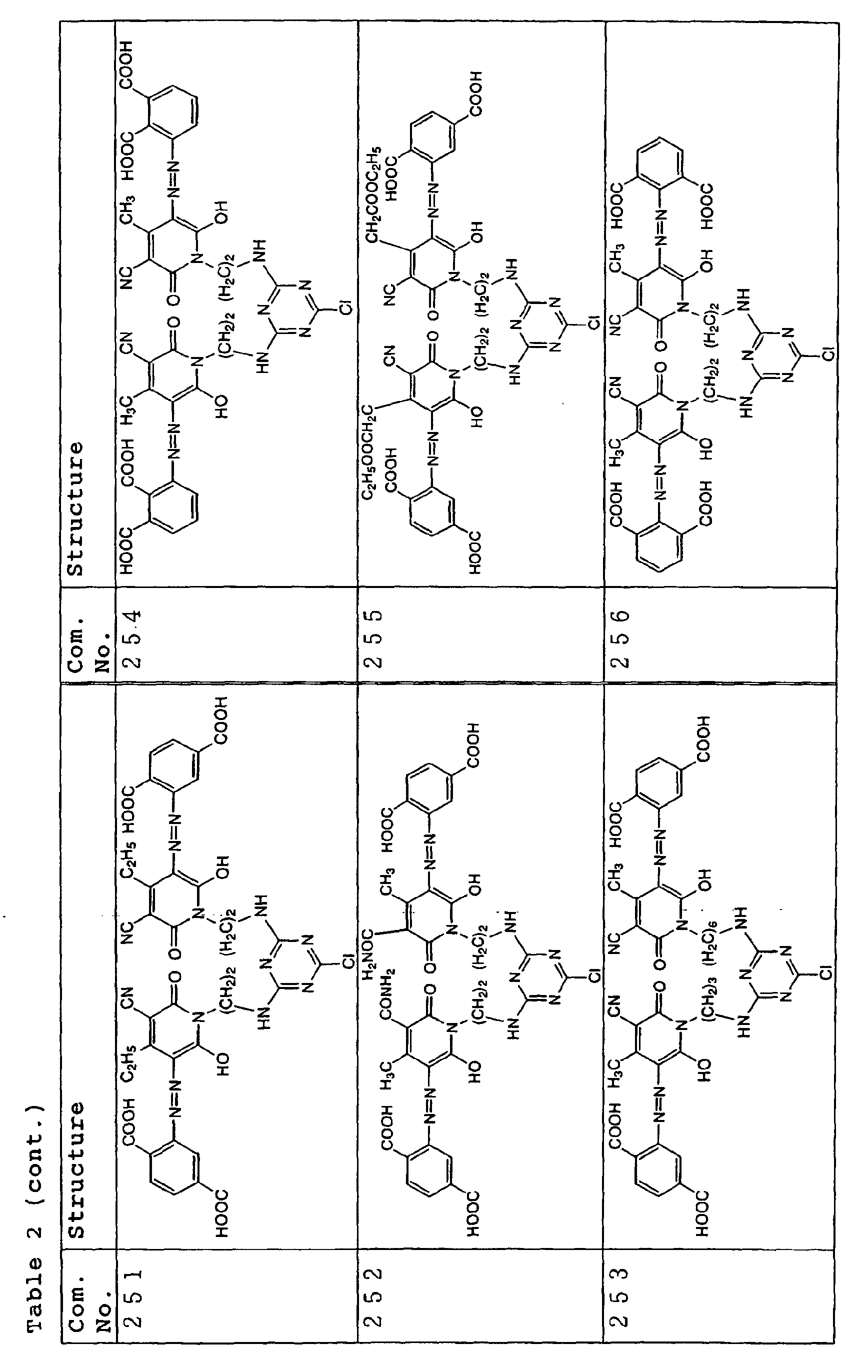

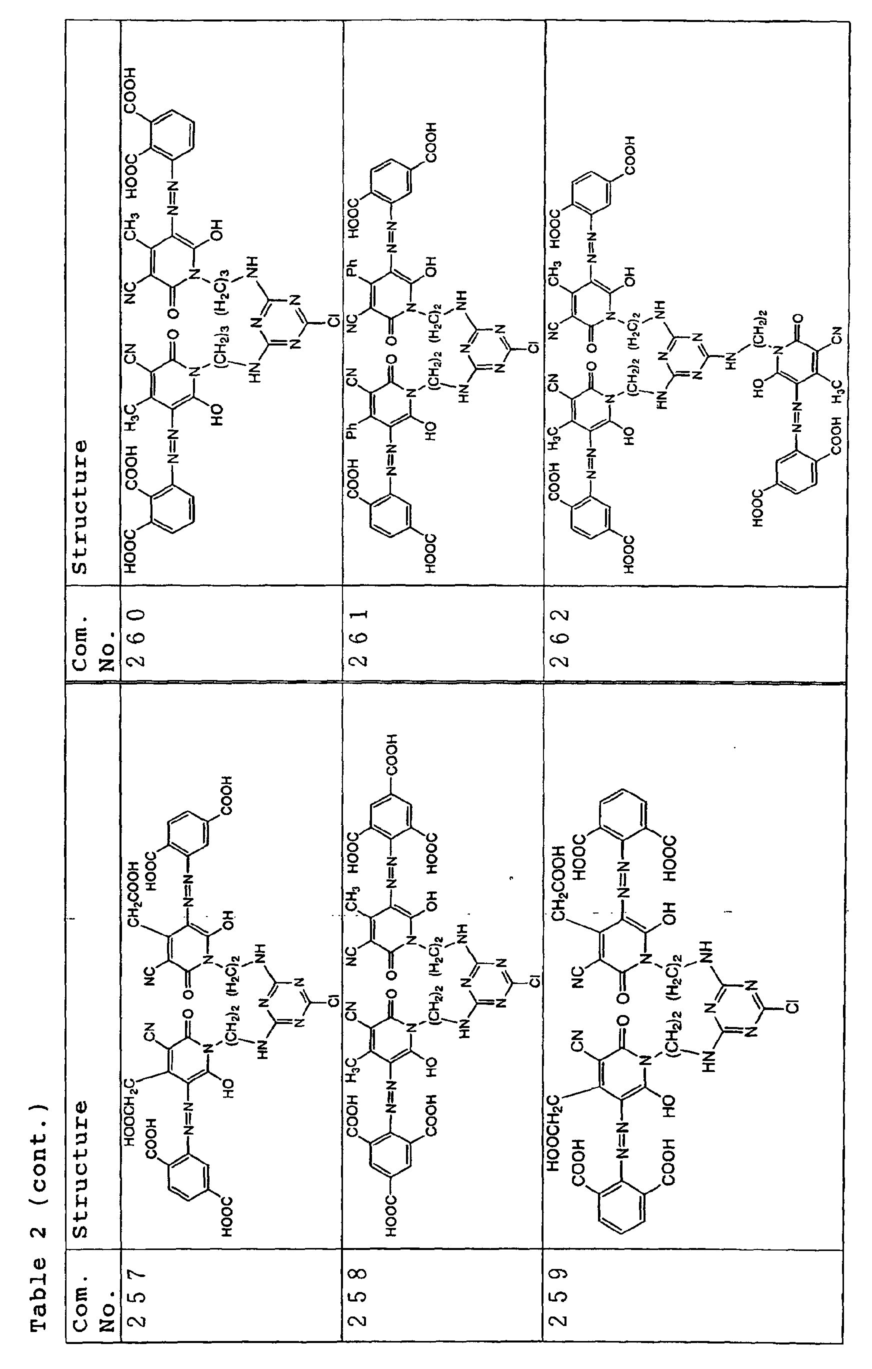

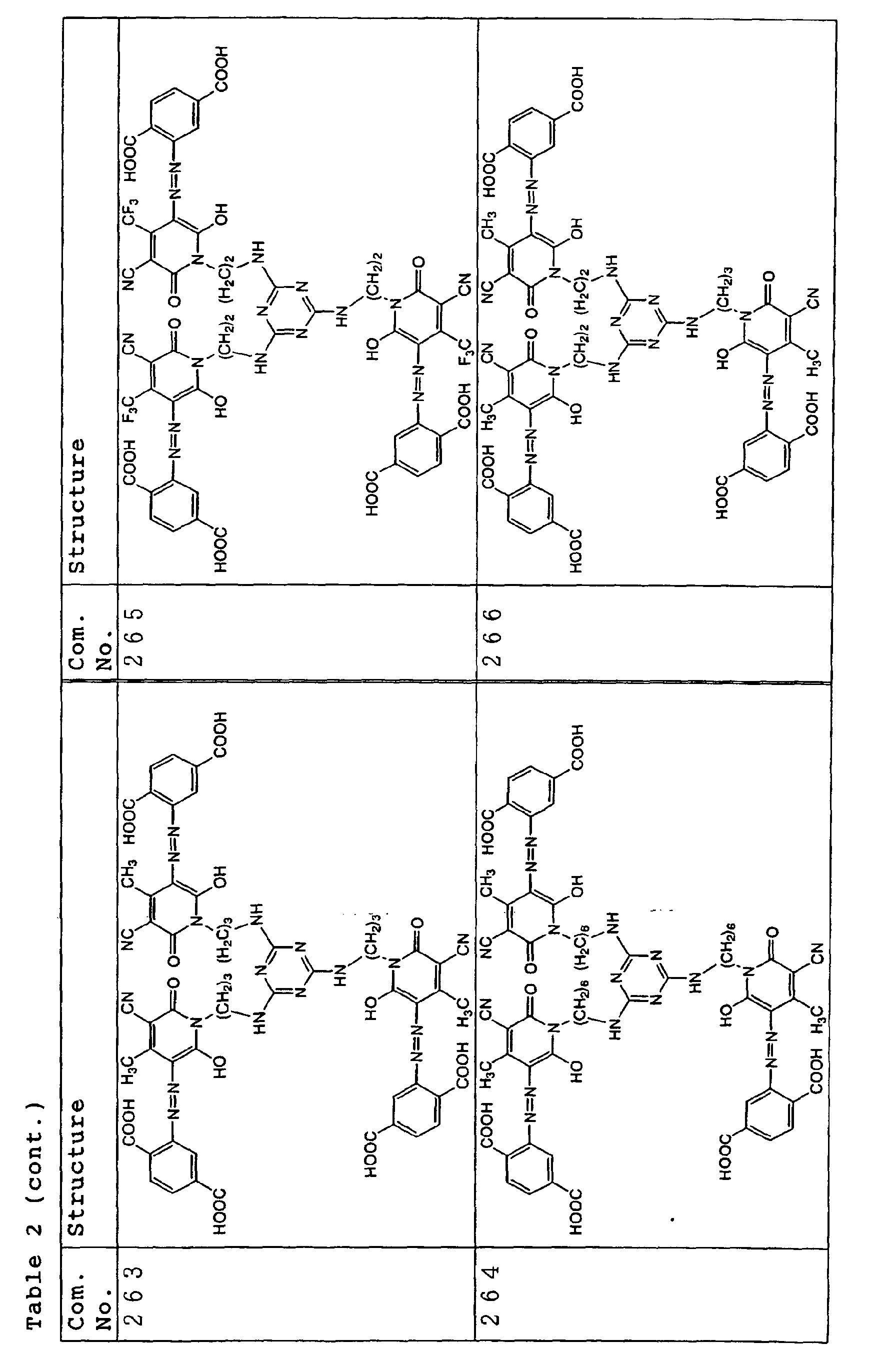

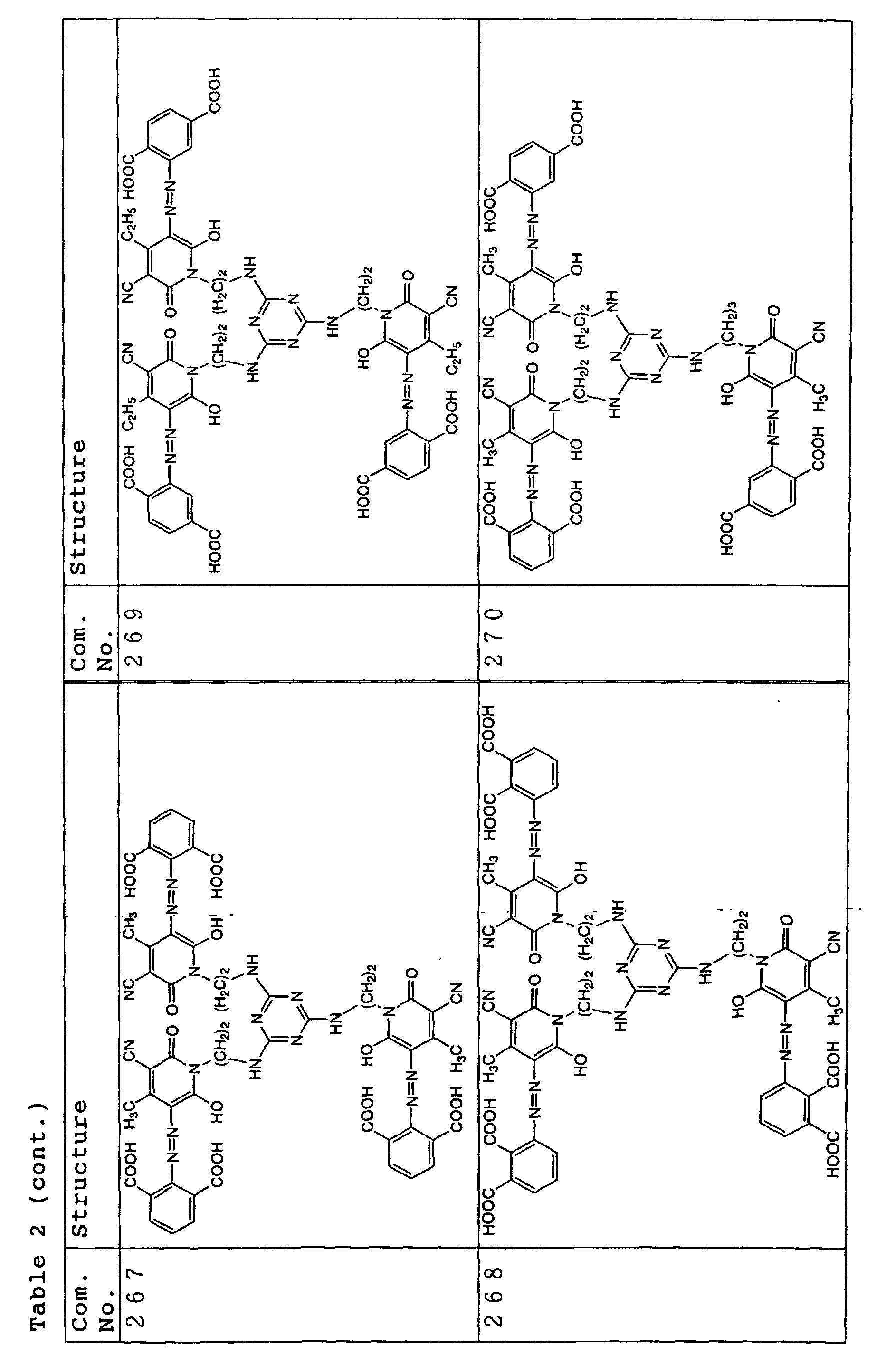

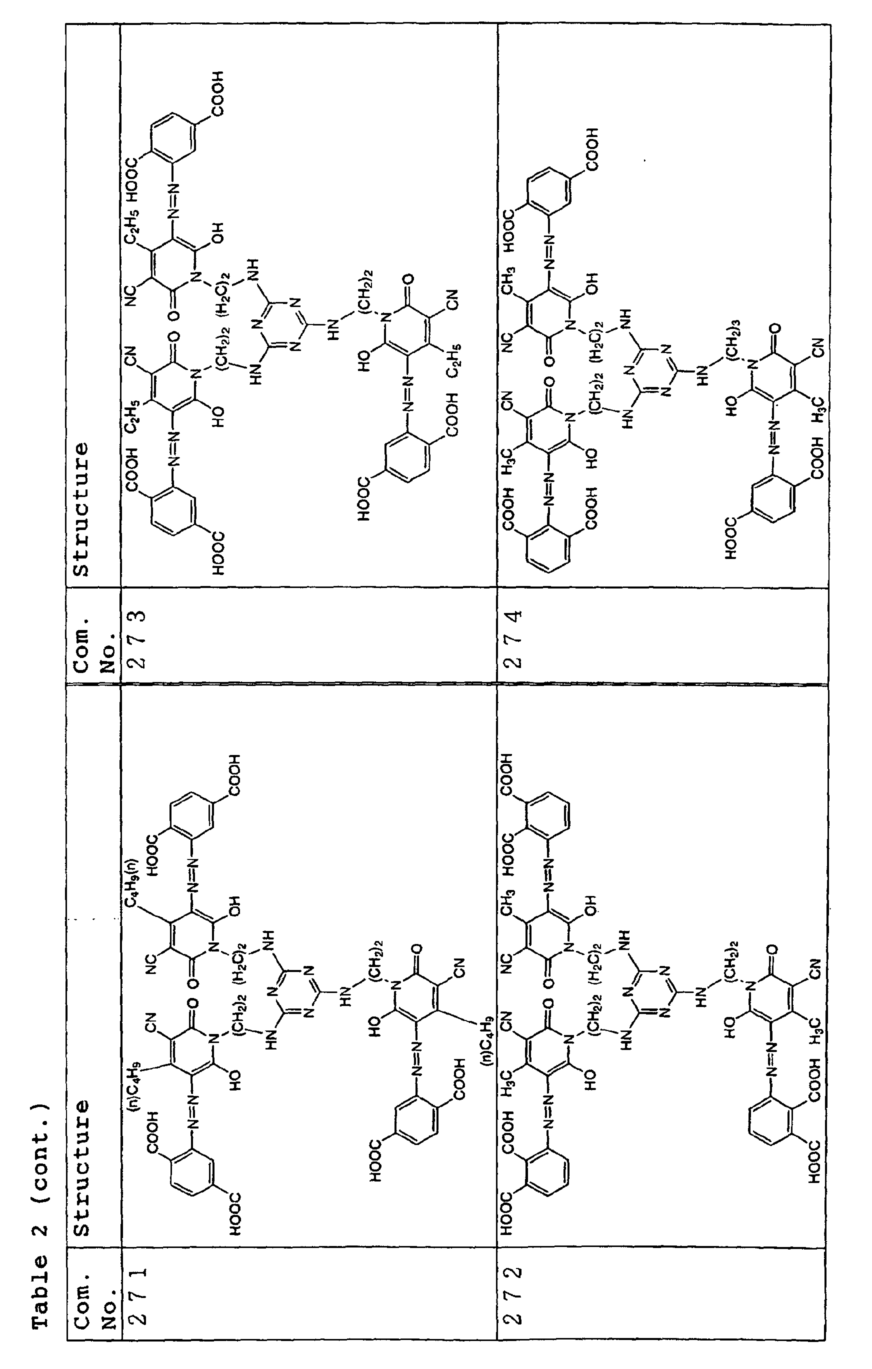

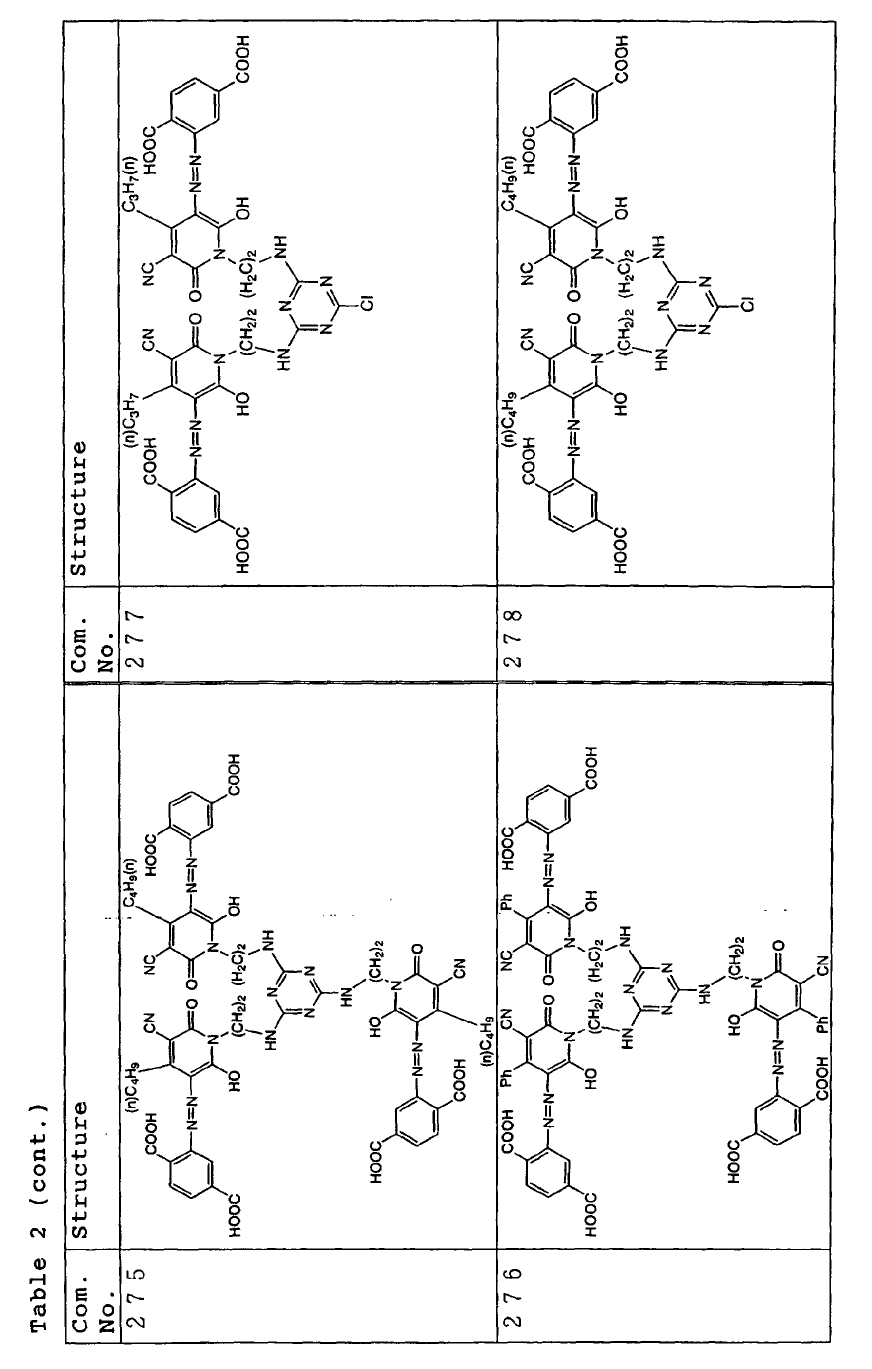

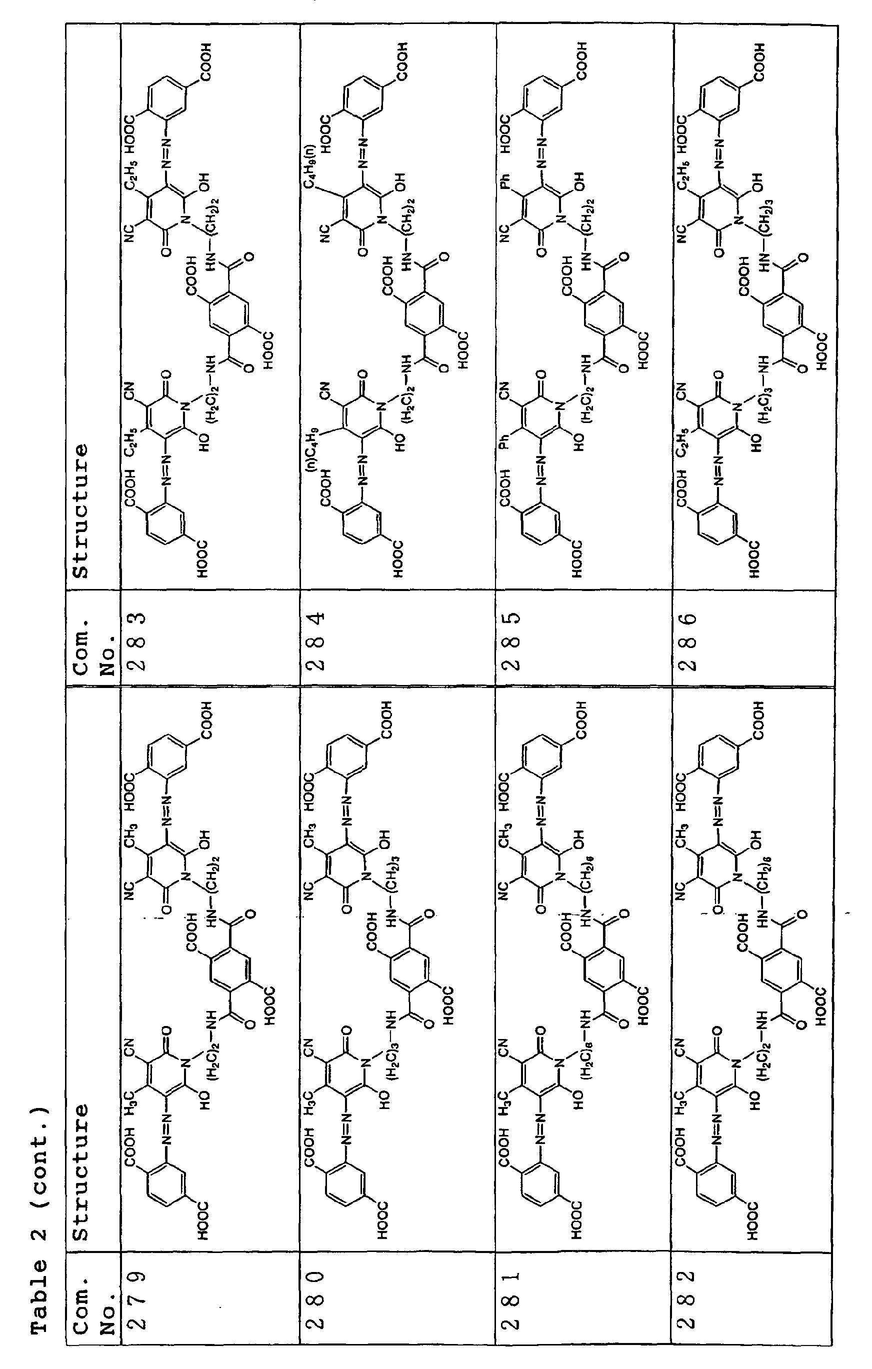

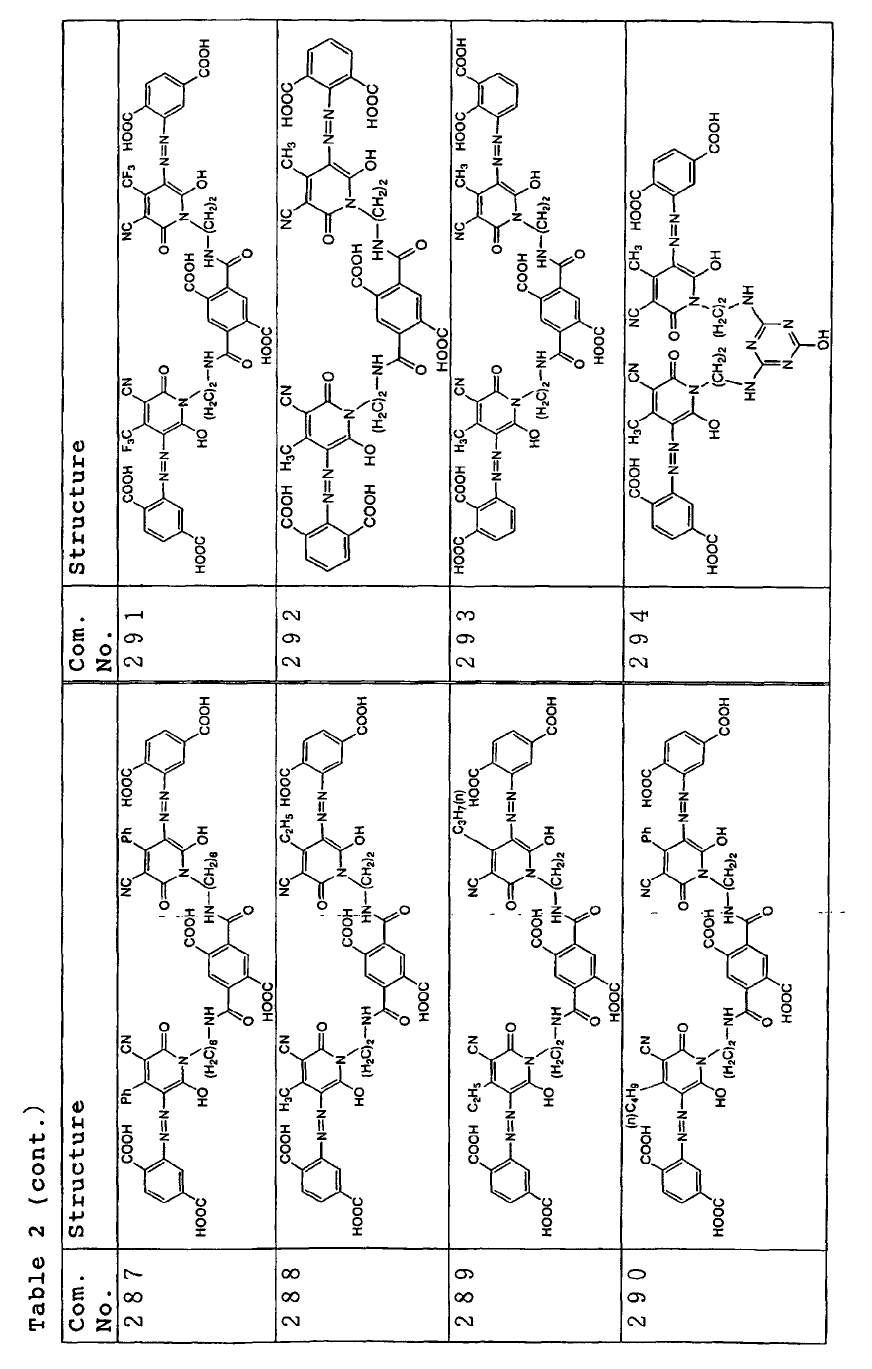

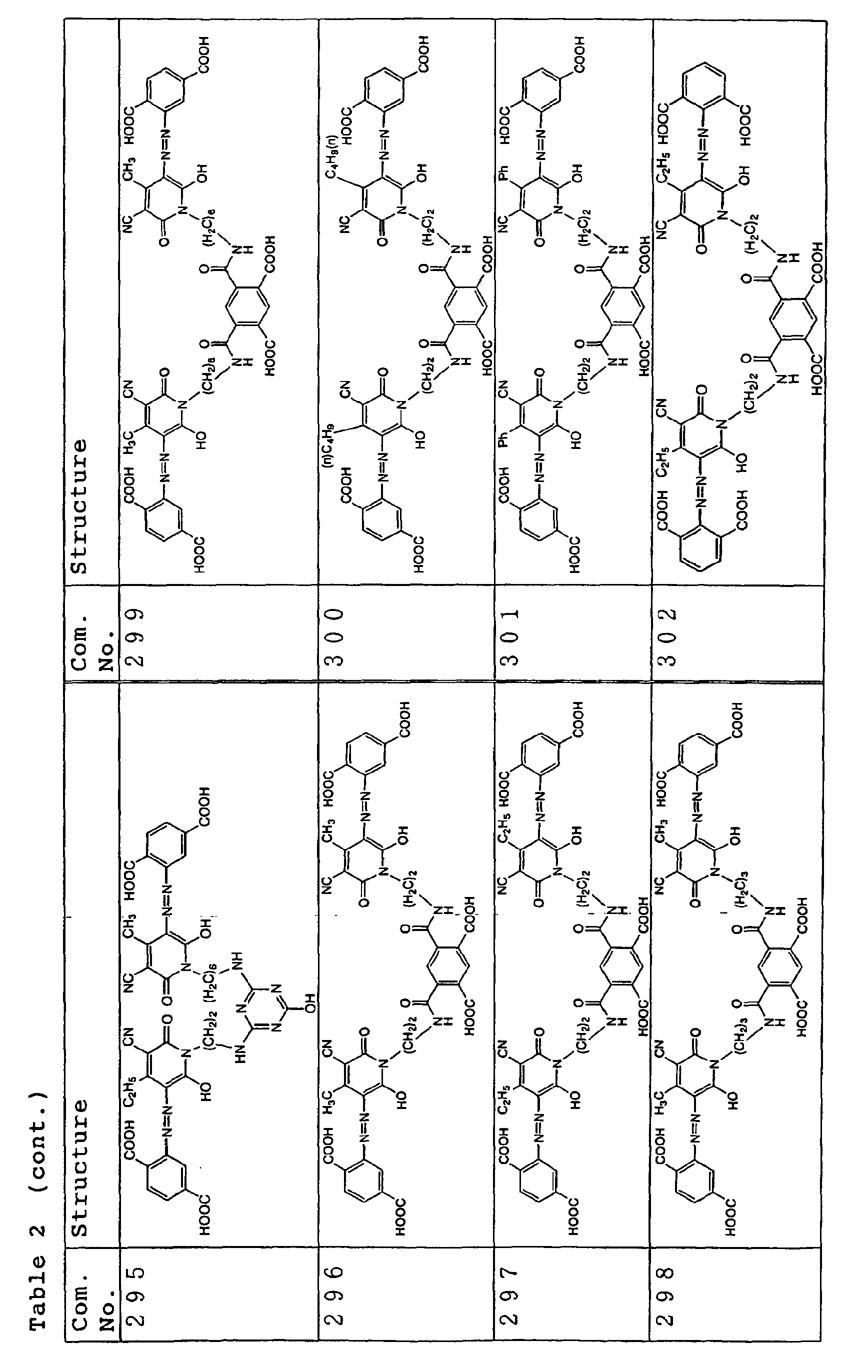

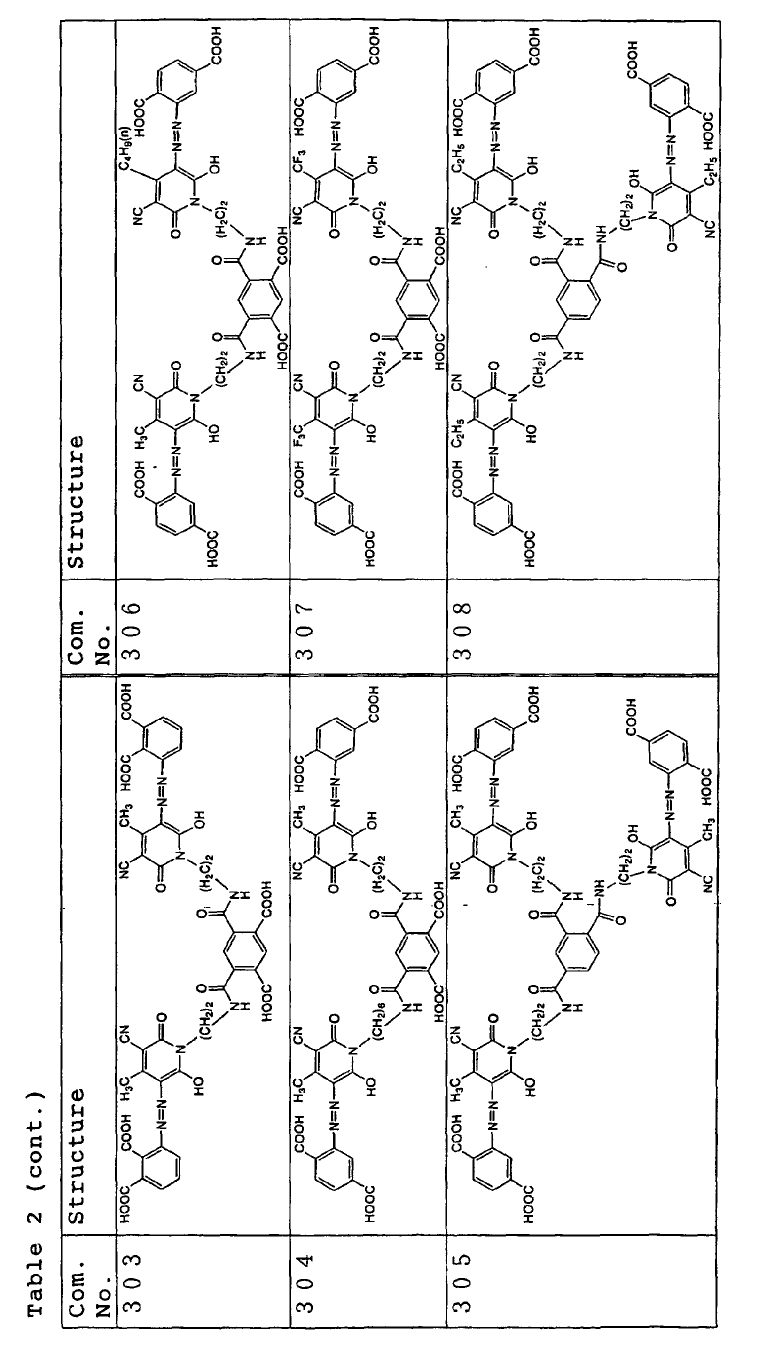

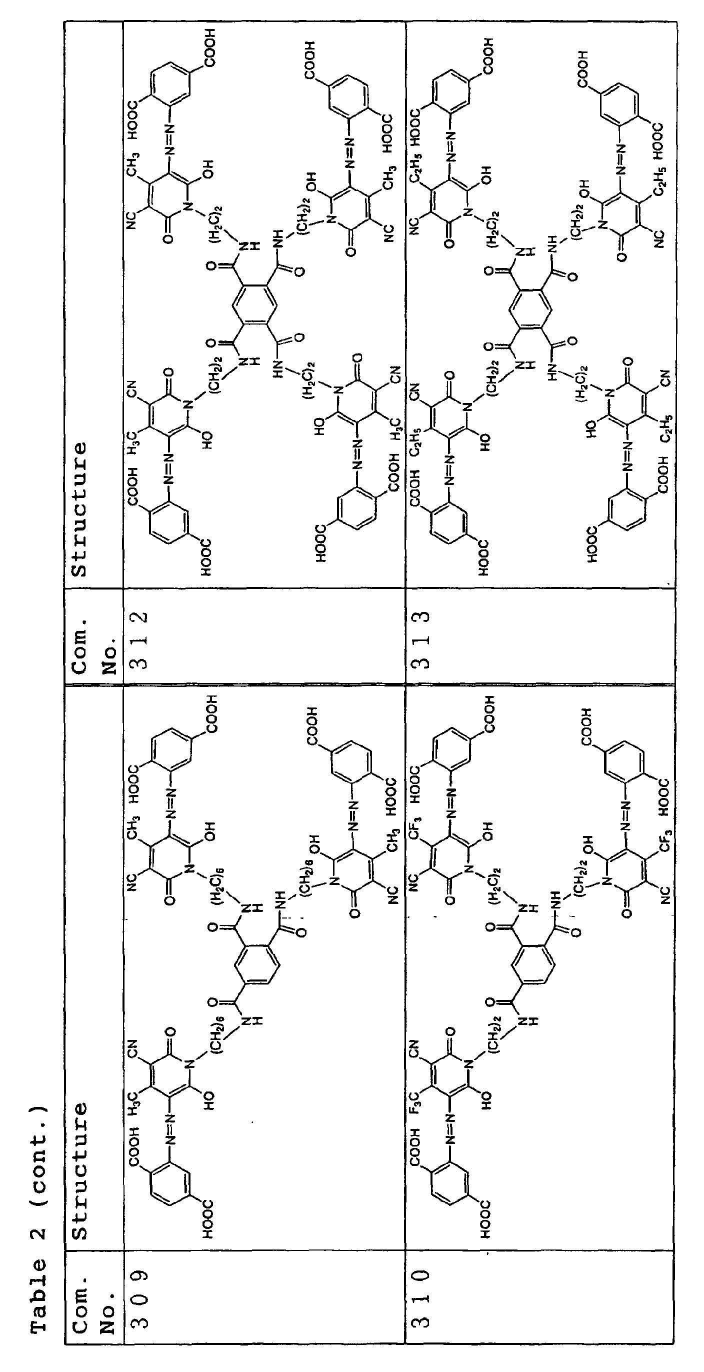

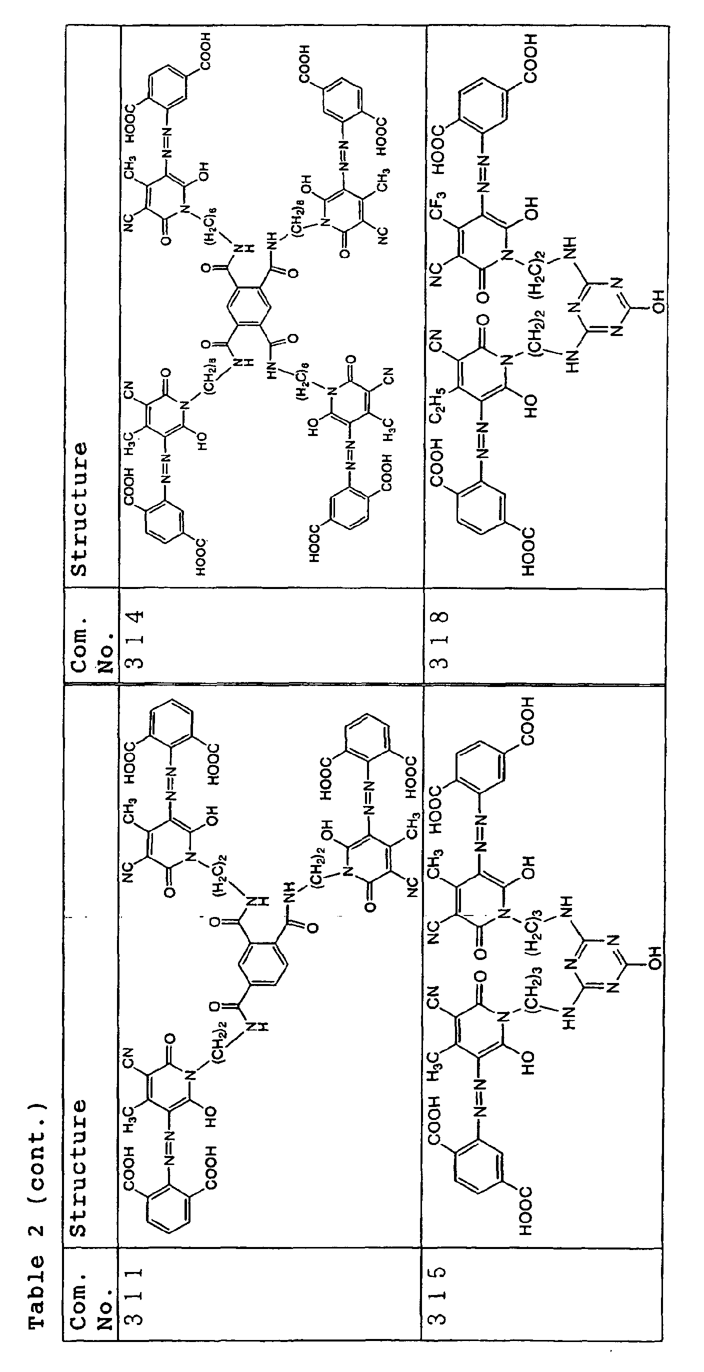

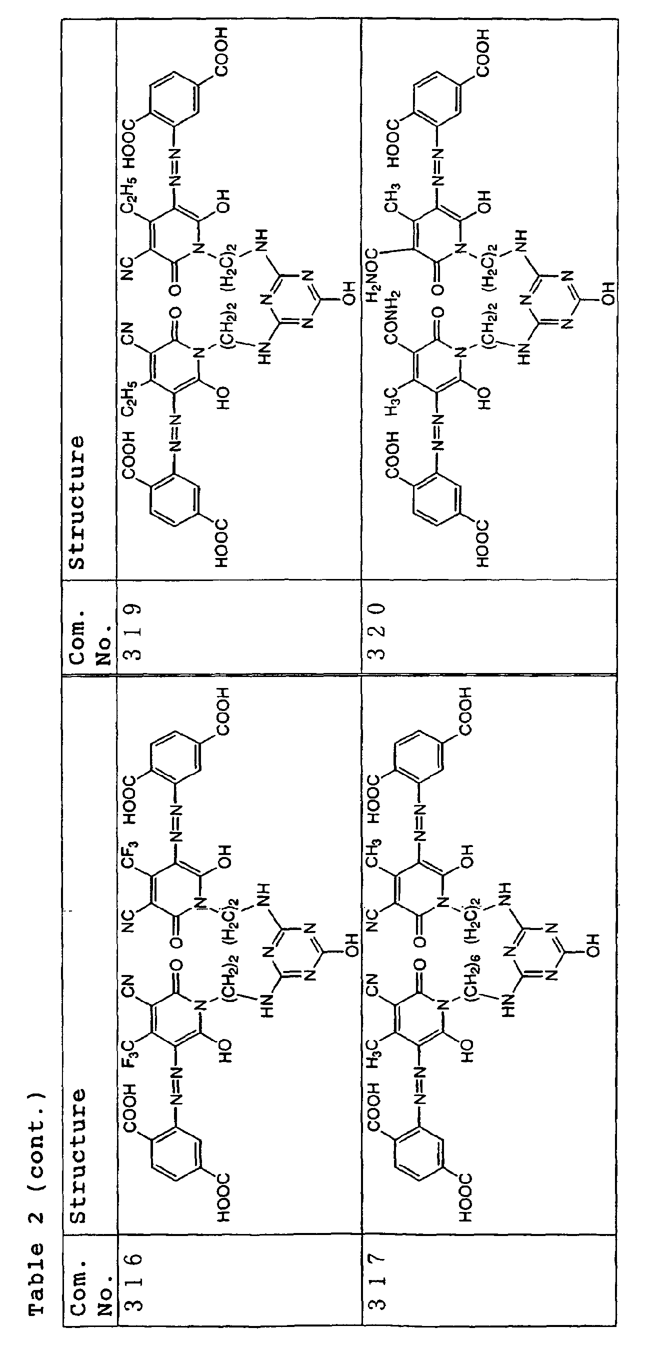

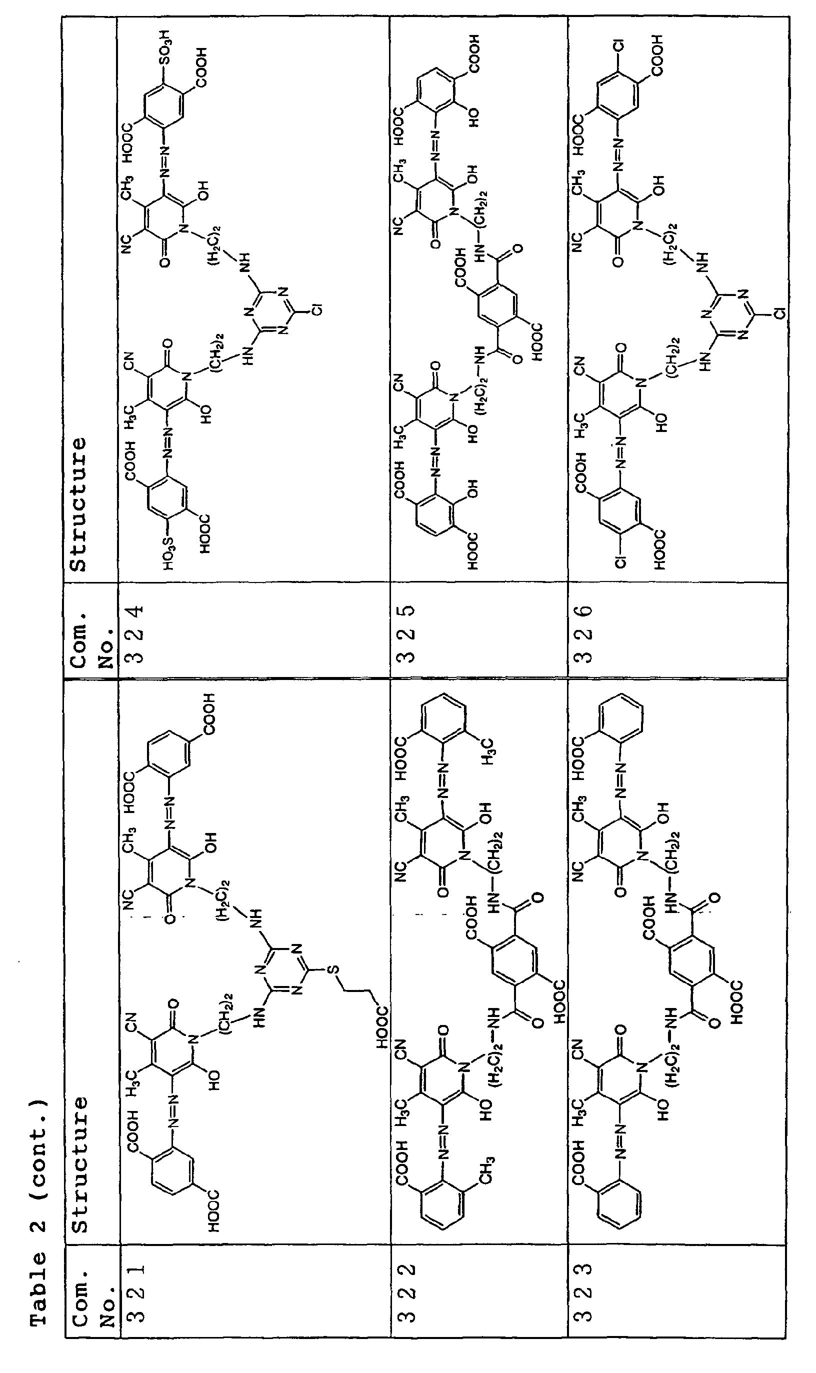

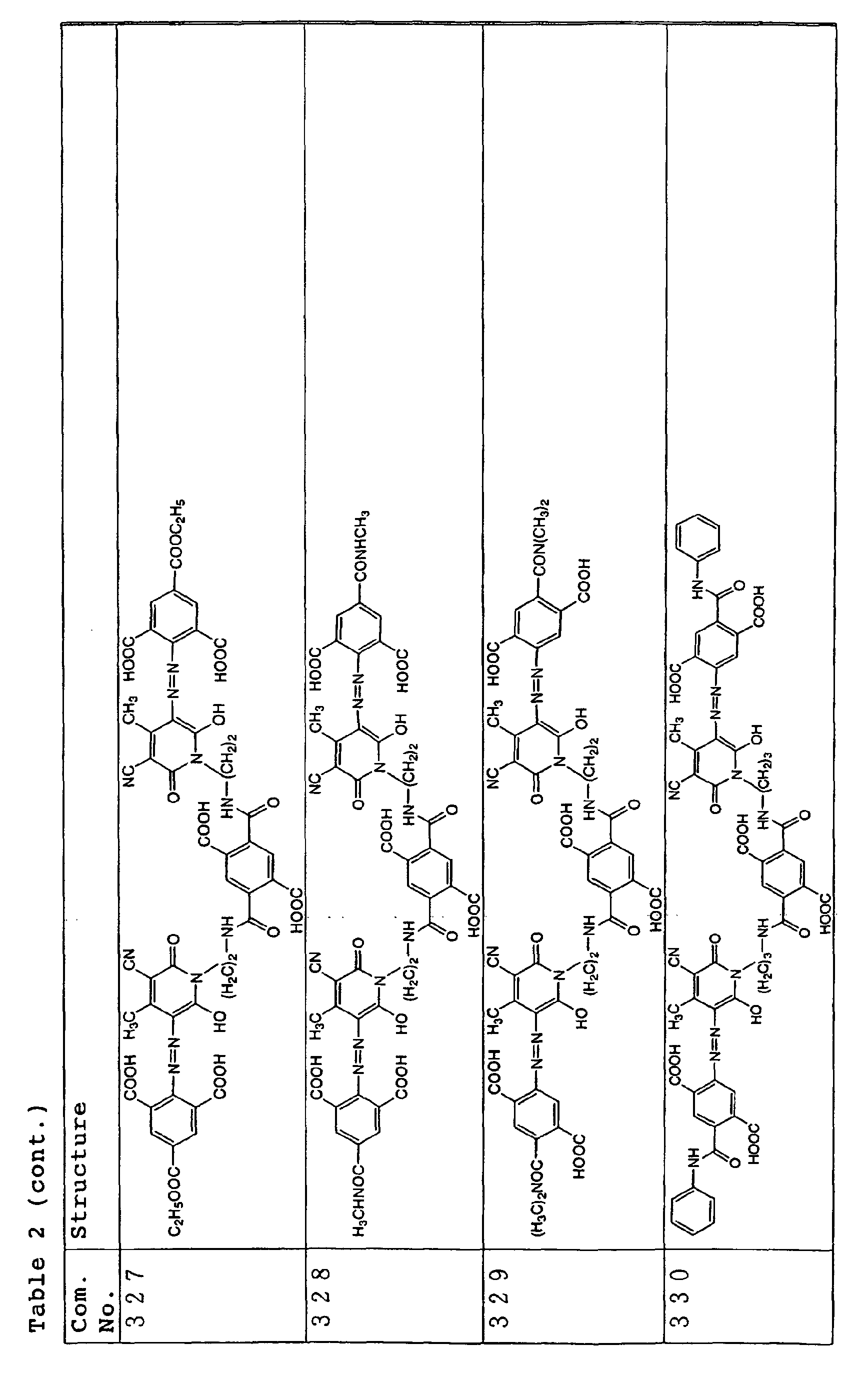

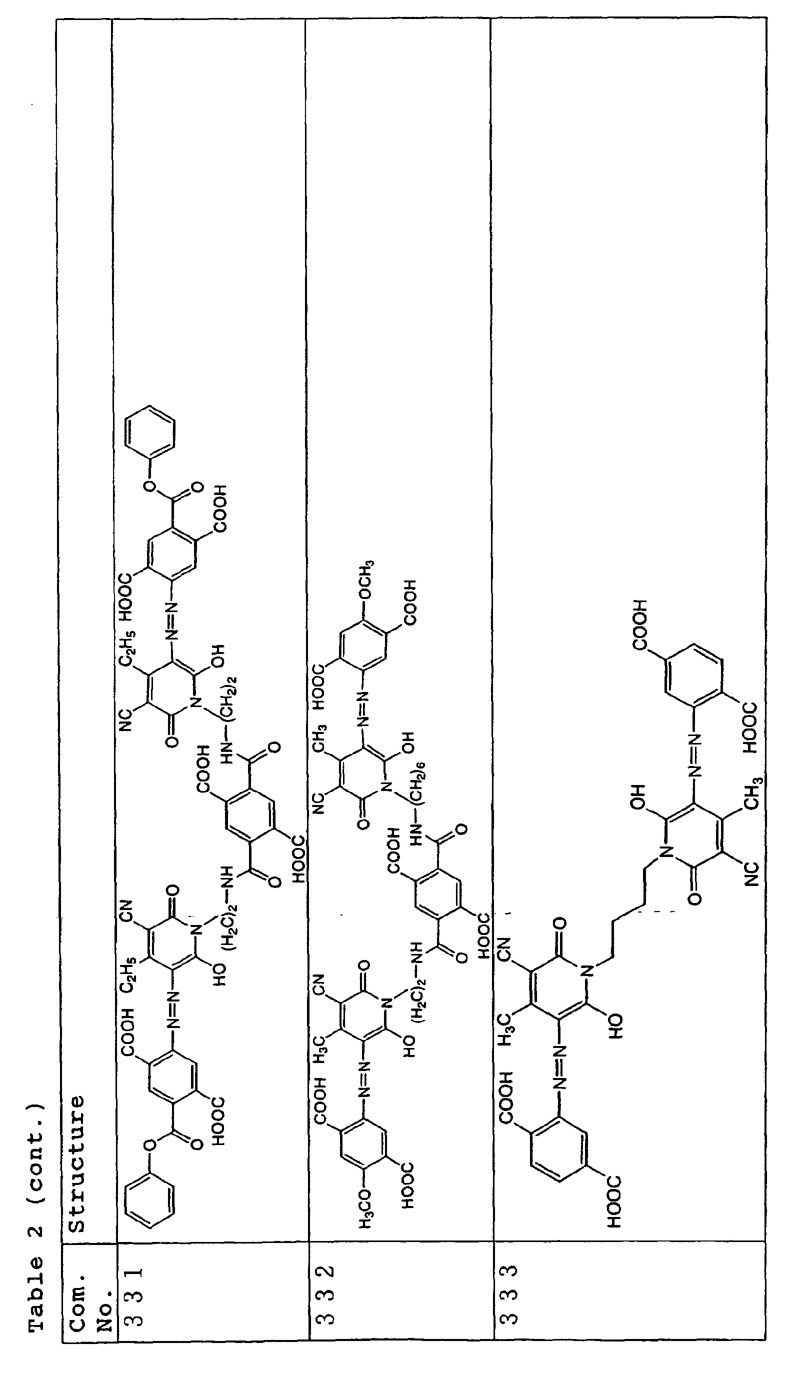

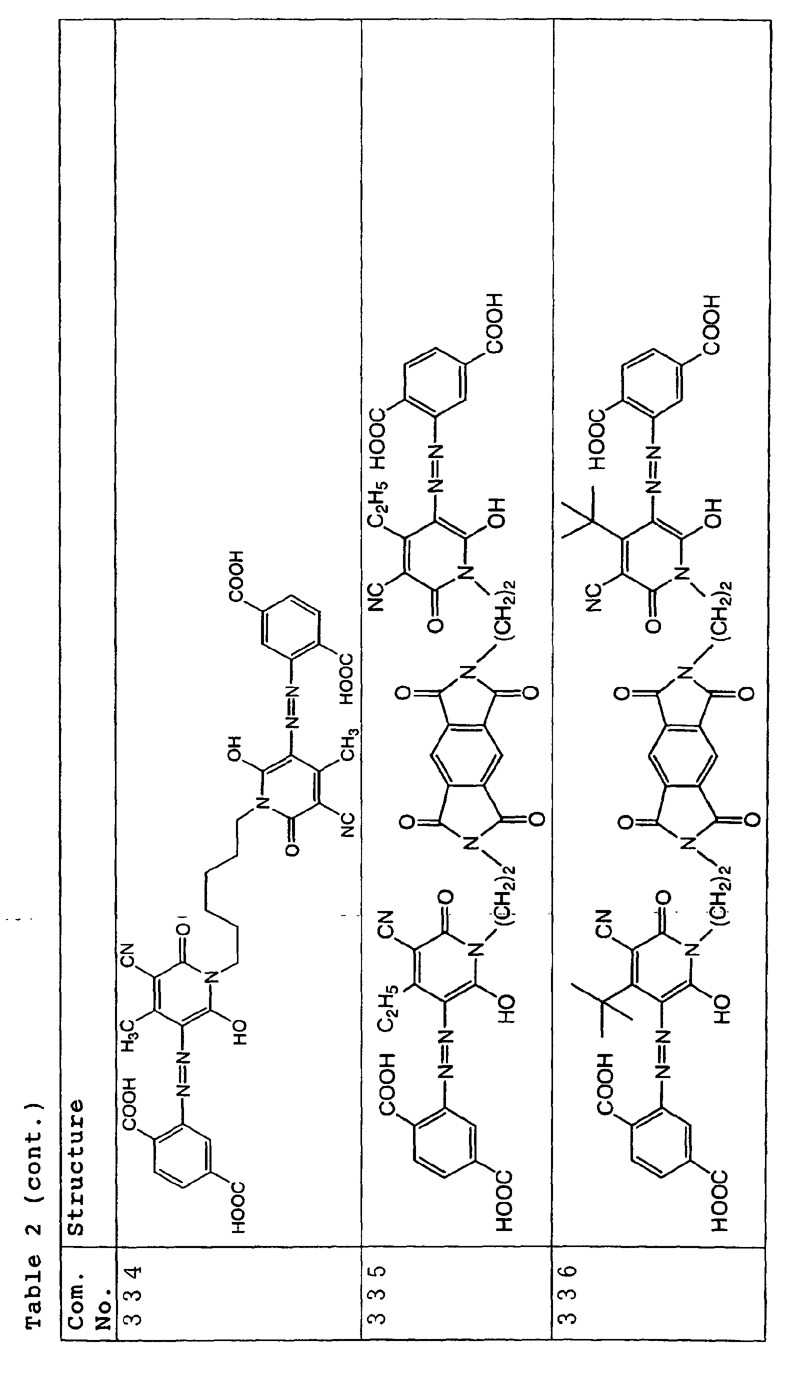

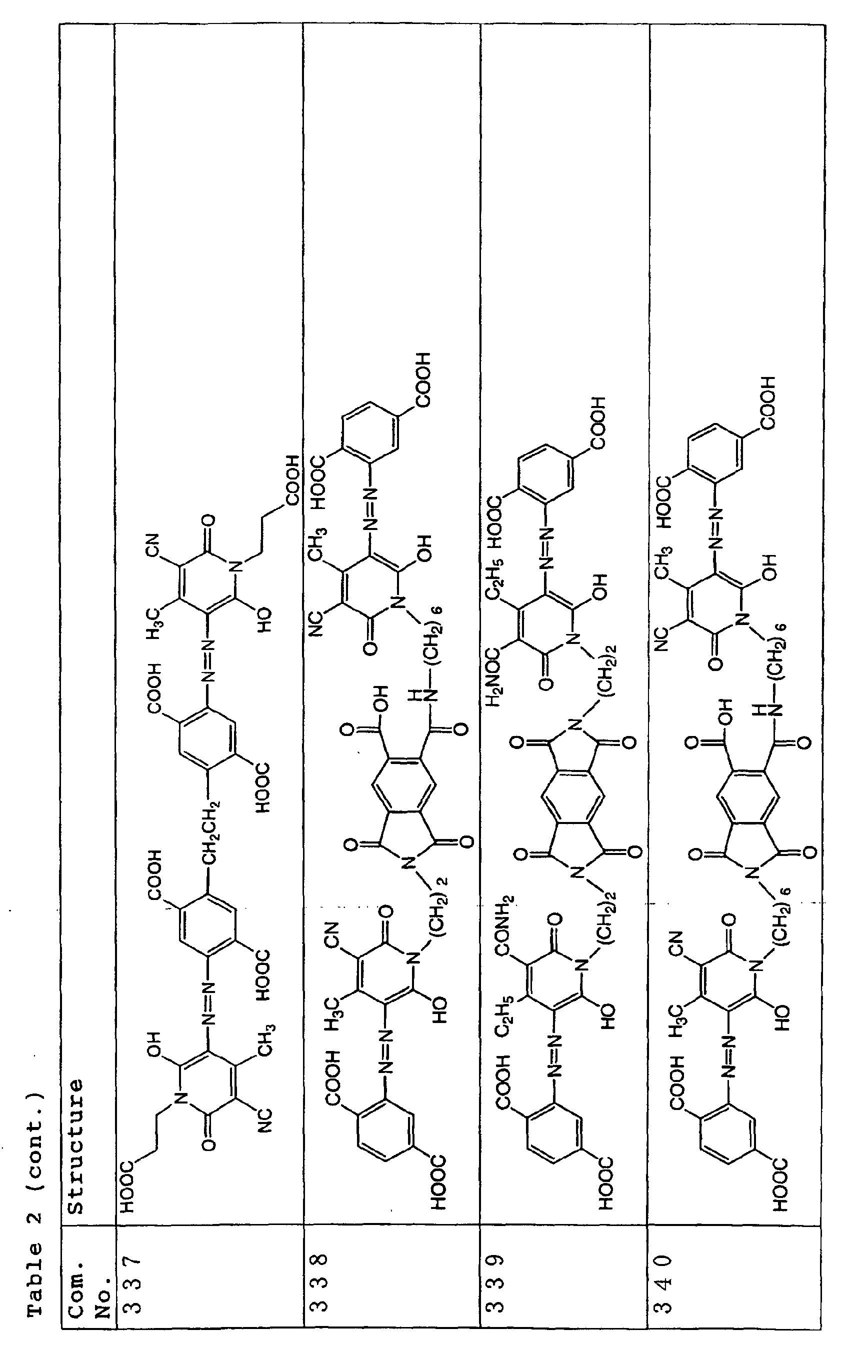

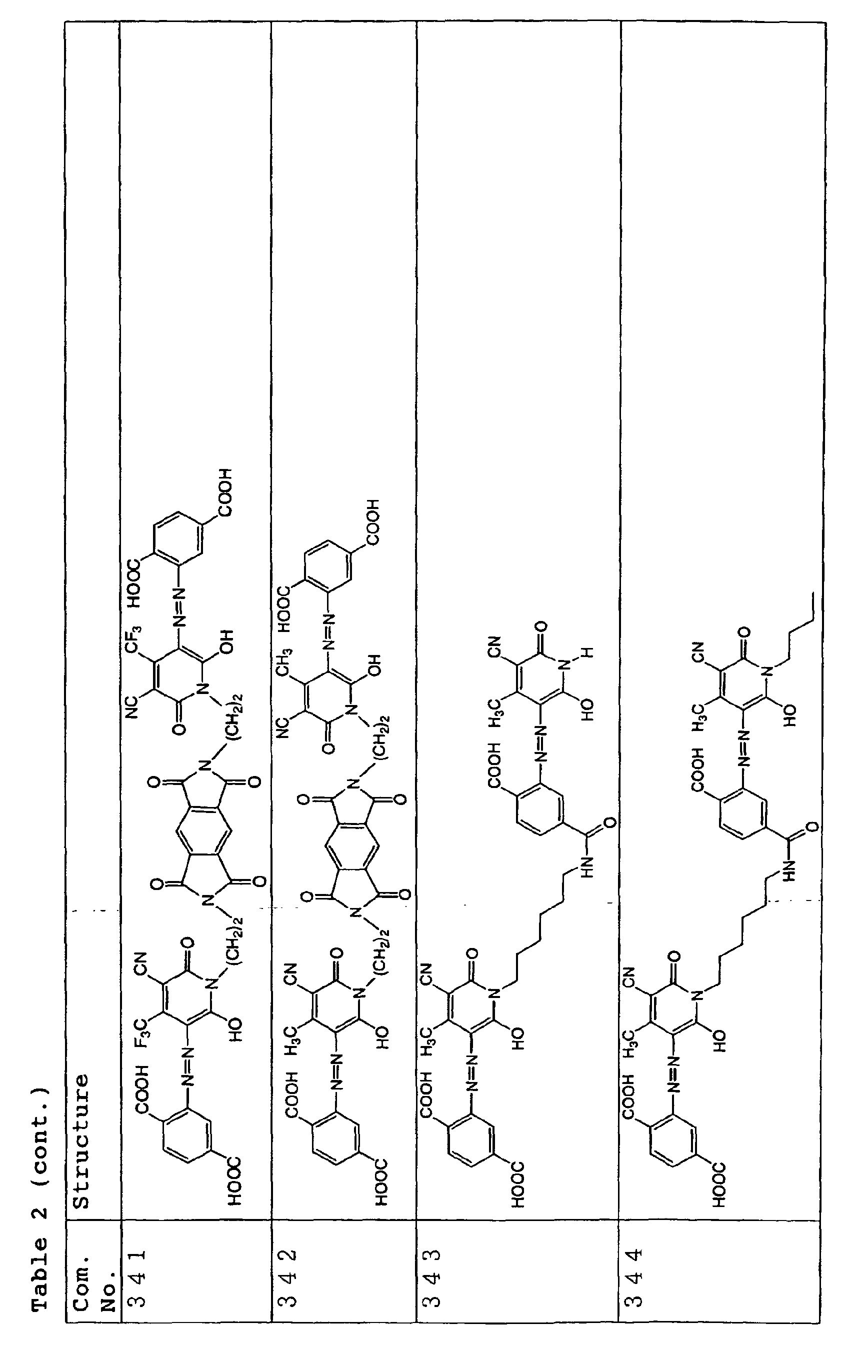

The compounds represented by general formula (1) which are contained in the aqueous ink of the invention are, structurally preferably in view of the moisture resistance of ink, the compounds having two to four structures, more preferably the compounds having two or three structures, further preferably the compounds having two structures represented by general formula (1) in the molecule.

In view of the water solubility of ink, one or more of substituents of R₁ to R₅ and R₇ are preferably polar groups (specifically, -OH, -COOH, -SO₃H, substituents having these groups and salts thereof), more preferably -COOH, -SO₃H, substituents having these groups and salts thereof. As a group with a property consistent with the moisture resistance, -COOH, substituents having -COOH and salts thereof are especially preferable.

In view of the light fastness of ink, the compounds that satisfy either of the conditions that (1) R₅ is an unsubstituted or substituted alkyl group, an unsubstituted or substituted aryl group or salts thereof and (2) R₇ is a group having an amide bond or an imide bond (including a salt and a bonding group) are preferable, and the compounds that satisfy both of them are more preferable.

The compounds that satisfy all of the conditions that (1) two structures represented by general formula (1) and four to six -COOHs (or salts thereof) are present in the molecule, (2) R₅ is an unsubstituted or substituted alkyl group, an unsubstituted or substituted aryl group, or salts thereof and (3) R₇ is a group having an amide bond or an imide bond (also including a salt or a bonding group) are especially preferable because they are excellent in all of the foregoing properties.

The structure represented by general formula (1) in the invention may be a structure represented by general formula (55) or the like through tautomerization.

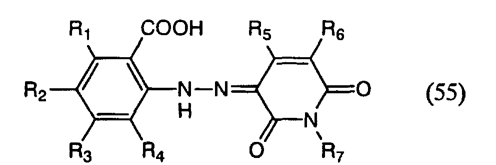

The compounds having the structure represented by general formula (1) in the molecule in the invention is produced according to the ordinary azo coupling method, for example, by coupling anilines represented by general formula (56) and pyridones represented by general formula (57). 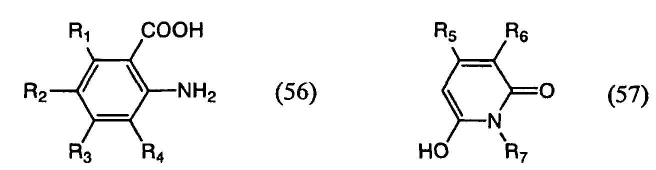 wherein R₁ to R₇ are the same as those in general formula (1).

The compounds having the structure represented by general formula (1) are specifically obtained by, for example, adding a sodium nitrite aqueous solution to anilines represented by general formula (56) in hydrochloric acid for diazotization, adding the diazotized compounds to pyridones represented by general formula (57) to conduct an azo coupling reaction, charging the reaction solution into a poor solvent after completion of the reaction and separating the resulting compounds by filtration.

The polymeric structures represented by general formula (1) can be produced by, for example, reacting compounds having an amino group in the end of any of substituents of R₁ to R₅ and R₇ with compounds having an acid anhydride in two or more positions.

The process for producing the compounds having the structure represented by general formula (1) is not limited to this process.

The aqueous ink of the invention can be used as various aqueous inks such as aqueous marker ink, aqueous printing ink, ink for a recording liquid in an ink-jet recording system and a dye liquid for ink-jet printing. It is especially useful as aqueous ink for a recording liquid in an ink-jet recording system.

The compounds having the structure represented by general formula (1) which are contained in the aqueous ink of the invention can be used as such. Further, when it is used as a recording liquid of an ink-jet recording system in particular, it may be used after conducting purification through desalting treatment with an ion exchange resin or by ultrafiltration or another desalting treatment in order to prevent clogging of jet nozzles of a recording apparatus due to inorganic salts or the like contained in a coloring matter.

The aqueous ink of the invention contains the compounds having the structure represented by general formula (1), water and, as required, an organic solvent or various additives.

As the aqueous ink, it is preferable that the compounds having the structure represented by general formula (1) are from 0.5 to 20% by weight, water is from 10 to 99.5% by weight and the organic solvent is from 0 to 80% by weight. It is more preferable that the compounds having the structure represented by general formula (1) are from 1 to 10% by weight, water is from 30 to 94% by weight and the organic solvent is from 5 to 60% by weight.

The compounds having the structure represented by general formula (1) may be used either singly or in combination.

For adjusting the hue of ink, the ink may contain other water-soluble dyes or known dyes or pigments in the form of an emulsion or in a finely dispersed state unless the characteristics of ink are impaired.

In the aqueous ink of the invention, the solvent component is mainly water. However, water may be used by being mixed with an organic solvent for preventing drying of the ink, improving the solubility of the compounds and the like.

Examples of the organic solvent which may be used in this case include polyhydric alcohols such as ethylene glycol, diethylene glycol, triethylene glycol, polyethylene glycol, propylene glycol, polypropylene glycol, 1,3-propanediol, glycerin and thioglycol,
polyhydric alcohol ethers such as ethylene glycol monoethyl ether, ethylene glycol monobutyl ether, diethylene glycol monobutyl ether, dipropylene glycol monoethyl ether, triethylene glycol monomethyl ether and triethylene glycol monobutyl ether,
ketones such as acetone and methyl ethyl ketone, amides such as N,N-dimethylformamide, N,N-diethylformamide and N,N-dimethylacetamide, nitrogen-containing compounds such as 2-pyrrolidone, N-methyl-2-pyrrolidone, N-vinyl-2-pyrrolidone and 1,3-dimethyl-2-imidazolidinone, ethers such as tetrahydrofuran and dioxane, and
alcohols such as methanol, ethanol, 1-propanol, 2-propanol, 1-butanol and 2-butanol.

These organic solvents may be used either singly or in admixture of two or more types. The use amount thereof is preferably from 0 to 80% by weight, more preferably from 5 to 60% by weight, further preferably from 10 to 40% by weight of the aqueous ink.

In the aqueous ink of the invention, ammonia, amines and hydroxyamines such as triethanolamine may be used in combination for improving the water resistance. The use amount thereof is preferably 10% by weight or less, more preferably from 0.1 to 5% by weight based on the aqueous ink. These amines may form a counter ion with the compounds represented by general formula (1).

Urea, thiourea and derivatives thereof may be used in combination. The use amount thereof is from 0.1 to 15% by weight, preferably from 3 to 10% by weight.

When the aqueous ink of the invention is used as ink for ink-jet recording, various additives which have been ordinarily used, such as an ultraviolet absorber, an antioxidant, a chelating agent, a water-soluble polymer, a masking agent, a rust-proofing agent, a preservative, a viscosity modifier, a surfactant, a surface tension modifier, a pH adjustor, a specific resistance modifier, a near-infrared absorber and a penetrant may be added as required.

The aqueous ink of the invention formed according to the foregoing formulation is excellent in recording characteristics, storage stability, fixability onto a recording medium, sharpness of a recorded image, light fastness and the like as ink for writing instruments and the like or ink of an ink-jet recording system.

The aqueous ink of the invention is produced by dissolving the compounds having the structure represented by general formula (1) along with water, the organic solvent and the additives listed above and then filtering the mixture through a filter having a micropore diameter, such as a membrane filter for removing insoluble matters. The thus-produced aqueous ink can be used as aqueous marker ink, aqueous printing ink, ink for a recording liquid of an ink-jet recording system, a dye liquid for ink-jet printing or the like. It is especially useful as a recording liquid of an ink-jet recording system. Moreover, it is used as ink of writing instruments such as a ball point pen, a felt tipped pen and a fountain pen, or for dyeing or printing of various fibers, papers, leathers, silks, resins and the like.

### Examples

The invention is illustrated more specifically below by referring to Examples. However, the invention is not limited to the following Examples.

Incidentally, "%" and "part(s)" indicate "% by weight" and "part(s) by weight" respectively.

### Example 1

### • Synthesis of pyridone compound (58)

A flask was charged with 50 parts of 28% aqueous ammonia, and 43 parts of ethyl cyanoacetate was added dropwise while being stirred. After 1 hour of the stirring, 56 parts of ethyl 3-oxobutyrate was added dropwise, and the mixture was stirred at 80°C for 3 hours. Subsequently, 30 parts of water and 27 parts of 95% sulfuric acid were charged, and the solution was stirred at 50°C for 30 minutes, and then discharged into 600 parts of ice water. The resulting precipitate was separated by filtration, washed with water, and dried to obtain 45 parts of a pyridone compound of formula (58) (yield 80%).

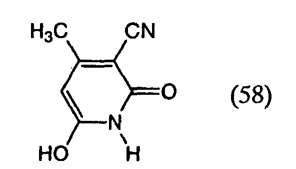

### • Synthesis of compound No. 11 (M₁ and M₂ are both a hydrogen atom)

A flask was charged with 6.6 parts of 2-aminoterephthalic acid, 50 parts of water and 10 parts of 36% hydrochloric acid. The mixture was then stirred, and cooled to 10°C or less. 9.2 parts of a 30% sodium nitrite aqueous solution was added dropwise thereto, and the mixture was stirred for 1 hour. Subsequently, 0. 2 parts of sulfamic acid was added, and the solution was stirred for 30 minutes (diazonium salt solution). Meanwhile, 50 parts of water, 5.5 parts of the compound of formula (58) and 5.3 parts of a 30% sodium hydroxide aqueous solution were cooled to 10°C or less while being mixed. The foregoing diazonium salt solution was added thereto, and a reaction was performed for 3 hours. Then, 200 parts of water was charged therein, and the mixture was stirred for 30 minutes. Thereafter, the resulting precipitate was separated by filtration, washed with water, and dried to obtain 11 parts of a pyridone azo compound being compound No. 11 in Table 1 (yield 89%). The elemental analysis values of this compound were as shown below.

| | C % | H % | N % |
|---|---|---|---|
| Calculated | 52.64 | 2.94 | 16.37 |
| Found | 52.58 | 2.97 | 16.40 |

Further, the results of the mass spectrum were as shown below.

| | |
|---|---|
| Calculated: 342 | Found: 342 |

### • Synthesis of a potassium salt of compound No. 11

A flask was charged with 50 parts of distilled water and 3.0 parts of compound No. 11, and the mixture was heated at 70°C while being stirred. 17 parts of a 10% potassium hydroxide aqueous solution was added thereto dropwise, and the mixture was stirred for 2 hours. This reaction solution was discharged into 700 parts of acetone. The resulting precipitate was separated by filtration, washed with acetone, and dried to obtain 3.7 parts of a potassium salt of compound No. 11 (yield 100%).

The maximum absorption wavelength of this salt in the aqueous solution was 441 nm, and the gram absorption coefficient was 85,000 ml/g·cm. The solubility in water was 3% or more.

### • Preparation of ink

According to the following formulation, the respective compounds were mixed and dissolved, and the mixture was filtered with a membrane filter having a pore diameter of 0.45 micron to prepare ink.

| | |
|---|---|
| potassium salt of compound No. 11 | 3% |
| diethylene glycol | 30% |
| N-methyl-2-pyrrolidone | 10% |
| distilled water | 57% |

### • Evaluation of characteristics

The prepared ink was filled in an ink cartridge for a piezo-type ink-jet printer EM-930C (manufactured by Seiko Epson). The printing was performed with this printer, and the light fastness was evaluated. Further, the storage stability of the ink was evaluated.

The results were good as shown below.

| | | |
|---|---|---|
| (A)-1 | Evaluation of light fastness-1 | ⓞ |
| (A)-2 | Evaluation of light fastness-2 | ⓞ |
| (B) | Evaluation of moisture resistance | ○ |
| (C) | Evaluation of ink storage stability | ○ |

The evaluation criteria of the respective test items are as follows.

### (A)-1 Evaluation of light fastness-1

Printing density (OD value) was measured after irradiating a printed portion with light for 100 hours using a xenon fadeometer (manufactured by Suga Shikenki) to evaluate the light fastness.

### Evaluation criteria:

| | |
|---|---|
| OD value is at least 80% of a value before irradiation | ⓞ |
| OD value is at least 70% and less than 80% of a value before irradiation | ○ |
| OD value is at least 50% and less than 70% of a value before irradiation | Δ |
| OD value is less than 50% of a value before irradiation | × |

### (A)-2 Evaluation of light fastness-2

Printing density (OD value) was measured after irradiating a printed portion with light for 400 hours using a xenon fadeometer (manufactured by Suga Shikenki), and the light fastness was evaluated.

### Evaluation criteria:

| | |
|---|---|
| OD value is at least 90% of a value before irradiation | ⓞⓞ |
| OD value is at least 80% and less than 90% of a value before irradiation | ⓞ |
| OD value is at least 70% and less than 80% of a value before irradiation | ○ |
| OD value is at least 50% and less than 70% of a value before irradiation | Δ |
| OD value is less than 50% of a value before irradiation | × |

### (B) Evaluation of moisture resistance

An image-recorded paper was allowed to stand under conditions of a temperature of 40°C and a humidity of 85% for a fixed period of time, and a condition of the recorded image was visually observed to evaluate the moisture resistance.

### Evaluation criteria:

| | |
|---|---|
| An image is unchanged after the paper is allowed to stand for 1 week | ⓞ |
| An image is unchanged after the paper is allowed to stand for 48 hours, and a coloring matter is bled in an edge portion of the recorded image after the paper is allowed to stand for 1 week | ○ |
| An image is unchanged after the paper is allowed to stand for 24 hours, and a coloring matter is bled in an edge portion of the recorded image after the paper is allowed to stand for 48 hours | Δ |
| A coloring matter is bled in an edge portion of the recorded image after the paper is allowed to stand for 24 hours | × |

### (C) Evaluation of storage stability of ink:

After a recording liquid was stored at 40°C for 3 months, it was observedwhether or not an insoluble matter was precipitated, and whether or not clogging occurred after continuous recording with the foregoing printer for a long period of time.

### Evaluation criteria:

| | |
|---|---|
| In a recording liquid, precipitation of an insoluble matter is not observed, nor is clogging observed | ○ |
| In a recording liquid, precipitation of an insoluble matter is observed, but clogging is not observed | Δ |
| In a recording liquid, precipitation of an insoluble matter is observed and clogging is also observed | × |

### Example 2

### • Synthesis of a tetramethylammonium salt of compound No. 11

A flask was charged with 50 parts of distilled water and 3.0 parts of a pyridone azo compound being compound No. 11 synthesized in Example 1, and the mixture was heated at 70°C while being stirred. 16 parts of a 10% tetramethylammonium hydroxide aqueous solution was added thereto dropwise, and the mixture was stirred for 5 hours. The reaction solution was discharged into 600 parts of acetone. The resulting precipitate was separated by filtration, washed with acetone, and dried to obtain 2.5 parts of a tetramethylammonium salt of compound No. 11 (yield 57%).

The maximum absorption wavelength of this salt in the aqueous solution was 441 nm, and the gram absorption coefficient was 71,400 ml/g·cm. The solubility in water was 3% or more.

### • Preparation of ink

Ink was prepared in the same manner as in Example 1 except that a tetramethylammonium salt of compound No. 11 was used instead of a potassium salt of compound No. 11.

### • Evaluation of characteristics

The same tests as in Example 1 were performed using the prepared ink. The results were good as shown below.

| | | |
|---|---|---|
| (A)-1 | Evaluation of light fastness-1 | ⓞ |
| (A)-2 | Evaluation of light fastness-2 | ⓞ |
| (B) | Evaluation of moisture resistance | ○ |
| (C) | Evaluation of ink storage stability | ○ |

### Example 3

### • Synthesis of compound No. 1 (M₁ and M₂ are both a hydrogen atom)

A flask was charged with 6.6 parts of 3-aminophthalic acid, 50 parts of water and 10 parts of 36% hydrochloric acid, and the mixture was stirred, and cooled to 10°C or less. 9.2 parts of a 30% sodium nitrite aqueous solution was added thereto dropwise. After the mixture was stirred for 2 hours, 0.2 part of sulfamic acid was added, and the solution was stirred for 30 minutes (diazonium salt solution). Meanwhile, 50 parts of water, 5.5 parts of the compound of formula (58) synthesized in Example 1 and 5.3 parts of a 30% sodium hydroxide aqueous solution were cooled to 10°C or less while being mixed. The foregoing diazonium salt solution was added thereto, and a reaction was conducted for 3 hours. Then, 200 parts of water was charged, and the mixture was stirred for 30 minutes. The resulting precipitate was then separated by filtration, washed with water, and dried to obtain 12 parts of a pyridone azo compound being compound No. 1 in Table 1 (yield 96%). The elemental analysis values of this compound were as shown below.

| | C % | H % | N % |
|---|---|---|---|
| Calculated | 52.64 | 2.94 | 16.37 |
| Found | 52.60 | 2.97 | 16.41 |

Further, the results of the mass spectrum were as shown below.

| | |
|---|---|
| Calculated: 342 | Found: 342 |

### • Synthesis of a sodium salt of compound No. 1

A flask was charged with 50 parts of distilled water and 3.0 parts of compound No. 1, and the mixture was heated at 70°C while being stirred. 12 parts of a 10% sodium hydroxide aqueous solution was added thereto dropwise, and the mixture was stirred for 2 hours. This reaction solution was discharged into 700 parts of acetone. The resulting precipitate was separated by filtration, washed with acetone, and dried to obtain 3.3 parts of a sodium salt of compound No. 1 (yield 97%).

The maximum absorption wavelength of this salt in the aqueous solution was 442 nm, and the gram absorption coefficient was 84,000 ml/g·cm. The solubility in water was 3% or more.

### • Preparation of ink

Ink was prepared in the same manner as in Example 1 except that the sodium salt of compound No. 1 was used instead of the potassium salt of compound No. 11.

### • Evaluation of characteristics

The same tests as in Example 1 were performed using the prepared ink. The results were good as shown below.

| | | |
|---|---|---|
| (A)-1 | Evaluation of light fastness-1 | ⓞ |
| (A)-2 | Evaluation of light fastness-2 | ⓞ |
| (B) | Evaluation of moisture resistance | ○ |
| (C) | Evaluation of ink storage stability | ○ |

### Example 4

### • Synthesis of a tetrabutylammonium salt of compound No. 1

A flask was charged with 50 parts of distilled water and 3.0 parts of the pyridone azo compound being compound No. 1 synthesized in Example 3, and the mixture was heated at 70°C while being stirred. 48 parts of a 10% tetrabutylammonium hydroxide aqueous solution was added thereto dropwise, and the mixture was stirred for 5 hours. This reaction solution was discharged into 600 parts of acetone, and the resulting precipitate was separated by filtration, washed with acetone, and dried to obtain 5.3 parts of a tetrabutylammonium salt of compound No. 1 (yield 73%).

The maximum absorption wavelength of this salt in the aqueous solution was 442 nm, and the gram absorption coefficient was 55,000 ml/g·cm. The solubility in water was 3% or more.

### • Preparation of ink

Ink was prepared in the same manner as in Example 1 except that the tetrabutylammonium salt of compound No. 1 was used instead of the sodium salt of compound No. 11.

### • Evaluation of characteristics

The same tests as in Example 1 were performed using the prepared ink. The results were good as shown below.

| | | |
|---|---|---|
| (A)-1 | Evaluation of light fastness-1 | ⓞ |
| (A)-2 | Evaluation of light fastness-2 | ⓞ |
| (B) | Evaluation of moisture resistance | ○ |
| (C) | Evaluation of ink storage stability | ○ |

### Examples 5 to 46

Inks were prepared as in Example 1 using the various compounds and their salts of the invention listed in Table 1, and the light fastness, the moisture resistance and the storage stability were evaluated. Consequently, it was found, as shown in Table 3, that the inks obtained by using the compounds and their salts of the invention exhibited the storage stability over a long period of time, the excellent light fastness and the excellent moisture resistance.

In Table 3, M₁ represents the type of the salt when -COOH in the structural formulas represented by general formulas (1) and (2) forms a salt, and M₂, M₃ and M₄ represent the type of the salt when R₁, R₂, R₃ and/or R₄ of the compounds listed in Table 1 are a hydrogen atom, a hydroxyl group, -COOH or -SO₃H.

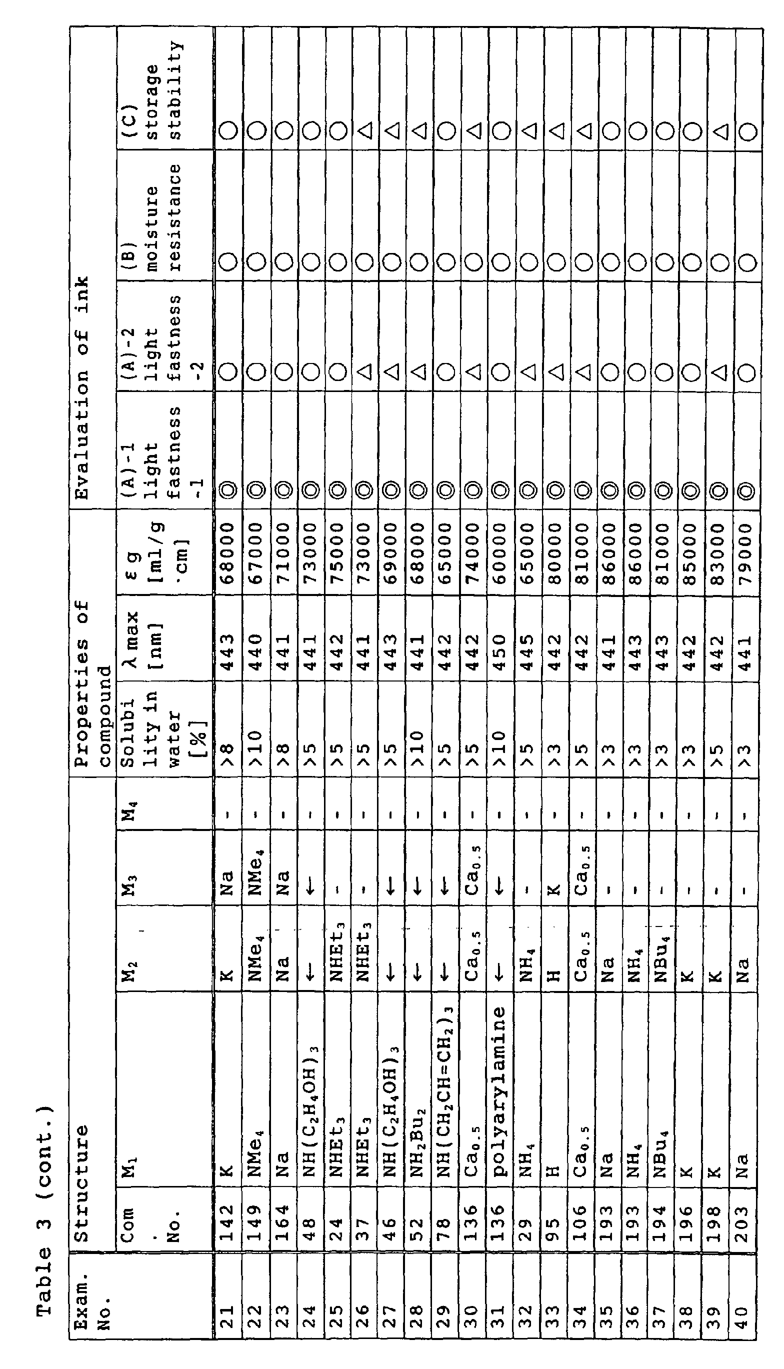

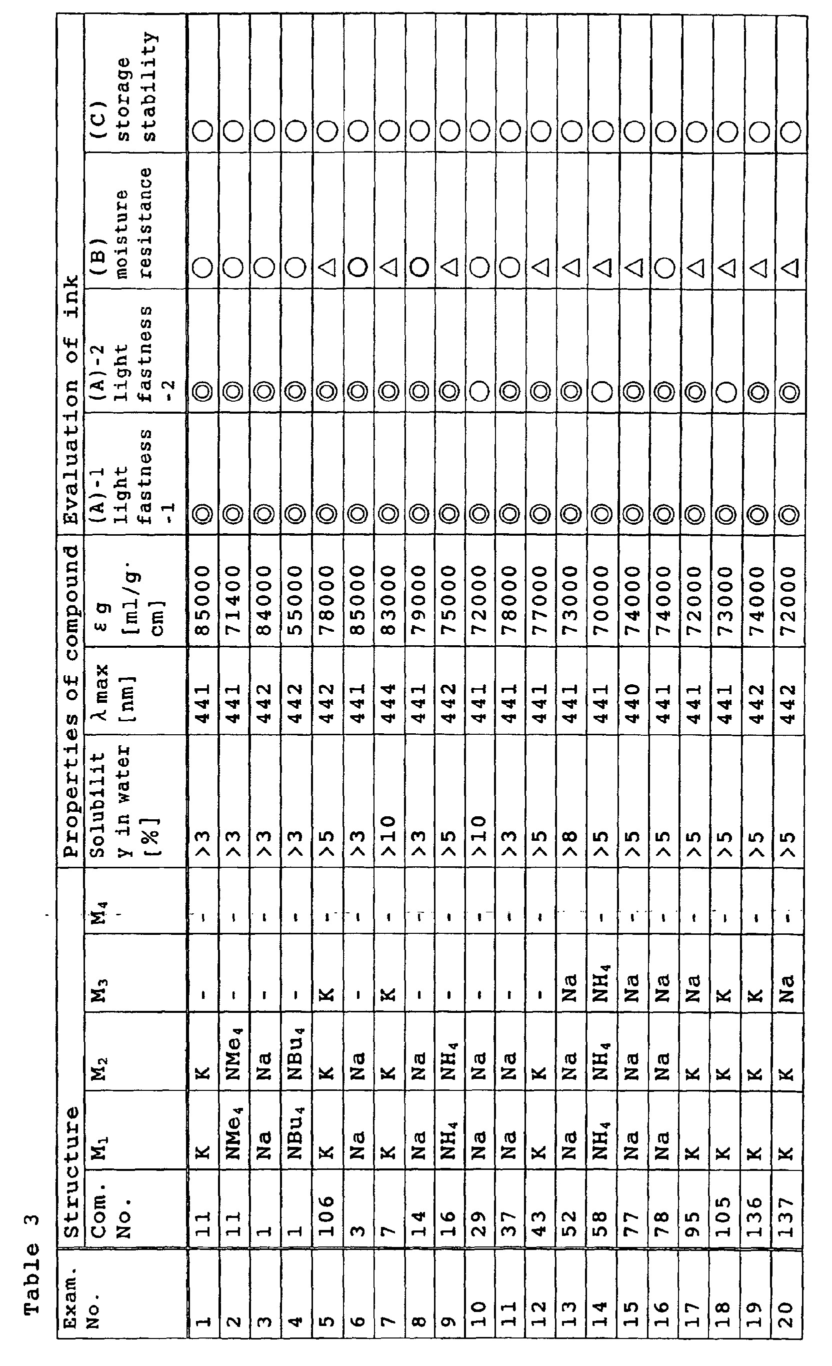

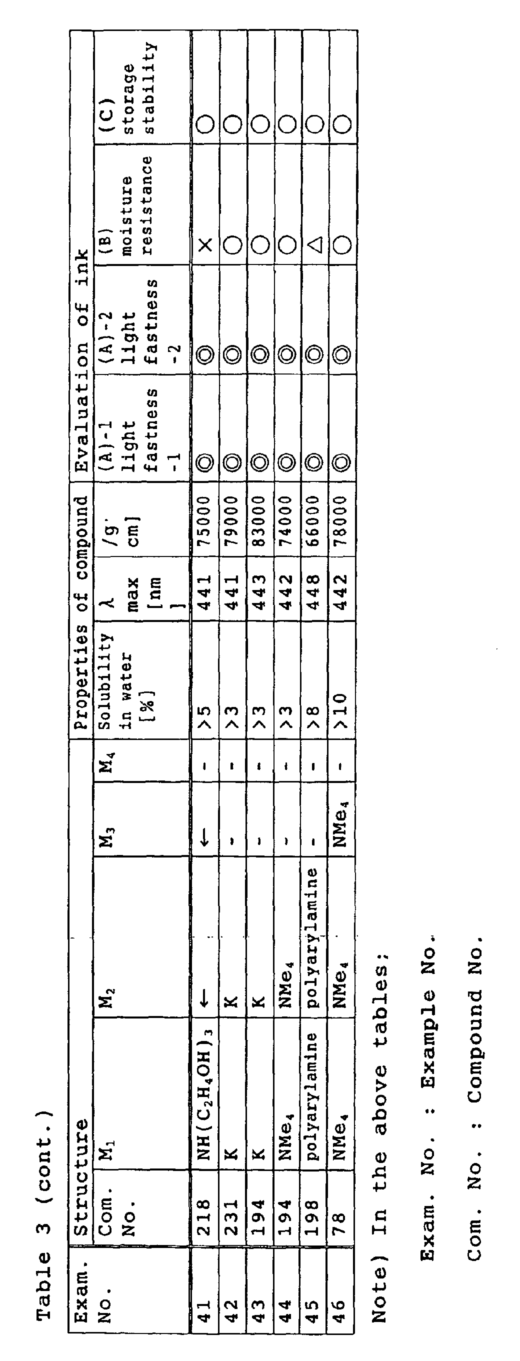

### Example 47

### • Synthesis of a compound of formula (59)

A flask was charged with 50 parts of ethanol and 108 parts of ethylenediamine, and the solution was stirred. 68 parts of ethyl cyanoacetate was added thereto dropwise, and the mixture was stirred for 5 hours. Subsequently, the reaction solution was heated to 90°C while 70 parts of methyl 3-oxobutyrate was added thereto dropwise, and the mixture was stirred for 11 hours. The resulting mixture was allowed to cool, then discharged into 500 parts of ethanol, and stirred for 1 hour. The resulting precipitate was separated by filtration, washed with ethanol, and dried to obtain 56 parts of a pyridone compound represented by formula (59)(yield 48%).

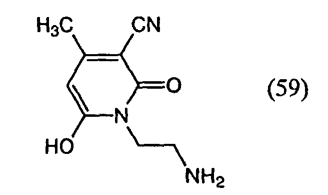

### • Synthesis of an intermediate pyridone azo compound of formula (60)

A flask was charged with 30 parts of 2-aminoterephthalic acid, 240 parts of water and 46 parts of 36% hydrochloric acid, and the mixture was stirred, and cooled to 10°C or less. An aqueous solution of 13 parts of sodium nitrite in 50 parts of water was added thereto dropwise. The resulting mixture was stirred at 10°C or less for 3 hours, 1.2 parts of sulfamic acid was then added thereto, and the solution was stirred for 30 minutes (diazonium salt solution). Meanwhile, 240 parts of water, 32 parts of the compound represented by formula (59) and 24 parts of a 30% sodium hydroxide aqueous solution were charged into a flask, and cooled to 10°C or less while being stirred. The foregoing diazonium salt solution was added thereto, and the mixture was stirred at 10°C or less for 2 hours. This reaction solution was discharged into 1,000 parts of water, and the mixture was stirred for 1 hour. Then, pH was adjusted to 2 or less with hydrochloric acid, and the solution was stirred for 1 hour. The resulting precipitate was separated by filtration, washed with water, and dried to obtain 60 parts of a pyridone azo compound represented by formula (60)(yield 86%).

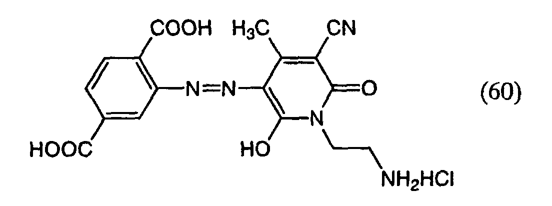

The elemental analysis values of this compound were as shown below.

| | C % | H % | N % |
|---|---|---|---|
| Calculated | 48.46 | 3.82 | 16.60 |
| Found | 48.40 | 3.84 | 16.36 |

Further, the results of the mass spectrum were as shown below.

| | |
|---|---|
| Calculated: m/z=421 | Found: m/z=421 |

### • Synthesis of compound No. 245

A flask was charged with 44 parts of cyanuric chloride and 100 parts of acetone, and the mixture was heated to 50°C while being stirred. After this solution was charged into 1,000 parts of ice water stirred, 126 parts of a 30% sodium hydroxide aqueous solution was added thereto. While the temperature was kept at 5°C or less, 100 parts of the compound represented by formula (60) was gradually added, and the mixture was stirred at 5°C or less for 2 hours. 126 parts of a 30% sodium hydroxide aqueous solution was added thereto. Then, while the temperature was kept at 5°C or less, 100 parts of the compound represented by formula (60) was gradually charged therein, and the mixture was stirred at 5°C or less for 1 hour. Subsequently, the solution was heated to 40°C, stirred at from 40°C to 50°C for 8 hours, then allowed to cool, adjusted to pH of 3 or less with hydrochloric acid, and stirred for 1 hour. The resulting precipitate was separated by filtration, washed with water, and dried to obtain 170 parts of compound No. 245 in Table 2 (yield 80%).

The elemental analysis values of this compound were as shown below.

| | C % | H % | N % |
|---|---|---|---|
| Calculated | 50.38 | 3.20 | 20.64 |
| Found | 50.26 | 3.27 | 20.43 |

Further, the results of the mass spectrum were as shown below.

| | |
|---|---|
| Calculated: m/z=882 | Found: m/z=882 |

### • Synthesis of a sodium salt of compound No. 245

A flask was charged with 36 parts of distilled water and 3.0 parts of compound No. 245 in Table 1, and the mixture was heated at 70°C while being stirred. 1.8 parts of a 30% sodium hydroxide aqueous solution was added thereto dropwise, and the resulting mixture was stirred for 2 hours. This reaction solution was discharged into 400 parts of acetone, and the resulting precipitate was separated by filtration, washed with acetone, and dried to obtain 3.3 parts of a sodium salt of compound No. 245 in Table 2 (yield 100%).

The maximum absorption wavelength of this salt in the aqueous solution was 440 nm, and the gram absorption coefficient was 83,000 ml/g·cm. The solubility in water was 3% or more.

### • Preparation of ink

Ink was prepared in the same manner as in Example 1 except that the sodium salt of compound No. 245 was used instead of the potassium salt of compound No. 11.

### • Evaluation of characteristics

The same tests as in Example 1 were performed using the prepared ink.

The results were good as shown below.

| | | |
|---|---|---|
| (A)-1 | Evaluation of light fastness-1 | ⓞ |
| (A)-2 | Evaluation of light fastness-2 | ○ |
| (B) | Evaluation of moisture resistance | ⓞ |
| (C) | Evaluation of ink storage stability : | ○ |

### Example 48

### • Synthesis of a potassium salt of compound No. 245

A flask was charged with 36 parts of distilled water and 3.0 parts of compound No. 245 in Table 2, and the mixture was heated at 70°C while being stirred. 2.5 parts of a 30% potassium hydroxide aqueous solution was added thereto, and the resulting mixture was stirred for 2 hours. This reaction solution was discharged into 400 parts of acetone, and the resulting precipitate was separated by filtration, washed with acetone, and dried to obtain 3.5 parts of a potassium salt of compound No. 245 in Table 2 (yield 100%).

The maximum absorption wavelength of this potassium salt in the aqueous solution was 442 nm, and the gram absorption coefficient was 81,000 ml/g·cm. The solubility in water was 3% or more.

### • Preparation of ink

Ink was prepared in the same manner as in Example 1 except that the potassium salt of compound No. 245 was used instead of the potassium salt of compound No. 11.

### • Evaluation of characteristics

The same tests as in Example 1 were performed using the prepared ink.

The results were good as shown below.

| | | |
|---|---|---|
| (A)-1 | Evaluation of light fastness-1 | ⓞ |
| (A)-2 | Evaluation of light fastness-2 | ○ |
| (B) | Evaluation of moisture resistance | ⓞ |
| (C) | Evaluation of ink storage stability | ○ |

### Example 49

### • Synthesis of a tetramethylammonium salt of compound No. 245

A flask was charged with 36 parts of distilled water and 3.0 parts of compound No. 245 in Table 2, and the mixture was stirred. 13 parts of a 10% tetramethylammonium hydroxide aqueous solution was added thereto, and the resulting mixture was stirred for 2 hours. This reaction solution was discharged into 400 parts of acetone, and the resulting precipitate was separated by filtration, washed with acetone, and dried to obtain 3.0 parts of a tetramethylammonium salt of compound No. 245 (yield 76%).

The maximum absorption wavelength of this tetramethylammonium salt in the aqueous solution was 442 nm, and the gram absorption coefficient was 69,000 ml/g·cm. The solubility in water was 5% or more.

### • Preparation of ink

Ink was prepared in the same manner as in Example 1 except that the tetramethylammonium salt of compound No. 245 was used instead of the potassium salt of compound No. 11.

### • Evaluation of characteristics

The same tests as in Example 1 were performed using the prepared ink.

The results were good as shown below.

| | | |
|---|---|---|
| (A)-1 | Evaluation of light fastness-1 | ⓞ |
| (A)-2 | Evaluation of light fastness-2 | ○ |
| (B) | Evaluation of moisture resistance | ⓞ |
| (C) | Evaluation of ink storage stability | ○ |

### Example 50

### • Synthesis of a compound of formula (61)

A flask was charged with 100 parts of ethanol, 100 parts of hexamethylenediamine and 37 parts of piperidine, and the mixture was stirred. 41 parts of ethyl cyanoacetate was added thereto dropwise, and the solution was stirred for 19 hours. Subsequently, 42 parts of methyl 3-oxobutyrate was added thereto dropwise, and the mixture was stirred for 3 hours. The temperature was then elevated to 100°C, and the mixture was stirred at 100°C for 5 hours. The reaction solution was allowed to cool, then discharged into 2,000 parts of water, and stirred for 1 hour. This solution was concentrated, and the crystals precipitatedwerefiltered, washed with water, and dried to obtain 25 parts of a compound represented by formula (61) (yield 28%).

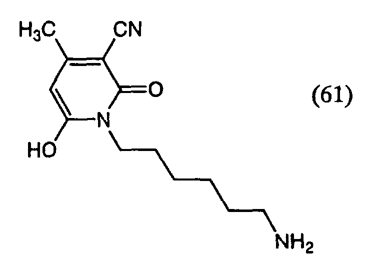

### • Synthesis of a pyridone azo compound of formula (62)

A flask was charged with 7.3 parts of 2-aminoterephthalic acid, 30 parts of water and 12 parts of 36% hydrochloric acid, and the mixture was then stirred, and cooled to 10°C or less. An aqueous solution of 3.1 parts of sodium nitrite in 15 parts of water was added thereto dropwise, and the mixture was stirred at 10°C or less for 3 hours. Then, 1 part of sulfamic acid was added thereto, and the solution was stirred for 30 minutes (diazonium salt solution). Meanwhile, 100 parts of water and 10 parts of a compound represented by formula (62) were charged into a flask, and cooled to 10°C or less while being stirred. The foregoing diazonium salt solution was gradually added thereto while pH in the flask was kept at from 3 to 4 with hydrochloric acid or a sodium hydroxide aqueous solution, and the mixture was stirred at 10°C or less for 3 hours. After pH was adjusted to 1 or less with hydrochloric acid, the reaction solution was discharged into ethanol, and the crystals precipitated were separated by filtration, washed with ethanol, and dried to obtain 13 parts of the pyridone azo compound represented by formula (62) (yield 67%).

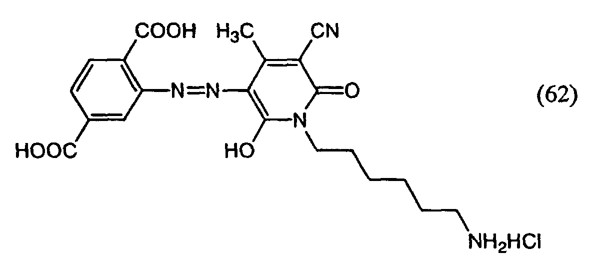

### • Synthesis of compound No. 246

A flask was charged with 3.9 parts of cyanuric chloride and 8 parts of acetone, and the mixture was heated to 50°C while being stirred. This solution was charged into 80 parts of ice water stirred, and 11 parts of a 30% sodium hydroxide aqueous solution was then added thereto. While the temperature was kept at 5°C or less, 10 parts of the compound represented by formula (62) was gradually added, and the mixture was stirred at 5°C or less for 2 hours. 11 parts of a 30% sodium hydroxide aqueous solution was added thereto, and 10 parts of the compound represented by formula (62) was then gradually charged therein. Thereafter, the mixture was heated to 50°C, stirred at from 50°C to 60°C for 7 hours, allowed to cool, and discharged into 300 parts of water. While the mixture was stirred, pH was adjusted to 1 or less with hydrochloric acid, and the crystals precipitated were separated by filtration, washed with water, and dried to obtain 18 parts of compound No. 246 in Table 2 (yield 86%).

The elemental analysis values of this compound were as shown below.

| | C % | H % | N % |
|---|---|---|---|
| Calculated | 54.35 | 4.46 | 18.31 |
| Found | 53.90 | 4.58 | 17.92 |

Further, the results of the mass spectrum were as shown below.

| | |
|---|---|
| Calculated: m/z=993 | Found: m/z=993 |

### • Synthesis of a tetramethylammonium salt of compound No. 246

A flask was charged with 36 parts of distilled water and 3.4 parts of compound No. 246 in Table 2, and the mixture was stirred. 13 parts of a 10% tetramethylammonium hydroxide aqueous solution was added thereto, and the resulting mixture was stirred for 2 hours. This reaction solution was discharged into 400 parts of acetone, and the resulting precipitate was separated by filtration, washed with acetone, and dried to obtain 4.1 parts of a tetramethylammonium salt of compound No. 246 (yield 93%).

The maximum absorption wavelength of this tetramethylammonium salt in the aqueous solution was 438 nm, and the gram absorption coefficient was 68,000 ml/g·cm. The solubility in water was 5% or more.

### • Preparation of ink

Ink was prepared in the same manner as in Example 1 except that the tetramethylammonium salt of compound No. 246 was used instead of the potassium salt of compound No. 11.

### • Evaluation of characteristics

The same tests as in Example 1 were performed using the prepared ink.

The results were good as shown below.

| | | |
|---|---|---|
| (A)-1 | Evaluation of light fastness-1 | ⓞ |
| (A)-2 | Evaluation of light fastness-2 | Δ |
| (B) | Evaluation of moisture resistance | ⓞ |
| (C) | Evaluation of ink storage stability | Δ |

### Example 51

### • Synthesis of compound No. 279

A flask was charged with 2.0 parts of the pyridone azo compound represented by formula (62) which was synthesized in Example 47, 10 parts of pyridine and 0.52 part of pyromellitic dianhydride, and the mixture was stirred for 3 hours. Subsequently, the reaction system was heated, and stirred at from 100 to 120°C for 37 hours. The reaction solution was cooled to room temperature, then discharged into a hydrochloric acid solution, and stirred. The crystals precipitated were separated by filtration, washed with water, and dried to obtain 2.2 parts of compound No. 279 in Table 2 (yield 94%).

The elemental analysis values of this compound were as shown below.

| | C % | H % | N % |
|---|---|---|---|
| Calculated | 53.45 | 3.26 | 14.17 |
| Found | 53.22 | 3.33 | 13.96 |

Further, the results of the mass spectrum were as shown below.

| | |
|---|---|
| Calculated: m/z=988 | Found: m/z=988 |

### • Synthesis of a sodium salt of compound No. 279

A flask was charged with 36 parts of distilled water and 3.3 parts of compound No. 279 in Table 2, and the mixture was heated at 70°C while being stirred. 1.8 parts of a 30% sodium hydroxide aqueous solution was added thereto, and the resulting mixture was stirred for 2 hours. This reaction solution was discharged into 400 parts of acetone, and the resulting precipitate was separated by filtration, washed with acetone, and dried to obtain 3.5 parts of a sodium salt of compound No. 279 (yield 97%).

The maximum absorption wavelength of this sodium salt in the aqueous solution was 438 nm, and the gram absorption coefficient was 81,000 ml/g·cm. The solubility in water was 3% or more.

### • Preparation of ink

Ink was prepared in the same manner as in Example 1 except that the sodium salt of compound No. 279 was used instead of the potassium salt of compound No. 11.

### • Evaluation of characteristics

The same tests as in Example 1 were performed using the prepared ink.

The results were good as shown below.

| | | |
|---|---|---|
| (A)-1 | Evaluation of light fastness-1 | ⓞ |
| (A)-2 | Evaluation of light fastness-2 | ⓞ |
| (B) | Evaluation of moisture resistance | ⓞ |
| (C) | Evaluation of ink storage stability | ○ |

### Examples 52 to 100

Inks were prepared as in Example 1 using the compounds of the invention listed in Table 2, and the light fastness, the moisture resistance and the storage stability were evaluated. Consequently, it was found, as shown in Table 4, that the inks obtained by using the compounds and their salts of the invention exhibited the storage stability over a long period of time, the excellent light fastness and the excellent moisture resistance.

**Table 4**

| Ex. | Compound No. | Type of salt | Evaluation of characteristics | | | |
|---|---|---|---|---|---|---|
| | | | (A)-1 Light fastness-1 | (A)-2 Light fastness-2 | (B) Moisture resistance | (C) Storage stability |
| 47 | 245 | sodium | ⓞ | ○ | ⓞ | ○ |
| 48 | 245 | potassium | ⓞ | ○ | ⓞ | ○ |
| 49 | 245 | tetramethylammonium | ⓞ | ○ | ⓞ | ○ |
| 50 | 246 | tetramethylammonium | ⓞ | Δ | ⓞ | Δ |
| 51 | 279 | sodium | ⓞ | ⓞ | ⓞ | 0 |
| 52 | 247 | tetramethylammonium | ⓞ | ○ | ⓞ | ○ |
| 53 | 248 | tetramethylammonium | ⓞ | ⓞ | ⓞ | Δ |
| 54 | 249 | tetramethylammonium | ⓞ | ○ | ⓞ | Δ |
| 55 | 252 | tetramethylammonium | ⓞ | ○ | ⓞ | Δ |
| 56 | 255 | tetramethylammonium | ⓞ | ○ | ⓞ | ○ |
| 57 | 256 | tetramethylammonium | ⓞ | ○ | ⓞ | Δ |
| 58 | 260 | tetramethylammonium | ⓞ | ○ | ⓞ | Δ |
| 59 | 261 | tetramethylammonium | ⓞ | ○ | ⓞ | Δ |
| 60 | 262 | tetramethylammonium | ⓞ | ○ | ⓞ | Δ |
| 61 | 264 | tetramethylammonium | ⓞ | ○ | ⓞ | Δ |
| 62 | 265 | tetramethylammonium | ⓞ | ⓞ | ⓞ | Δ |
| 63 | 267 | tetramethylammonium | ⓞ | ○ | ⓞ | Δ |
| 64 | 268 | tetramethylammonium | ⓞ | ○ | ⓞ | Δ |
| 65 | 270 | tetramethylammonium | ⓞ | ⓞ | ⓞ | Δ |
| 66 | 271 | tetramethylammonium | ⓞ | ⓞ | ⓞ | ○ |
| 67 | 273 | tetramethylammonium | ⓞ | ⓞ | ⓞ | ○ |
| 68 | 274 | tetramethylammonium | ⓞ | ○ | ⓞ | Δ |
| 69 | 278 | tetramethylammonium | ⓞ | ○ | ⓞ | Δ |
| 70 | 279 | tetramethylammonium | ⓞ | ⓞ | ⓞ | ○ |
| 71 | 280 | potassium | ⓞ | ⓞ | ⓞ | ○ |
| 72 | 280 | tetramethylammonium | ⓞ | ⓞ | ⓞ | ○ |
| 73 | 281 | tetramethylammonium | ⓞ | ○ | ⓞ | ○ |
| 74 | 272 | potassium | ⓞ | ○ | ⓞ | Δ |
| 75 | 283 | potassium | ⓞ | ⓞⓞ | ⓞ | ○ |
| 76 | 283 | tetramethylammonium | ⓞ | ⓞⓞ | ⓞ | ○ |
| 77 | 284 | tetramethylammonium | ⓞ | ⓞⓞ | ⓞ | ○ |
| 78 | 285 | tetramethylammonium | ⓞ | ⓞⓞ | ⓞ | ○ |
| 79 | 287 | tetramethylammonium | ⓞ | ⓞ | ⓞ | ○ |
| 80 | 291 | tetramethylammonium | ⓞ | ⓞⓞ | ⓞ | ○ |
| 81 | 92 | tetramethylammonium | ⓞ | ⓞⓞ | ⓞ | ○ |
| 82 | 293 | potassium | ⓞ | ⓞⓞ | ⓞ | ○ |
| 83 | 294 | tetramethylammonium | ⓞ | ○ | ⓞ | Δ |
| 84 | 2295 | tetramethylammonium | ⓞ | Δ | ⓞ | Δ |
| 85 | 296 | tetramethylammonium | ⓞ | ⓞ | ⓞ | ○ |
| 86 | 296 | sodium | ⓞ | ⓞ | ⓞ | ○ |
| 87 | 297 | tetramethylammonium | ⓞ | ⓞ | ⓞ | ○ |
| 88 | 297 | potassium | ⓞ | ⓞ | ⓞ | ○ |
| 89 | 299 | tetramethylammonium | ⓞ | ○ | ⓞ | ○ |
| 90 | 305 | tetramethylammonium | ⓞ | ⓞ | ⓞ | Δ |
| 91 | 308 | tetramethylammonium | ⓞ | ⓞ | ⓞ | Δ |
| 92 | 312 | tetramethylammonium | ⓞ | ⓞ | ⓞ | Δ |
| 93 | 321 | tetramethylammonium | ⓞ | ○ | ⓞ | Δ |
| 94 | 323 | tetramethylammonium | ⓞ | ⓞ | ⓞ | Δ |
| 95 | 323 | tetrabutylammonium | ⓞ | ⓞ | ⓞ | Δ |
| 96 | 250 | tetramethylammonium | ⓞ | ○ | ⓞ | Δ |
| 97 | 251 | tetramethylammonium | ⓞ | ⓞ | ⓞ | Δ |
| 98 | 335 | tetramethylammonium | ⓞ | ⓞⓞ | ⓞ | ○ |
| 99 | 336 | tetramethylammonium | ⓞ | ⓞⓞ | ⓞ | ○ |
| 100 | 342 | tetramethylammonium | ⓞ | ⓞ | ⓞ | ○ |

### Comparative Example 1

Ink was prepared by the method of preparing ink in Example 1 using a yellow azo dye (C. I. Acid Yellow 23), and the characteristics were also evaluated. The results were as follows.

| | | |
|---|---|---|
| (A)-1 | Evaluation of light fastness-1 | × |
| (A)-2 | Evaluation of light fastness-2 | × |
| (B) | Evaluation of moisture resistance | × |
| (C) | Evaluation of ink storage stability | ○ |

The light fastness and the moisture resistance were poor in comparison to those of the ink of the invention.

### Comparative Example 2

Ink was prepared by the method of preparing ink in Example 1 using a pyridone azo compound of formula (63), and the characteristics were also evaluated. 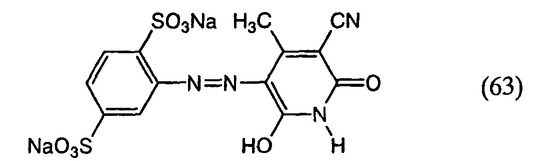 The results were as follows.

| | | |
|---|---|---|
| (A)-1 | Evaluation of light fastness-1 | × |
| (A)-2 | Evaluation of light fastness-2 | × |
| (B) | Evaluation of moisture resistance | × |
| (C) | Evaluation of ink storage stability | ○ |

The light fastness and the moisture resistance were poor in comparison to those of the ink of the invention.

### Industrial Availability

The aqueous ink which contains the compounds represented by general formula (1) in the invention has the clear hue, and is excellent in light fastness and storage stability. When it is used as ink for ink-jet recording in particular, the high-quality recording liquid excellent in moisture resistance and light fastness is provided.

## Claims

1. Aqueous ink which contains compounds having a structure represented by general formula (1) in a molecule or salts thereof 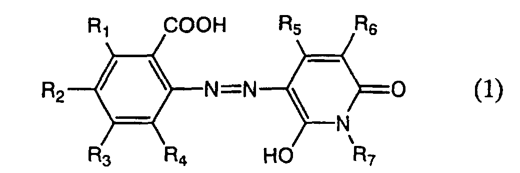 wherein
each of R₁ to R₅ and R₇ independently, represents a hydrogen atom, a halogen atom, an unsubstituted or substituted alkyl group, an unsubstituted or substituted alkoxy group, an unsubstituted or substituted alkenyl group, an unsubstituted or substituted aryl group, a hydroxyl group, -COOH, -SO₃H, -CONR₈R₉ in which each of R₈ and R₉ independently, represents a hydrogen atom, an unsubstituted or substituted alkyl group, an unsubstituted or substituted aryl group or an unsubstituted or substituted heterocyclic group and R₈ and R₉ may form a ring by an unsubstituted or substituted alkylene group, -COXR₁₀ in which R₁₀ represents a hydrogen atom, an unsubstituted or substituted alkyl group, an unsubstituted or substituted aryl group or an unsubstituted or substituted heterocyclic group and X represents an oxygen atom or a sulfur atom, or a divalent to tetravalent bonding group through which compounds represented by formula (1) are bonded to form a polymeric structure, and R₆ represents a hydrogen atom, a cyano group, an aminocarbonyl group, a sulfomethyl group or -SO₃H.

2. Aqueous ink which contains compounds having two or more groups represented by general formula (2) in a molecule or salts thereof 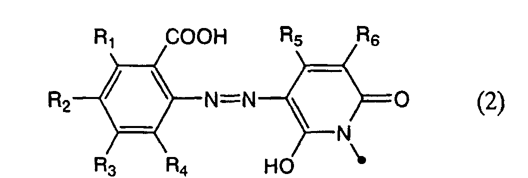 wherein
each of R₁ to R₅ independently, represents a hydrogen atom, a halogen atom, an unsubstituted or substituted alkyl group, an unsubstituted or substituted alkoxy group, an unsubstituted or substituted alkenyl group, an unsubstituted or substituted aryl group, a hydroxyl group, -COOH, -SO₃H, -CONR₈R₉ in which each of R₈ and R₉ independently, represents a hydrogen atom, an unsubstituted or substituted alkyl group, an unsubstituted or substituted aryl group or an unsubstituted or substituted heterocyclic group and R₈ and R₉ may form a ring by an unsubstituted or substituted alkylene group, or -COXR₁₀ in which R₁₀ represents a hydrogen atom, an unsubstituted or substituted alkyl group, an unsubstituted or substituted aryl group or an unsubstituted or substituted heterocyclic group and X represents an oxygen atom or a sulfur atom, and R₆ represents a hydrogen atom, a cyano group, an aminocarbonyl group, a sulfomethyl group or -SO₃H.

3. Ink for ink-jet recording which contains the aqueous ink according to claim 1 or 2.

4. Compounds having a structure represented by general formula (1) in a molecule or salts thereof 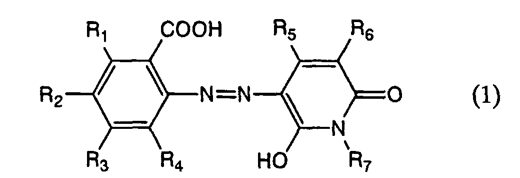 wherein
each of R₁ to R₅ and R₇ independently, represents a hydrogen atom, a halogen atom, an unsubstituted or substituted alkyl group, an unsubstituted or substituted alkoxy group, an unsubstituted or substituted alkenyl group, an unsubstituted or substituted aryl group, a hydroxyl group, -COOH, -SO₃H, -CONR₈R₉ in which each of R₈ and R₉ independently, represents a hydrogen atom, an unsubstituted or substituted alkyl group, an unsubstituted or substituted aryl group or an unsubstituted or substituted heterocyclic group and R₈ and R₉ may form a ring by an unsubstituted or substituted alkylene group, -COXR₁₀ in which R₁₀ represents a hydrogen atom, an unsubstituted or substituted alkyl group, an unsubstituted or substituted aryl group or an unsubstituted or substituted heterocyclic group and X represents an oxygen atom or a sulfur atom, or a divalent to tetravalent bonding group through which compounds represented by formula (1) are bonded to form a polymeric structure, and R₆ represents a hydrogen atom, a cyano group, an aminocarbonyl group, a sulfomethyl group or -SO₃H, provided that at least one of R₁ to R₅ and R₇ represents a divalent to tetravalent bonding group through which compounds represented by formula (1) are bonded to form a polymeric structure.

5. Compounds having a structure represented by general formula (1) in a molecule or salts thereof 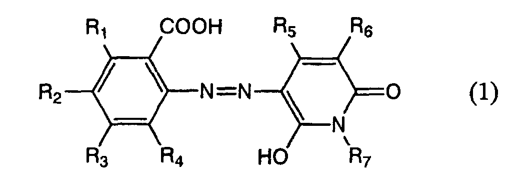 wherein
each of R₁ to R₅ and R₇ independently, represents a hydrogen atom, a halogen atom, an unsubstituted or substituted alkyl group, an unsubstituted or substituted alkoxy group, an unsubstituted or substituted alkenyl group, an unsubstituted or substituted aryl group, a hydroxyl group, -COOH, -SO₃H, -CONR₈R₉ in which each of R₈ and R₉ independently, represents a hydrogen atom, an unsubstituted or substituted alkyl group, an unsubstituted or substituted aryl group or an unsubstituted or substituted heterocyclic group and R₈ and R₉ may form a ring by an unsubstituted or substituted alkylene group, -COXR₁₀ in which R₁₀ represents a hydrogen atom, an unsubstituted or substituted alkyl group, an unsubstituted or substituted aryl group or an unsubstituted or substituted heterocyclic group and X represents an oxygen atom or a sulfur atom, or a divalent to tetravalent bonding group through which compounds represented by formula (1) are bonded to form a polymeric structure, and R₆ represents a hydrogen atom, a cyano group, an aminocarbonyl group, a sulfomethyl group or -SO₃H, provided that in at least one of structures represented by general formula (1) in the molecule, at least one of R₁ to R₅ and R₇ is -COOH, a substituted alkyl group substituted with -COOH, a substituted alkoxy group substituted with -COOH, a substituted alkenyl group substituted with -COOH or a substituted aryl group substituted with -COOH.

6. Compounds having two or more groups represented by general formula (2) in a molecule or salts thereof. 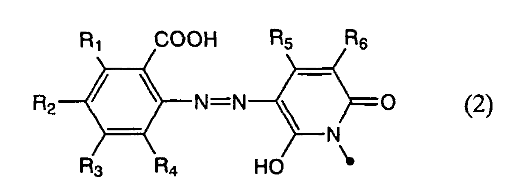 wherein
each of R₁ to R₅ independently, represents a hydrogen atom, a halogen atom, an unsubstituted or substituted alkyl group, an unsubstituted or substituted alkoxy group, an unsubstituted or substituted alkenyl group, an unsubstituted or substituted aryl group, a hydroxyl group, -COOH, -SO₃H, -CONR₈R₉ in which each of R₈ and R₉ independently, represents a hydrogen atom, an unsubstituted or substituted alkyl group, an unsubstituted or substituted aryl group or an unsubstituted or substituted heterocyclic group and R₈ and R₉ may form a ring by an unsubstituted or substituted alkylene group, or -COXR₁₀ in which R₁₀ represents a hydrogen atom, an unsubstituted or substituted alkyl group, an unsubstituted or substituted aryl group or an unsubstituted or substituted heterocyclic group and X represents an oxygen atom or a sulfur atom, and R₆ represents a hydrogen atom, a cyano group, an aminocarbonyl group, a sulfomethyl group or -SO₃H.
